# EUROPEAN PATENT APPLICATION

(11) **EP 4 183 409 A1**
(43) Date of publication of application: **24.05.2023**
(21) Application number: 21208786.0
(22) Date of filing: 17.11.2021
(51) Int. Cl.: A61K 39/12, A61P 31/14, C07K 14/165

(54) **VACCINE WITH IMPROVED IMMUNOGENICITY AGAINST MUTANT CORONAVIRUSES**

(71) Applicant: Charité - Universitätsmedizin Berlin, 10117 Berlin (DE)
(72) Inventor: Cichon, Günter, 12203 Berlin (DE); Trimpert, Jakob, 10115 Berlin (DE)
(74) Representative: Hertin und Partner Rechts- und Patentanwälte PartG mbB

(57) **Abstract**

The invention relates to an isolated nucleic acid molecule encoding a variant spike (spike-like) protein of a corona virus or fragment thereof wherein the variant spike protein comprises an amino acid sequence with at least two amino acid differences over SARS-CoV-2 "Wuhan Sequence", and wherein said variant spike protein comprises amino acid differences over SARS-CoV-2 "Wuhan Sequence" from at least two of the B.1.1.7 (alpha-variant), B.1.351 (beta-variant), B.1.1.28 (gamma-variant), B1.617.2 (delta-variant), and C.37 (lambda-variant) spike proteins. The variant comprises an amino acid sequence comprising preferably at least four amino acid differences over SARS-CoV-2 "Wuhan Sequence" being capable of inducing an immunogenic response in a human subject against a human coronavirus and variants thereof. In further aspects, the invention relates to a pharmaceutical composition in the form of a vaccine for use in the prevention and/or reduction of risk of a human coronavirus infection. The invention further relates to the medical use and corresponding methods of administering a vaccine of the invention in the reduction of risk of a human coronavirus infection and/or prevention of a medical condition associated with coronavirus, in particular SARS-COV-2 and SARS-CoV-2 variants.

## Description

The invention relates to the field of vaccination and immune stimulation with nucleic acids encoding a protein of a coronavirus or a fragment thereof.

The invention relates to an isolated nucleic acid molecule encoding a variant spike (spike-like) protein of a corona virus or fragment thereof wherein the variant spike protein comprises an amino acid sequence with at least two amino acid differences over SARS-CoV-2 "Wuhan Sequence", and wherein said variant spike protein comprises amino acid differences over SARS-CoV-2 "Wuhan Sequence" from at least two of the B.1.1.7 (alpha-variant), B.1.351 (beta-variant), B.1.1.28 (gamma-variant), B1.617.2 (delta-variant), and C.37 (lambda-variant) spike proteins. The variant preferably comprises an amino acid sequence comprising at least four amino acid differences over SARS-CoV-2 "Wuhan Sequence" being capable of inducing an immunogenic response in a human subject against a human coronavirus and variants thereof.

In further aspects, the invention relates to a pharmaceutical composition in the form of a vaccine for use in the prevention and/or reduction of risk of a human coronavirus infection.

The invention further relates to the medical use and corresponding methods of administering a vaccine of the invention in the reduction of risk of a human coronavirus infection and/or prevention of a medical condition associated with coronavirus, in particular SARS-COV-2 and SARS-CoV-2 variants.

### BACKGROUND OF THE INVENTION

In December 2019, a novel SARS-coronavirus 2 (SARS-CoV-2) emerged in Wuhan (Hubei province, China). Subsequently, SARS-CoV-2 rapidly spread within China and Asian countries before causing a worldwide pandemic of a new disease called Covid-19. Like SARS-CoV and MERS-CoV, the most recently emerged coronavirus SARS-CoV-2 can cause severe respiratory illness.

Even though a majority of patients with COVID-19 present with mild symptoms, such as cough and fever, or no symptoms at all, hospitalization is required for approximately 5% of infected subjects and patients may develop a critical disease course with acute respiratory failure. Approximately one quarter of subjects hospitalized with COVID-19-associated pneumonia require respiratory support in an intensive care unit (ICU), and the need for invasive mechanical ventilation (IMV) has been associated with high mortality¹⁻³. Long-term damages occur, even in asymptomatic patients or patients with mild or moderate disease course.

All currently used mRNA- and vector-based Covid19 vaccines worldwide use the complete gene of the spike protein of the original SARS CoV2 virus strain identified in Wuhan (China) in 2019 as vaccine gene (https://www.ncbi.nlm.nih.gov/nuccore/1798174254). With the help of the 'spike protein', the coronavirus couples to a surface receptor presented on human cells (angiotensin converting enzyme receptor 2, or ACE2). Antibodies directed against the spike protein and blocking the binding to the ACE2 receptor prevent the infection of a cell with SARS CoV2 and are called neutralizing antibodies.

However, numerous viral variants have now been isolated worldwide having different mutations in the spike protein (https://www.gisaid.org/hcov19-variants/). Mutant SARS-CoV-2 strains, such as those known as British, South African, Brazilian, Japanese, Californian or Indian variants, are usually designated according to the country of their initial isolation and greatest prevalence, but usually occur as a distinct combination of mutation patterns and travel to other countries.

The WHO classifies new SARS-CoV-2 variants as Variant of Interest (VOl) or Variants of Concern (VOC). VOCs are virus variants with altered pathogen characteristics that have been shown to adversely affect epidemiology (particularly increased transmissibility), clinical presentation (particularly increased virulence), or the effectiveness of countermeasures, diagnostic detection methods, vaccines or therapeutics (World Health Organization, 2021b). WHO has currently categorized four SARS-CoV-2 variants as VOCs: B.1.1.7, B.1.351, P.1 and B.1.617.2, also referred to as alpha, beta, gamma and delta variants according to Greek letters in the order of discovery.⁴⁻⁷

These viral variants are a significant problem in the spread of the virus and vaccination strategy. Mutations that give the virus a selection advantage often spread more rapidly in the population than the original strain. In addition, antibodies formed against the spike protein of the original strain have a reduced affinity for the spike protein variants.

The SARS-CoV-2 virus variants that have mutations in the spike protein can thereby reduce the efficacy of vaccines based on sequence information from the original strain or render them completely ineffective. Mutations affect one or more amino acids in the sequence. For example, in the variant B1.1.7 designated as the British mutant or Alpha variant, a lysine (E484K) appears at position 484 of the 1273 amino acid long amino acid sequence of the spike protein instead of the amino acid glutamic acid as in the original Wuhan strain. The E484K mutation also occurs in the beta (B.1.351) and gamma (P.1) variants, making the virus less sensitive to pre-existing immunity to the WUHAN SARS-CoV-2 strain or through currently available vaccines. The K417N polymorphism (in the SARS-CoV-2 beta and gamma variants) is also known to reduce the sensitivity of the virus to neutralizing antibodies. In the case of the South African B1.3.5.1, the reduced sensitivity to current vaccines is attributed in particular to the point mutation in position 501 of the amino acid sequence, in which a tyrosine is incorporated instead of asparagine (N501Y). The reduced protective efficacy of available vaccines against the new SARS-CoV-2 viral variants has been widely described. Further SARS-CoV-2 virus variants are expected.

The reduced sensitivity to the currently available vaccines may lower the protective effect of vaccination. In consequence, there is a need for and development of more and more new vaccines or combination of vaccines that encompass the emerging spike protein variants to ensure high sensitivity and effective protection against the new viral variants and that are safe at the same time. The need for new vaccines poses enormous challenges to industry, policymakers, the health system and the population.

Despite the high medical need, there is currently no vaccine available that protects against existing SARS-CoV-2 variants or provides effective protection against virus variants in combination, without the need for developing and vaccinating a subject with various vaccines.

The present invention demonstrates the design, method and characterization of a vaccine gene combining SARS-CoV-2 spike protein variants as immunogenetic elements for neutralizing the SARS-CoV-2 WUHAN virus strain and SARS-CoV-2 variants, suitable for prophylactic applications. The identification, design and recombinant or synthetic production of such vaccines enables several strategies for effective prophylaxis of disease.

### SUMMARY OF THE INVENTION

In light of the prior art, the technical problem underlying the invention was the provision of an alternative or improved agent in the form of a vaccine gene suitable as a prophylactic agent for preventing medical conditions associated with a SARS coronavirus and mutated SARS coronavirus variants. Another technical problem underlying the invention was the provision of a vaccine with improved efficacy also against mutated SARS coronavirus variants. The present invention solves the further technical problem of combining spike protein amino acid mutations into a single vaccine gene while conferring improved antigenicity to different coronavirus variants. Another technical problem underlying the invention was the provision of a vaccine gene combining mutations in the spike protein of SARS coronavirus variants while retaining structure, function, and antigenicity.

This problem is solved by the features of the independent claims. Preferred embodiments of the present invention are provided by the dependent claims.

The invention therefore relates to an isolated nucleic acid molecule encoding a variant spike protein (spike-like protein) of a corona virus or fragment thereof, wherein the variant spike protein comprises an amino acid sequence with at least two amino acid differences over SEQ ID NO 1 (Wuhan Sequence), and wherein said variant spike protein comprises amino acid differences over SEQ ID NO 1 from at least two of the B.1.1.7 (SEQ ID NO 2), B.1.351 (SEQ ID NO 3), B1.617.2 (SEQ ID NO 4), B.1.1.28 (SEQ ID NO 5) and C.37 (SEQ ID NO 6) spike proteins.

The invention further relates to a an isolated nucleic acid molecule encoding a variant spike (spike-like) protein of a corona virus or fragment thereof, wherein the variant (spike-like protein) comprises an amino acid sequence with at least one amino acid difference over SEQ ID NO 1 (Wuhan Sequence) from at least two of the B.1.1.7 (SEQ ID NO 2), B.1.351 (SEQ ID NO 3), B1.617.2 (SEQ ID NO 4), B.1.1.28 (SEQ ID NO 5), andC.37 (SEQ ID NO 6) spike proteins.

As described at length herein, the identification and recombinant production of an isolated nucleic acid molecule encoding a variant spike (spike-like) protein of a corona virus or fragment thereof for the use as a vaccine gene triggering neutralizing antibodies against coronavirus variants in a subject represents a novel and advantageous approach towards developing an effective Coronavirus and Coronavirus variant prophylactic treatment.

In one embodiment, the coronavirus is a SARS coronavirus. In one embodiment, the SARS coronavirus is SARS-CoV-2.

The functional property of having conferred effective prophylaxis by triggering immune response and neutralizing antibodies against SARS coronavirus and SARS coronavirus variants in a subject is a special technical feature that not only confers unique and advantageous properties to the isolated nucleic acid molecule, encoding a variant of the spike protein (spike-like) of a coronavirus, as claimed, unique and advantageous properties, but further represents a unifying functional feature of the isolated nucleic acid molecule disclosed herein in the form of a vaccine gene.

The novel isolated nucleic acid molecule encoding a variant spike (spike-like) protein of a corona virus, described herein, enables prevention of a disease, for which the human population and medical community is in desperate need of means to minimize or to eliminate the risk of being infected with corona viruses.

Although there have been attempts to produce functional vaccines for coronaviruses, particularly SARS-CoV-2, including mRNA vaccines, vector vaccines, and protein vaccines, the therapeutic and/or preventive efficacy of these vaccines has been shown to be less effective and protective for new SARS-CoV-2 spike protein variants. Each new variant requires the development of a new vaccine that would have to be used to immunize the population worldwide and significant disadvantage of the vaccines in the prior art. Surprisingly, the inventors have discovered that the combination of mutations of one or more coronavirus spike protein variants, particularly the SARS-CoV-2 spike protein, in a single nucleic acid molecule results in a vaccine gene that has demonstrated significant and unexpected immune responses in vivo, sufficient to demonstrate a superior efficacy over known solutions.

The invention described herein comprises therefore the recombinant or synthetic production and functional characterization of an isolated nucleic acid molecule encoding a variant spike (spike-like) protein of a corona virus for the uses as a vaccine gene capable of triggering SARS-CoV-2, and SARS-CoV-2 variants, neutralizing antibodies. The properties of the vaccine gene and the corresponding sequences are described below.

In one embodiment, the isolated nucleic acid molecule encoding a variant spike protein of the coronavirus comprises mutations from the spike proteins of B.1.1.7, B.1.351, B1.617.2, B.1.1.28, and C.37, combined in the nucleic acid molecule.

In one embodiment, the isolated nucleic acid molecule encoding a variant spike protein of the coronavirus is also a vaccine gene.

In one aspect, the present invention provides a vaccine gene comprising a single nucleic acid molecule encoding a variant spike protein of SARS-CoV-2 and comprising an amino acid sequence having at least two amino acid differences over SEQ ID NO 1 (Wuhan Sequence), and wherein said variant spike protein comprises amino acid differences over SEQ ID NO 1 from at least two of the B.1.1.7, B.1.351, B1.617.2, B.1.1.28, and C.37.

In another aspect, the present invention provides an effective vaccine comprising a vaccine gene comprising a nucleic acid molecule encoding a variant spike protein of SARS-CoV-2 and, when administered to a subject, provides immunity by neutralizing against the SARS-CoV-2 variants, in particular B.1.1.7, B.1.351, B1.617.2, B.1.1.28, and C.37.

Another aspect of the present invention is to provide a system that allows expression of a SARS-CoV-2 variant derived antigen, more specifically spike protein, in a mammalian cell. The vaccine may also comprise a variant spike protein of coronavirus as a polypeptide and act as an antigen.

The present invention provides a vaccine gene comprising a nucleic acid molecule encoding a variant of the coronavirus spike protein, more specifically the spike like protein, for use in protecting against infection with coronaviruses, more specifically SARS-CoV-2 and the SARS-CoV-2 variants, or against pathogenesis caused by coronaviruses, more specifically SARS-CoV-2 and the SARS-CoV-2 variants, administered to a subject. A subject being vaccinated with the vaccine gene of the invention has a significantly lower risk for infections with a SARS-CoV-2 variant, such as alpha, beta, gamma, delta, lambda, compared to a subject vaccinated with an immunogen of the original WUHAN SARS-CoV-2 strain. In one embodiment, the present invention provides a vaccine gene comprising a nucleic acid molecule encoding a variant of the coronavirus spike protein, more specifically the spike like protein, according to SEQ ID NO 15.

The concept underlying the invention is the combination of amino acid differences of the spike protein over SEQ ID NO 1 (Wuhan sequence) from multiple variants combined in a vaccine gene encoding a spike-like protein variant.

Combinations of mutations in the spike protein, as disclosed herein for the spike like protein as a vaccine gene, do not naturally occur. It is known to those skilled in the art that a large number of mutations occurring in combination in a protein would alter the structure of the protein to such an extent that folding, function or activity would be disrupted or abolished and vaccine genes would lose antigenic properties, for example against SARS coronavirus.

It is surprising and beneficial that the vaccine gene which carries a combination of mutations in the spike protein, as disclosed herein, retains its antigenic properties. The person skilled in the art also could not have expected the vaccine gene to have antigenic properties against all SARS coronavirus variants. Surprisingly, the antigenic properties of the vaccine gene against the SARS coronavirus variants are superior compared to the vaccine gene in the prior art (see Examples).

In one embodiment, the variant (spike-like protein) comprises amino acid sequence changes over SEQ ID NO 1 (Wuhan Sequence), including L18F, T19R, T20N, P26S, HV-del 69-70, G75V, T76I, D138Y, E156G, FR-del 157-158, R190S, D215G, RSYLTPG-del 246-252, D253N, K417T, L452R, T478K, E484Q, F490S, N501Y, D614G, H655Y, P681R, T716I, T859N, D950N, S982A, T1027I, D1118H and/or V1176F.

In some embodiments, the variant comprises an amino acid sequence comprising at least four amino acid differences over SEQ ID NO 1 (Wuhan Sequence) at positions 18, 19, 20, 26, 69, 70, 75, 76, 138, 144, 156, 157, 158, 190, 242-244, 246-252, 253, 417, 452, 478, 484, 490, 501, 570, 614, 655, 681, 716, 859, 950, 982, 1027, 1118 and/or 1176, with reference to SEQ ID NO 1.

Since the beginning of the spread of SARS-CoV-2, an increasing number of polymorphic nucleotide positions in various reading frames of the viral genome have been identified, wherein mutations in the coronavirus spike protein define SARS-CoV-2 variants.

WHO has currently categorised four SARS-CoV-2 variants as variant of concern (VOCs): B.1.1.7, B.1.351, P.1 and B.1.617.2. The lambda variant (C.37) is classified as variant of interest (VOl). A skilled person is able to find information about the SARS-CoV-2 variants and their sequences in the relevant literature and databases, such as NCBI, GISAID or published in Germany by the Robert Koch Institute.

In some embodiments, the variant comprises amino acid differences over SEQ ID NO 1 (Wuhan Sequence) located in two or more coronavirus spike protein domains, selected from the group consisting of an N-terminal domain (NTD), a receptor binding domain (RBP), a fusion peptide (FP), a heptapeptide repeat sequence 1 (HR1), a heptapeptide repeat sequence 2 (HR2), a transmembrane domain (TM) or cytoplasm domain (CT) and a protein sequence linking said domains.

The spike protein is responsible for entry into the host cell and consists of two subunits: The S1 subunit, comprising amino acid positions 14-685, contains the NTD and RBD, which binds to the host cell receptor, followed by cleavage sites; the S2 subunit subsequently mediates the fusion of the viral envelope and cell membrane. The spike protein induces neutralizing (protective) antibodies and is therefore of highest interest for vaccine development, wherein the NTD and RBD are most sensitive to changes for neutralizing variants. These domains affect virus transmission, entry, and sensitivity to neutralizing antibodies. The NTD comprise amino acid positions 14-303, and RBP comprising amino acid positions 306-541.

In one embodiment, the vaccine gene comprises amino acid differences over SEQ ID NO 1 in the RBD. In one embodiment, the vaccine gene comprises amino acid differences over SEQ ID NO 1 in the NTD. The RBD domain has a core subdomain and a receptor-binding motif that directly interact with the host ACE2. A vaccine should be safe, highly immunogenic and should not become obsolete as soon as there are viral mutations. Amino acid exchanges at positions 417, 452, 478, 484, and 501 in the RBD, which occur in almost all SARS-CoV-2 variants, are known to reduce the sensitivity of the mutant virus to neutralizing antibodies. Amino acid exchanges or deletions at positions 69, 70, 144, 156, 157, and 178 in the NTD, increase virus transmission, and have been associated with reduced sensitivity of the mutant virus to neutralizing antibodies. A vaccine gene comprising mutations located within the RBD and/or NTD encoding regions occurring in SARS-CoV-2 variants in the art is beneficial for high immune response and high effective SARS-CoV-2 variants neutralizing antibodies.

In some embodiments, the variant comprises an amino acid sequence comprising at least four amino acids differences over SEQ ID NO 1 (Wuhan sequence), and wherein the variant comprises amino acid differences over SEQ ID NO 1 of at least two of B.1.1.7 (SEQ ID NO 2), B.1 .351 (SEQ ID NO 3), B1.617.2 (SEQ ID NO 4), B.1.1.28 (SEQ ID NO 5), and C.37 (SEQ ID NO 6) having spike proteins at positions 18, 19, 20, 26, 69, 70, 75, 76, 138, 144, 156, 157, 158, 190, 242-244, 246-252, 253, 417, 452, 478, 484, 490, 501, 570, 614, 655, 681, 716, 859, 950, 982, 1027, 1118 and/or 1176, with reference to SEQ ID NO 1.

In some embodiments, the variant comprises an amino acid sequence comprising at least four amino acid differences over SEQ ID NO 1 (Wuhan Sequence) of two or more of the B.1.1.7 (SEQ ID NO 2), B.1.351 (SEQ ID NO 3), B1.617.2 (SEQ ID NO 4), B.1.1.28 (SEQ ID NO 5), and C.37 (SEQ ID NO 6) spike protein sequences, at positions 18, 19, 20, 26, 69, 70, 75, 76, 138, 144, 156, 157, 158, 190, 242-244, 246-252, 253, 417, 452, 478, 484, 490, 501, 570, 614, 655, 681, 716, 859, 950, 982, 1027, 1118 and/or 1176, with reference to SEQ ID NO 1.

In one embodiment, the vaccine gene encoding the variant spike protein comprises amino acid differences over SEQ ID NO 1 at positions 69, 70, 144, 156, 157, 178, 417, 452, 478, 484, and 501.

In one embodiment, the vaccine gene (SEQ ID NO 7), named ABG, encoding the variant spike protein, comprises an amino acid sequence comprising amino acids differences of each of B.1.1.7 (SEQ ID NO 2), B.1.351 (SEQ ID NO 3), and B1.617.2 (SEQ ID NO 4) spike protein sequences, with reference to SEQ ID NO 1.

In one embodiment, the vaccine gene (SEQ ID NO 8), named ABGD, encoding the variant spike protein comprises an amino acid sequence comprising amino acids differences of each of B.1.1.7 (SEQ ID NO 2), B.1.351 (SEQ ID NO 3), B1.617.2 (SEQ ID NO 4), and B.1.1.28 (SEQ ID NO 5) spike protein sequences, with reference to SEQ ID NO 1.

In one embodiment, the vaccine gene, (SEQ ID NO 9), named ABGDL, encoding the variant spike protein, comprises an amino acid sequence comprising amino acids differences of each of B.1.1.7 (SEQ ID NO 2), B.1.351 (SEQ ID NO 3), B1.617.2 (SEQ ID NO 4), B.1.1.28 (SEQ ID NO 5), and C.37 (SEQ ID NO 6) spike protein sequences, with reference to SEQ ID NO 1.

Any of the above combinations of mutations in the spike protein, results in a vaccine gene with high immunogenicity and effective protection against SARSCoV-2 and SARS-CoV-2 variants.

In some embodiments, the variant comprises an amino acid sequence "ABG" (SEQ ID NO 7) comprising L18F, T20N, P26S, HV-del 69-70, D138Y, Y144 deletion, R190S, K417T, E484K, N501Y, A570D, D614G, H655Y, P681H, T716I, S982A, T1027I, and D1118H, with reference to SEQ ID NO 1.

In some embodiments, the variant comprises an amino acid sequence "ABGD" (SEQ ID NO 8) comprising L18F, T20N, P26S, HV-del 69-70, D138Y, Y144 deletion, R190S, K417T, E484K, N501Y, P681H, T716I, S982A, T1027I, and D1118H, with reference to SEQ ID NO 1.

In some embodiments, the variant comprises an amino acid sequence "ABGD" comprising L18F, T20N, P26S, HV-del 69-70, D138Y, Y144 deletion, R190S, K417T, E484K, N501Y, P681R, T716I, S982A, T1027I, and D1118H, with reference to SEQ ID NO 1.

In some embodiments, the variant comprises an amino acid sequence "ABGD" comprising L18F, T20N, P26S, HV-del 69-70, D138Y, Y144 deletion, R190S, K417T, E484Q, N501Y, P681H, T716I, S982A, T1027I, and D1118H, with reference to SEQ ID NO 1.

In some embodiments, the variant comprises an amino acid sequence "ABGD" comprising L18F, T20N, P26S, HV-del 69-70, D138Y, Y144 deletion, R190S, K417T, E484Q, N501Y, P681R, T716I, S982A, T1027I, and D1118H, with reference to SEQ ID NO 1.

In some embodiments, the variant comprises an amino acid sequence "ABGD ver2" (SEQ ID NO 10) comprising L18F, T19R, T20N, P26S, HV-del 69-70, D138Y, Y144 deletion, FR157-158 deletion, R190S, K417T, L452R, T478K, E484K, N501Y, P681H, T716I, D950N, S982A, T1027I, and D1118H, with reference to SEQ ID NO 1.

In some embodiments, the variant comprises an amino acid sequence "ABGD ver2" comprising L18F, T19R, T20N, P26S, HV-del 69-70, D138Y, Y144 deletion, FR157-158 deletion, R190S, K417T, L452R, T478K, E484K, N501Y, P681R, T716I, D950N, S982A, T1027I, and D1118H, with reference to SEQ ID NO 1.

In some embodiments, the variant comprises an amino acid sequence "ABGD ver2" comprising L18F, T19R, T20N, P26S, HV-del 69-70, D138Y, Y144 deletion, FR157-158 deletion, R190S, K417T, L452R, T478K, E484Q, N501Y, P681H, T716I, D950N, S982A, T1027I, and D1118H, with reference to SEQ ID NO 1.

In some embodiments, the variant comprises an amino acid sequence "ABGD ver2" comprising L18F, T19R, T20N, P26S, HV-del 69-70, D138Y, Y144 deletion, FR157-158 deletion, R190S, K417T, L452R, T478K, E484Q, N501Y, P681R, T716I, D950N, S982A, T1027I, and D1118H, with reference to SEQ ID NO 1.

In some embodiments, the variant comprises an amino acid sequence "ABGDL" (SEQ ID NO 9) comprising L18F, T19R, T20N, P26S, HV69-70 deletion, G75V, T76I, D138Y, Y144 deletion, E156G, FR157-158 deletion, R190S, D215G, RSYLTPG246-252 deletion, D253N, K417T, L452R, T478K, E484Q, F490S, N501Y, D614G, H655Y, P681R, T716I, T859N, D950N, S982A, T1027I, D1118H, and V1176F, with reference to SEQ ID NO 1.

In some embodiments, the variant comprises an amino acid sequence comprising L18F, T20N, P26S, HV69-70 deletion, G75V, T76I, D138Y, Y144 deletion, 156-157 deletion, R190S, 247-253 deletion, K417T, L452R, L452Q, T478K, E484K, E484Q, F490S, N501Y, A570D, D614G, H655Y, P681H, T716I, T859N, S982A, T1027I and D1118H, with reference to SEQ ID NO 1.

In some embodiment, the variant additionally comprises one or more prefusion stabilizing amino acids, preferably at any amino acid position after 810, with reference to SEQ ID NO 1.

Prefusion stabilizing mutations, when inserted into the vaccine gene, shall ensure enhanced antigenic presentation. A variant with six beneficial proline substitutions exhibiting higher expression than its parental construct (by a factor of 10) as well as the ability to withstand heat stress, storage at room temperature, and three freeze-thaw cycles.

In one embodiment, the prefusion stabilizing mutations occur at amino acid positions 817, 892, 899, 942, 986 and 987, with reference to SEQ ID NO 1, preferably F817P, A892P, A899P, A942P, K986P, and V987P.

Any one or more of the individual amino acids listed herein, indicated as different from the SEQ ID NO 1 reference sequence, may be combined with any other one or more other of the listed amino acids, in order to arrive at a sequence of the invention. The sequences of the invention are defined by an amino acid sequence with at least two amino acid differences over SEQ ID NO 1 (Wuhan Sequence), wherein said variant spike protein comprises amino acid differences over SEQ ID NO 1 from at least two of the B.1.1.7 (SEQ ID NO 2), B.1.351 (SEQ ID NO 3), B1.617.2 (SEQ ID NO 4), B.1.1.28 (SEQ ID NO 5) and C.37 (SEQ ID NO 6) spike proteins. Thus any combination of any one or more of the mentioned amino acid differences over SEQ ID NO 1 are encompassed by the invention and considered as disclosed herein.

In one embodiment, vaccine gene encoding the spike protein variant (spike like) comprise SARS-CoV-2 spike protein variants occurring in SARS-CoV-2 variant alpha, beta, gamma, delta, and/or lambda and stabilizing amino acid substitutions.

In some embodiments, the nucleic acid molecule encodes a variant spike protein comprising or consisting of a sequence with at least 80% sequence identity, preferably 90%, more preferably 95% sequence identity, to SEQ ID NO 1.

For example, the spike protein gene variant of the present invention may have up to 80%, 81%, 82%, 83%, 84%, 85%, 86%, 87%, 88%, 89%, 90%, 91%, 92%, 93%, 94%, 95%, 96%, 97%, 98%, or 99% identity with the WUHAN spike protein gene sequence. The variant coronavirus spike protein gene encodes a protein having at least one mutation in the SARS-CoV-2 spike protein compared to the wild-type sequence of the non-structural protein.

In one embodiment, the nucleic acid molecule encodes a variant spike protein comprising or consisting of an amino acid sequence according to SEQ ID NO 7.

In one embodiment, the nucleic acid molecule encodes a variant spike protein comprising or consisting of an amino acid sequence according to SEQ ID NO 8.

In one embodiment, the nucleic acid molecule encodes a variant spike protein comprising or consisting of an amino acid sequence according to SEQ ID NO 9.

In one embodiment, the nucleic acid molecule encodes a variant spike protein comprising or consisting of an amino acid sequence according to SEQ ID NO 10.

The combinations of properties described for the vaccine gene and isolated nucleic acid, described herein, could not have been predicted by a skilled person and therefore relate to non-obvious, special technical features of the invention.

In some embodiments, the spike protein gene variant of the present invention may have up to 80%, 81%, 82%, 83%, 84%, 85%, 86%, 87%, 88%, 89%, 90%, 91%, 92%, 93%, 94%, 95%, 96%, 97%, 98%, or 99% identity with the WUHAN spike protein gene sequence, wherein any one or more of the mutations disclosed herein may be used, alone or in combination with other mutations, to define the inventive sequence. The variant coronavirus spike protein gene encodes a protein having at least one mutation in the SARS-CoV-2 spike protein compared to the wild-type sequence of the protein. The sequence identity percentages described above may also be combined with embodiment described herein, for example any embodiment regarding a list of mutations, other details regarding the presence or absence of any given mutations, may be complemented by the sequence identity values provided herein.

The invention further relates to a recombinant variant spike protein of a corona virus encoded by the nucleic acid molecule as described herein, preferably comprising or consisting of an amino acid sequence according to SEQ ID NO 7.

The invention also relates to a recombinant variant spike protein of a corona virus encoded by the nucleic acid molecule as described herein, preferably comprising or consisting of an amino acid sequence according to SEQ ID NO 8.

The invention further relates to a recombinant variant spike protein of a corona virus encoded by the nucleic acid molecule as described herein, preferably comprising or consisting of an amino acid sequence according to SEQ ID NO 9.

The invention further relates to a recombinant variant spike protein of a corona virus encoded by the nucleic acid molecule as described herein, preferably comprising or consisting of an amino acid sequence according to SEQ ID NO 10.

The invention further relates to a pharmaceutical composition in the form of a vaccine for use in the prevention and/or reduction of risk of a human coronavirus infection. In some embodiments, said vaccine comprises a nucleic acid molecule or a recombinant protein, with a pharmaceutically acceptable carrier.

In some embodiments, the composition comprises:
i) A messenger ribonucleic acid (mRNA) molecule encoding the variant spike protein of a corona virus according to any of claims 1 to 10, or
ii) A recombinant viral vector (such as adenovirus, MVA, CMV, adeno-associated virus) comprising a nucleotide sequence encoding a variant spike protein of a corona virus as described herein.
iii) A polypeptide of the variant spike protein of a corona virus comprising an amino acid sequence encoded by a nucleotide sequence as described herein.

In one embodiment, the vaccine comprises mRNA encoding the variant spike protein of a corona virus.

In one embodiment, the vaccine comprises a recombinant viral vector comprising a nucleotide sequence encoding a variant spike protein of a corona virus.

In one embodiment, the vaccine comprises a polypeptide of the variant spike protein of a corona virus.

Another advantage of the invention is that the vaccine can be used in any favoured form of vaccines in the prior art without losing effectiveness and safety. For example, the vaccine gene can be produced recombinantly, e.g. by transfection into a cell system, or synthetically, e.g. by mRNA synthesis or in vitro transcription. Methods are known to and can be easily selected by skilled person.

In some embodiments, the composition induces an immunogenic response in a human subject against a human coronavirus, wherein the human coronavirus is a SARS corona virus, preferably SARS-CoV-2, more preferably a mutant of SARS-CoV-2 including alpha, beta, gamma, delta, lambda or any combination thereof.

In some embodiments, the viral particles are to be neutralized in the body of a subject infected with the virus. In one embodiment, the composition comprising the vaccine gene is administered to a human subject. In one embodiment, the translated product of the vaccine gene within subject's cell or the polypeptide administered induces an immunogenic response in said subject, including formation of a neutralizing antibody. In one embodiment, the neutralizing antibody binds and neutralize the SARS-CoV-2 and SARS-CoV-2 variants. In one embodiment, the neutralizing antibody inhibits the interaction between angiotensin-converting enzyme 2 (ACE2) in SARS-CoV-2 and SARS-CoV-2 variants. In some embodiments, the inhibition of interaction between ACE2 and SARS-CoV-2 spike-protein is at least about 5%, 10%, 15%, 20%, 25%, or preferably 30%, 35%, 40%, 45%, more preferably 50% or 55%, or more preferably at least about 60%, 70%,75%, 80%, 85%, 90% or 95% or more, in an in vitro competition assay in which HEK-SARS-CoV-2-Spike-S1-RBD glycoprotein is immobilized on a solid phase and incubated with the antibody or antibody fragment and subsequently with ACE2.

In one embodiment, a subject may have immunity to SARS-CoV-2 and/or at least one SARS-CoV-2 variant prior to administration, may have lost immunity, or may be immune naive to SARS-CoV-2 and/or SARS-CoV-2 variants. In some embodiments, the vaccine gene encoding the variant spike protein of the invention are capable of disrupting the interaction between ACE2 and the RBD of the Spike protein. Therefore, infection of a host cell can be prevented by neutralizing antibodies triggered by the vaccine gene of the present invention. In one embodiment, the composition comprising the vaccine gene administered to a subject inhibits the infection of epithelial cells (preferably kidney epithelial cells, such as VeroE6-cells) with SARS-CoV-2 or SARS-CoV-2 variant in said subject.

In a further aspect, the invention relates to an isolated nucleic acid molecule or vaccine gene as described herein for use in the treatment and/or prevention of a medical condition associated with a SARS Coronavirus or SARS Coronavirus variant.

In one embodiment, the medical condition associated with a SARS Coronavirus or SARS Coronavirus variant is COVID-19.

In one embodiment, the medical condition associated with a SARS Coronavirus or SARS Coronavirus variant is a SARS Coronavirus-associated respiratory disease.

The invention therefore relates to corresponding methods of administration of a composition in form of a vaccine as described herein to a subject in need thereof in order to prevent a medical condition associated with a SARS Coronavirus.

In some embodiments, the composition in the form of a vaccine as described herein may be administered to various patient groups, preferably prophylactically (e.g. to patients who are at risk of having contracted a SARS Coronavirus infection.

In some embodiments, the recombinant adenovirus or recombinant adeno-associated virus is selected from the group of replication-deficient adeno-associated viral vectors and replication-deficient adenoviral vectors, including serotype 1, serotype 2, serotype 5, serotype 6, serotype 26, serotype 35, serotype 41, or e.g. the human replication-deficient E1/E3 deleted adenovirus serotype 6.

In one embodiment, the mRNA comprises
i) A 5'-cap structure,
ii) A 5'-UTR,
iii) An open reading frame encoding the variant spike protein of a corona virus as described herein,
iv) A 3'-UTR, and
v) A Poly-A-region of at least 50 nucleotides in length.

The RNA can preferably be produced by a synthesis process or also by RNA in vitro transcription. Methods for RNA synthesis and RNA in vitro transcription are known to a skilled person.

In one embodiment, the composition in the form of a vaccine is administered via intramuscular, subcutaneous, on an external muconasal route of application (e.g. nasal spray) or intravenous, preferably intramuscular means. In one embodiment, the amount of a vaccine gene in the composition administered to a patient can be 5000 ng, 4000 ng, 3000 ng, 2000 ng, 1900 ng, 1800 ng, 1700 ng, 1600 ng, 1500 ng, 1400 ng, 1300 ng, 1200 ng, 1100 ng, 1000 ng, 900ng, 800 ng, 700 ng, 600 ng, 500 ng, 400 ng, 300ng, 200 ng, or 100 ng.

Single preparations, combinational preparations or compositions are known to the skilled person who is able to evaluate compatible carrier materials and formulation forms suitable for both active compounds in the combination. In some embodiments, the quantity of active ingredient that can be combined with the carrier materials to produce a single dosage, which varies depending on the patient being treated and the particular route of administration.

Further examples of pharmaceutical compositions, conditions, treatments, subjects, variants, genes, and targets are described in detail below.

The features of the invention regarding methods of prophylactic means, administration, and vaccine gene production, and descriptions of the composition comprising the vaccine gene, for use in the prevention of a medical condition caused by SARS corona virus, apply to the composition itself, and vice versa.

The present invention includes multiple aspects, features, and embodiments, wherein these multiple aspects, features, and embodiments may be combined and permuted in any desired manner. These and other aspects, features, and embodiments of the present invention will become apparent by reference to the remainder of this application, including the following detailed description. In addition, various references are provided herein that describe specific compositions and/or methods in greater detail; all such references are incorporated herein by reference in their entirety.

### DETAILED DESCRIPTION OF THE INVENTION

The present invention addresses the problem of inadequate prophylactic measures for preventing an infection with variants of an SARS coronavirus, particularly SARS-CoV-2 variants. Preventive measures, i.e. vaccines, known in the prior art are significantly less effective for preventing an infection with a SARS-CoV-2 variant, such as delta.

In accordance with the present disclosure, there is provided herein a composition having a protective effect for infections with SARS corona virus, particularly SARS-CoV-2 variants, and associated medical conditions, such as COVID-19. It has proven difficult in the art to identify prophylactic means of said medical condition that have a preventing effect on SARS-CoV-2 WUHAN virus and SARS-CoV-2 variants known in the art.

If such an effect is possible, it prevents an infection with SARS coronavirus variants known in the art and may help to delay or stop virus spreading and possibly the pandemic situation. Such an effect is found herein for a vaccine gene and a composition comprising said vaccine gen according to the disclosure, which is further described below.

All words and terms used herein shall have the same meaning commonly given to them by the person skilled in the art, unless the context indicates a different meaning. All terms used in the singular shall include the plural of that term and vice versa.

### Vaccine

The term "vaccine" refers to a composition comprising an active ingredient resembling a disease-causing agent and is designed to stimulate the production of antibodies and confer immunity to one or more diseases or prevent severe disease progression. Vaccines may be produced from the disease-causing molecule, its products, or a synthetic substitute, wherein the vaccine or a product synthesized intracellularly by a patient through the vaccine acts as an antigen without causing the disease. A "vaccine" as described herein may have the ability to protect against or ameliorate infection and/or pathogenesis without the risk of infection, a risk that exists with an infectious or whole virus vaccine. Different types of vaccines, use different strategies to reduce the risk of disease while maintaining the ability to elicit a positive immune response.

"Protein vaccine" includes a vaccine comprising dead or inactivated organisms, purified products derived from the organism or a producing cell, or synthetically produced products.

"Genetic vaccine" includes DNA vaccines, RNA vaccines, and viral vector vaccines. An mRNA vaccine (or RNA vaccine) is a vaccine comprising an mRNA packaged in a vector such as lipid nanoparticles, and wherein the mRNA is introduced by fusion into a cell of a subject to produce specific antigens, such as surface proteins, triggering an immune response. Viral vector vaccines use a safe virus to introduce pathogen genes into the body and produce specific antigens, such as surface proteins, that trigger an immune response.

The term "vaccine gene", as used herein, refers to a nucleic acid molecule encoding a spike like protein of a coronavirus, particularly of SARS-CoV-2.

In one embodiment, the vaccine gene ABG (SEQ ID NO 7) comprises an isolated nucleic acid molecule encoding for the spike like protein of SARS-CoV-2 variants alpha, beta, and gamma.

In one embodiment, the vaccine gene ABGD (SEQ ID NO 8) comprises an isolated nucleic acid molecule encoding for the spike like protein of SARS-CoV-2 variants alpha, beta, gamma, and delta.

In one embodiment, the vaccine gene ABGDL (SEQ ID NO 9) comprises an isolated nucleic acid molecule encoding for the spike like protein of SARS-CoV-2 variants alpha, beta, gamma, delta, and lambda.

In one embodiment, the vaccine gene ABGD ver2 (SEQ ID NO 10) comprises an isolated nucleic acid molecule encoding for the spike like protein of SARS-CoV-2 variants alpha, beta, gamma, and delta.

### Medical Indications:

In one aspect of the present invention there is provided an antibody or antibody fragment according to the invention as herein described for use in the treatment of a medical condition associated with a SARS Coronavirus, wherein the medical condition associated with a SARS Coronavirus is preferably COVID-19 or a SARS Coronavirus-associated respiratory disease.

As used herein, the "patient" or "subject" may be a vertebrate. In the context of the present invention, the term "subject" includes both humans and animals, particularly mammals, and other organisms.

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

In particular, the treatment described herein relates to either reducing or inhibiting Coronavirus infection or symptoms thereof via binding the viral Spike protein with the antibodies or fragments thereof of the present invention. The prophylactic therapy as described herein is intended to encompass prevention or reduction of risk of Coronavirus infection, due to a reduced likelihood of Coronavirus infection of cells via interaction with the ACE2 protein after treatment with the antibodies or fragments thereof described herein.

In some embodiments, symptoms of infection with a SARS-virus are fever, sore throat, cough, myalgia or fatigue, and in some embodiments, additionally, sputum production, headache, hemoptysis and/or diarrhea. In some embodiments, symptoms of an infection with a SARS-coronavirus, for example SARS-CoV-2, are fever, sore throat, cough, lack of taste and/or smell, shortness of breath and/or fatigue.

As used herein, the term "a patient that is at risk of developing a severe acute respiratory syndrome (SARS)" relates to a subject, preferably distinct from any given person in the general population, who has an increased (e.g. above-average) risk of developing SARS. In some embodiments, the patient has symptoms of SARS or symptoms of a SARS Coronavirus infection. In some embodiments, the patient has no symptoms of SARS or symptoms of a SARS Coronavirus infection. In some embodiments, the subject has been in contact with people with SARS Coronavirus infections or symptoms. In some embodiments, the person at risk of developing SARS has been tested for the presence of a SARS Coronavirus infection. In some embodiments, the person at risk of developing SARS has tested positive for the presence of a SARS Coronavirus infection, preferably a coronavirus infection.

In embodiments, the patient at risk of developing SARS is an asymptomatic patient that shows no specific symptoms of SARS (yet). An asymptomatic patient may be at risk of developing SARS because the patient has been in contact with a person infected with a SARS Coronavirus. For example, the asymptomatic patient may have been identified as being at risk of developing SARS by a software application (app) that is installed on his smart phone or corresponding (portable) device and that indicates physical proximity or short physical distance to an infected patient that uses a corresponding app on its respective mobile device/smart phone. Other methods of determining contact/physical proximity to an infected person are known to the skilled person and equally apply to the method of the invention.

In some embodiments, the patient that has or is at risk of developing a severe acute respiratory syndrome (SARS) has a coronavirus infection.

### Coronavirus

Coronaviruses are a group of related viruses that cause diseases in mammals and birds. The scientific name for coronavirus is Orthocoronavirinae or Coronavirinae. Coronavirus belongs to the family of Coronaviridae. The family is divided into Coronavirinae and Torovirinae subfamilies, which are further divided into six genera: Alphacoronavirus, Betacoronavirus, Gammacoronavirus, Deltacoronavirus, Torovirus, and Bafinivirus. While viruses in the genera Alphacoronaviruses and Betacoronaviruses infect mostly mammals, the Gammacoronavirus infect avian species and members of the Deltacoronavirus genus have been found in both mammalian and avian hosts.

In humans, coronaviruses cause respiratory tract infections that can be mild, such as some cases of the common cold, and others that can be lethal, such as SARS, MERS, and COVID-19. Coronaviruses are enveloped viruses with a positive-sense single-stranded RNA genome and a nucleocapsid of helical symmetry. The genome size of coronaviruses ranges from approximately 27 to 34 kilobases, the largest among known RNA viruses.

Various species of human coronaviruses are known, such as, without limitation, Human coronavirus OC43 (HCoV-OC43), of the genus β-CoV, Human coronavirus HKU1 (HCoV-HKU1), of the genus β-CoV, Human coronavirus 229E (HCoV-229E), α-CoV, Human coronavirus NL63 (HCoV-NL63), α-CoV, Middle East respiratory syndrome-related coronavirus (MERS-CoV), Severe acute respiratory syndrome coronavirus (SARS-CoV), Severe acute respiratory syndrome coronavirus 2 (SARS-CoV-2).

Coronaviruses vary significantly in risk factor. Some can kill more than 30% of those infected (such as MERS-CoV), and some are relatively harmless, such as the common cold. Coronaviruses cause colds with major symptoms, such as fever, and a sore throat, e.g. from swollen adenoids, occurring primarily in the winter and early spring seasons. Coronaviruses can cause pneumonia (either direct viral pneumonia or secondary bacterial pneumonia) and bronchitis (either direct viral bronchitis or secondary bacterial bronchitis). Coronaviruses can also cause SARS.

Advances in nucleic acid sequencing technology (commonly termed Next-Generation Sequencing, NGS) are providing large sets of sequence data obtained from a variety of biological samples and allowing the characterization of both known and novel virus strains. Established methods are therefore available for determining a Coronavirus infection.

Viruses encode a collection of proteins required to ensure self-replication and persistence of the encoding virus. Enzymes for genome mRNA production and genome replication, proteases for protein maturation, proteins for genome encapsidation, and proteins for undermining the host antiviral responses can be identified conserved protein motifs or domains. Likely because of selective pressures, viral genomes are streamlined and the functional protein content encoded by viruses is much higher than for a cellular organisms. Thus, describing a viral genome by the collection of encoded protein domains is a potentially useful classification method. Viral evolution can therefore be followed and novel strains of coronavirus can be determined based on sequence comparison to known coronavirus strains.

In some embodiments, the patient suffers from an infection, preferably with a SARS Coronavirus (SARS-CoV). As used herein, SARS Coronavirus refers to a Coronavirus that leads to severe acute respiratory syndrome (SARS). This syndrome is a viral respiratory disease of zoonotic origin that first surfaced in the early 2000s caused by the first-identified strain of the SARS coronavirus (SARS-CoV or SARS-CoV-1).

SARS is induced via droplet transmission and replication of the virus could be shown in the upper and lower respiratory tract or gastrointestinal mucosa. In parallel the virus is also capable of directly invading cells of different organs such as liver, kidney, heart and brain. Another distinct mechanism appears to be the direct invasion of the virus in T-cells. A significant number of SARS patients, who suffer COVID-19, have a clinically low concentration of lymphocytes in the blood, also known as Lymphocytopenia. Clinically, patients show respiratory symptoms such as dry cough and shortness of breath, fever or diarrhea. But symptoms associated with acute liver injury, heart injury or kidney injury can also occur. In a less severe state of SARS, patients can show mild or even no symptoms.

Clinical and scientific investigations show that SARS-CoVs bind to the epithelial cells via the Angiotensin converting enzyme 2 receptor (ACE2). ACE2 is a cell membrane linked carboxypeptidase which is expressed in vascular endothelia, kidney, bladder, heart, nasal mucosa, bronchus and lung. As one consequence binding of the virus leads to an epithelial and endothelial cell damage along with vascular leakage, which triggers a secretion of pro-inflammatory cytokines and chemokines. The virus also mediates ACE2 downregulation and shedding which further promotes a dysfunctional renin-angiotensin system (RAS). Once the RAS is disturbed it can lead an inflammatory response and to vascular permeability. Focusing on the respiratory system, ACE2 shedding can lead to pulmonary vascular permeability and subsequently to pulmonary edema.

Older patients (above 60 years), patients with chronic diseases (e.g. cardiovascular diseases, diabetes, cancer, COPD) or immune compromised patients are considered to be at a higher risk to face a severe development of SARS. Smoking and obesity are also considered as risk factors.

Embodiments of a SARS Coronavirus include, without limitation, any coronavirus that induces a SARS or SARS-similar pathology. Particular embodiments include, without limitation, the SARS Coronavirus (SARS-CoV-1) first discovered in 2003 (as described above), the Middle East respiratory syndrome (MERS-CoV) first discovered in 2012, and the SARS-CoV-2, which causes COVID-19, a disease which brought about the 2019-2020 coronavirus pandemic.

The strain SARS-CoV-2 causes COVID-19, a disease which brought about the ongoing 2019-2020 coronavirus pandemic. The disease was first identified in December 2019 in Wuhan, the capital of China's Hubei province, and spread globally. Common symptoms include fever, cough, and shortness of breath. Other symptoms may include muscle pain, diarrhea, sore throat, loss of taste and/or smell, and abdominal pain. While the majority of cases result in mild symptoms, some progress to viral pneumonia and multi-organ failure.

Coronaviruses can be determined by molecular techniques, for example sequence-based analysis, for example by PCR-based amplification of viral genetic material. Genome-wide phylogenetic analysis indicates that SARS-CoV-2 shares 79.5% and 50% sequence identity to SARS-CoV and MERS-CoV, respectively. However, there is 94.6% sequence identity between the seven conserved replicase domains in ORF1ab of SARS-CoV-2 and SARS-CoV, and less than 90% sequence identity between those of SARS-CoV-2 and other-CoVs, implying that SARS-CoV-2 belongs to the lineage of Beta-CoVs.

Similar to other CoVs, the SARS-CoV-2 virion with a genome size of 29.9 kb possesses a nucleocapsid composed of genomic RNA and phosphorylated nucleocapsid (N) protein. The nucleocapsid is buried inside phospholipid bilayers and covered by two different types of spike proteins: the spike glycoprotein trimmer (S) that exists in all CoVs, and the hemagglutinin-esterase (HE) only shared among some CoVs. The membrane (M) protein and the envelope (E) protein are located among the S proteins in the viral envelope. The SARS-CoV-2 genome has 5' and 3' terminal sequences (265 nt at the 5' terminal and 229 nt at the 3' terminal region), which is typical of -CoVs, with a gene order 5'-replicase open reading frame (ORF) 1ab-S-envelope(E)-membrane(M)-N-30. The predicted S, ORF3a, E, M, and N genes of SARS-CoV-2 are 3822, 828, 228, 669, and 1260 nt in length, respectively. Similar to SARS-CoV, SARS-CoV-2 carries a predicted ORF8 gene (366 nt in length) located between the M and N ORF genes.

According to Jin et al (Viruses 2020, 12, 372), in the initial 41 patients, fever (98%), cough (76%), and myalgia or fatigue (44%) were the most common symptoms. Less common symptoms were sputum production (28%), headache (8%), hemoptysis (5%), and diarrhea (3%). More than half of patients developed dyspnea. The average incubation period and basic reproduction number (R0) were estimated to be 5.2 d (95% Cl: 4.1-7.0) and 2.2 d (95% Cl, 1.4-3.9), respectively.

In some embodiments, subjects have been tested and determined to have a SARS-Cov. Various methods may be employed to detect SAR-CoV, e.g. SARS-CoV-2, such as a nucleic acid test, serologic diagnosis, CRISPR/Cas13-based SHERLOCK technology, or imaging technology, such as chest radiographs or CT-scans.

The terms "Severe acute respiratory syndrome coronavirus 2", "SARS coronavirus 2" or "SARS-CoV-2", as used herein, refer to an enveloped, single-stranded, positive-sense RNA virus that cause a disease referred to as "coronavirus disease 2019", "COVID-19", "Severe acute respiratory syndrome 2" or "SARS 2". Thus, the term is not limited to a particular strain or isolate or to viruses of a particular origin, but can encompass any coronavirus that causes or is associated with COVID-19. Examples of SARS-CoV-2 coronaviruses include The virus particles contain the genetic RNA material and structural proteins required for host cell invasion. In the cell, the infecting RNA encodes structural proteins that make up the virus particles, non-structural proteins that control virus assembly, transcription, replication, and host control, and accessory proteins whose function is not yet clear. The structural proteins of SARS-CoV-2 include the envelope protein (E), the spike or surface glycoprotein (S), the membrane protein (M), and the nucleocapsid protein (N). The spike glycoprotein is located on the outside of the virus particle and gives coronaviruses their crown-like appearance. This glycoprotein mediates attachment of the viral particle and entry into the host cell. The S protein is an important target for the development of vaccines, antibody therapies and antigen-based diagnostic tests.

The terms "Middle East coronavirus," "MERS coronavirus," or "MERS-CoV" as used herein refer to single-stranded RNA viruses of the genus β-coronavirus (lineage C) that typically cause a disease referred to as "Middle East respiratory syndrome (MERS)" or "camel flu." Thus, the term is not limited to a particular strain or isolate or to viruses of a particular origin, but can encompass any coronavirus that causes or is associated with MERS. For example, a MERS coronavirus referred to herein may originate from the Middle East, e.g., the Saudi Arabian Peninsula, or from East Asia, e.g., China or Korea. Examples of MERS coronaviruses include HCoV-EMC/2012, ChinaGDOI, or a related virus.

### SARS-CoV-2 variants

The term "SARS-CoV-2 variant", "spike protein variant", or "spike variant", as used herein, is also defined by mutations within the spike protein encoded by the spike gene. The term "WUHAN variant" refers to the first SARS-CoV-2 variant identified in infected humans in China in 2019.

SARS-CoV-2 have acquired an increasing number of polymorphic nucleotide positions in various reading frames of the viral genome (such as nsp2, nsp6, RDRP, S, ORF3A, ORF8 and N) since the beginning of the spread of SARS-CoV-2, on the basis of which the viruses can be divided into clades or lineages. Intensive research is currently being conducted to determine whether or in what way certain mutations affect the properties of the virus, such as transmissibility, virulence or immunogenicity. (Alm, E., Broberg, E.K., Connor, T., Hodcroft, E.B., Komissarov, A.B., Maurer-Stroh, S., Melidou, A., Neher, R.A., O'Toole, A., Pereyaslov, D., et al. (2020). Geographical and temporal distribution of SARS-CoV-2 clades in the WHO European Region, January to June 2020. Euro Surveill 25.).

The World Health Organization classifies virus variants as Variant of Interest (VOl) or Variants of Concern (VOC).

The term "variant of interest" or "VOl" refers to a SARS-CoV-2 variant that (1) has a phenotype change or carries mutations that are likely or certain to affect the phenotype, and (2) has caused multiple case clusters or cases in different countries in the context of community transmission; or (3) has been classified as a VOl by WHO (World Health Organization (2021b). COVID-19 Weekly Epidemiological Update (Suppl. 25 February 2021), Special Edition: Proposed working definitions of SARS-CoV-2 Variants of Interest and Variants of Concern (World Health Organization).

The term "Variants of Concern" or "VOC" refers to viral variants with altered pathogen characteristics that have been shown to adversely affect epidemiology (particularly increased transmissibility), clinical presentation (particularly increased virulence), or the effectiveness of countermeasures, diagnostic detection methods, vaccines or therapeutics (World Health Organization, 2021b).

WHO has currently categorised four SARS-CoV-2 variants as VOCs: B.1.1.7, B.1.351, P.1 and B.1.617.2 (World Health Organization (2021a). COVID-19 Weekly Epidemiological Update - 11 May 2021 (WHO); Oh, D.-Y., Kroger, S., Wedde, M., Hartkopf, F., Budt, M., Seifried, J., Radonic, A., Belarbi, E., Holzer, M., Bottcher, S., et al. (2021). SARS-CoV-2-Varianten: Evolution im Zeitraffer. Deutsches Arzteblatt 118, A-460 / B-388.). According to the WHO's complementary and simplifying nomenclature system, these are also referred to as alpha, beta, gamma and delta variants according to Greek letters in the order of discovery (World Health Organization, 2021c). A skilled person is able to find information about the SARS-CoV-2 variants and their sequences in the relevant literature and databases, such as NCBI, GISAID or published in Germany by the Robert Koch Institute (www.rki.de/covid-19-varianten-nomenklatur).

As used herein, the term "derived from" means that the spike protein gene comprises substantially the same nucleotide sequence as the WUHAN spike protein gene of the subject SARS-CoV-2. For example, the spike protein gene variant of the present invention may have up to 80%, 85%, 90%, 95%, 98%, or 99% identity with the WUHAN spike protein gene sequence. The variant coronavirus spike protein gene encodes a protein having at least one mutation in the SARS-CoV-2 spike protein compared to the wild-type sequence of the non-structural protein.

In one embodiment, SARS-CoV-2 spike protein variants occurring in alpha, beta, gamma, delta, and/or lambda comprise L18F, T19R, T20N, P26S, HV-del 69-70, G75V, T76I, D138Y, E156G, FR-del 157-158, R190S, D215G, RSYLTPG-del 246-252, D253N, K417T, L452R, T478K, E484Q, F490S, N501Y, D614G, H655Y, P681R, T716I, T859N, D950N, S982A, T1027I, D1118H, and V1176F.

In one embodiment, vaccine gene encoding the spike protein variant (spike like) comprise stabilizing amino acid substitutions F817P, A892P, A899P, A942P, K086P, V987P.

"Prefusion stabilizing mutations" refer to mutations inserted in the spike protein vaccine gene, for antigenic presentation of the vaccine gene. Hsieh et al. characterized 100 structure-guided spike designs and identified 26 individual substitutions that increased protein yields and stability. Testing combinations of beneficial substitutions resulted in the identification of HexaPro, a variant with six beneficial proline substitutions exhibiting higher expression than its parental construct (by a factor of 10) as well as the ability to withstand heat stress, storage at room temperature, and three freeze-thaw cycles. (Hsieh, C. L., et al. (2020). "Structure-based design of prefusion-stabilized SARS-CoV-2 spikes." Science 369(6510): 1501-1505)

In one embodiment, vaccine gene encoding the spike protein variant (spike like) comprise SARS-CoV-2 spike protein variants occurring in SARS-CoV-2 variant alpha, beta, gamma, delta, and/or lambda and stabilizing amino acid substitutions.

In one embodiment, variant coronavirus spike protein gene encodes a protein comprising at least one of the mutations including L18F, T19R, T20N, P26S, HV-del 69-70, G75V, T76I, D138Y, E156G, FR-del 157-158, R190S, D215G, RSYLTPG-del 246-252, D253N, K417T, L452R, T478K, E484Q, F490S, N501Y, D614G, H655Y, P681R, T716I, T859N, D950N, S982A, T1027I, D1118H, and/or V1176F, or all of said mutations.

The term "pathogenicity" is used herein in its usual meaning and refers to the potential of the virus to cause disease in a human. Typically, the pathogenicity of a coronavirus, such as SARS-CoV-2, is determined by examining disease-associated symptoms, such as fever, loss of taste, cough, sneezing, rhinitis, and reduction in oxygen saturation.

In some embodiments, the subject is at risk from an infection with at least one of the SARS-CoV-2 variants.

In virology, the term "clade" refers to groups of similar viruses based on their genetic sequences, which all trace back to a common ancestor. Genetic variations of a virus are grouped into clades, which may also be called subtypes, genotypes or groups. Current SARS-CoV-2 variants defined as clade or lineage are listed at the WHO, GISAID or published by the Robert Koch Institute Germany (www.rki.de/covid-19-varianten-nomenklatur) that is updated regularly.

A "SARS-CoV-2 clade", as used herein, refers to one of the virus variants listed herein, e.g. alpha, beta, gamma, defined by their genotype, more specifically by mutations in the spike protein. Within a clade, there may be "subclades" having one or more additional mutations and/or a different amino acid substitution at the same position in the spike protein as amino acid position defining said clade.

### SARS-CoV-2 nomenclature

On 31 May 2021, the World Health Organisation (WHO) introduced another nomenclature for SARS-CoV-2 virus variants. Variants that are considered Variant of Concern (VOC) or Variant of Interest (VOl) according to WHO are named after letters of the Greek alphabet.

These designations with letters of the Greek alphabet do not replace the established scientific designations. Scientific names include the nomenclatures according to (i) Pangolin (e.g. B.1.1.7), (ii) GISAID (e.g. GRY), or (iii) Nextstrain (e.g. 20l/S:501Y.V1), and are further used for professional exchange and professional public communication.

### Alpha (B.1.1.7)

"Alpha variant", "B.1.1.7", "VOC202012/01", "GR/501Y.V1", "20l/501Y.V1" or "UKvariant", as used herein, is a SARS-CoV-2 variant defined by mutations in the spike protein comprising del69/70, del144, N501Y, A570D, D614G, P681H, T716I, S982A, D1118H, (SEQ ID NO 2 and NO 13) and accounts for roughly 95% of new SARS-CoV-2 infections in England.

N501Y mutant virus exhibited consistent fitness gains for replication in the upper airway in the hamster model as well as primary human airway epithelial cells. N501Y mutation in the receptor-binding domain (RBD) of spike protein can enhance its affinity to the human ACE2 receptor (a major impact on fast spread of the variant). P681H mutation could potentially affect cell infectivity and replication of virus. Another variant has emerged from the "UK variant"; the "Bristol variant" including the E484K mutation (potentially favouring evasion form the immune response) also found in the "South African" and "Brazilian" variants. A sub-lineage of B.1.1.7 emerged through sequential acquisitions of V70L mutation in the membrane protein followed by a novel D178H mutation in the S protein. The percentage of this sub-lineage variant in the U.S. increased from 0.15% in February 2021 to 1.8% in April 2021. To date this sub-lineage appears to be U.S.-specific with reported cases in 31 states, including Hawaii. As of April 2021 it constituted 36.8% of all B.1.1.7 isolates in Washington. Structural analysis revealed that the D178H mutation in the S protein is in the N-terminal domain NTD) of the S protein and close to two other signature mutations of B.1.1.7, HV69-70del and Y144del. It is surface exposed and may alter NTD tertiary configuration or accessibility, and thus has the potential to affect neutralization by NTD directed antibodies.

Alpha variant spreads more easily and quickly than other previously known variants. Indeed, the variant has shown 30-50% enhanced transmissibility compared to initially emerged SARS-CoV-2. In the US, alpha has also shown to be ∼50% more transmissible than other circulating lineages and the proportion of cases caused by this variant is increasing at a rate of ∼7.5% per day. A reverse genetics approach has shown that a single amino acid change (N501Y) was a major determinant responsible for increased transmission of this variant.

In the UK, the mortality hazard ratio associated with infection with the variant compared with infection with previously circulating variants was 1.64 in patients in the community. In this comparatively low risk group, this represents an increase in deaths from 2.5 to 4.1 per 1000 detected cases.

Pfizer-BioNTech, Moderna, AstraZeneca-Oxford, Johnson and Johnson, Novavax have confirmed that their vaccines, based on different designs, is less but still effective against this variant.

### Beta (B1.351)

"Beta", "B.1.351", "501Y.V2", "VOC202012/02", "GH/501Y.V2", "20H/501Y.V2" or "South Africa variant" as used herein, is a SARS-CoV-2 variant defined by mutations, particularly non-synonymous mutations, in the spike protein comprising L18F, D80A, D215G, LAL242-244del, R246I, K417N, E484K, N501Y, D614G, A701V (SEQ ID NO 3 and 14), including three amino acid substitutions (K417N, E484K and N501Y) in the receptor binding domain of the spike protein, which can enhance its affinity to the human ACE2 receptor. (Tegally, H., Wilkinson, E., Giovanetti, M., Iranzadeh, A., Fonseca, V., Giandhari, J., Doolabh, D., Pillay, S., San, E., Msomi, N., et al. (2020). Emergence and rapid spread of a new severe acute respiratory syndrome-related coronavirus 2 (SARS-CoV-2) lineage with multiple spike mutations in South Africa.). In vitro data indicate an increase in ACE2 receptor affinity when the E484K and N501Y polymorphisms are combined (Zahradník, J., Marciano, S., Shemesh, M., Zoler, E., Chiaravalli, J., Meyer, B., Dym, O., Elad, N., and Schreiber, G. (2021). SARS-CoV-2 RBD <em>in vitro</em> evolution follows contagious mutation spread, yet generates an able infection inhibitor. bioRxiv, 2021.2001.2006.425392.). The E484K mutation might lead to escape from the immune response and was first detected in South Africa. Beta variant emerged with an unusually large number of mutations in South Africa. As the variant spreads globally and faster than other earlier variants of the virus, the variant may be driving the second wave of the pandemic.

Beta variant is associated with in-hospital mortality that is 20% higher in the second wave in South Africa than in the first wave.

It has been known for some time that the K417N and E484K polymorphisms reduce the sensitivity of the virus to neutralising antibodies. This suggests that after infection with the variant of origin or vaccination with a vaccine based on it, the immune response against beta (B.1.351) could be reduced in effectiveness (Andreano, E., Piccini, G., Licastro, D., Casalino, L., Johnson, N.V., Paciello, I., Monego, S.D., Pantano, E., Manganaro, N., Manenti, A., et al. (2020). SARS-CoV-2 escape in vitro from a highly neutralizing COVID-19 convalescent plasma. bioRxiv, 2020.2012.2028.424451.; Greaney, A.J., Loes, A.N., Crawford, K.H.D., Starr, T.N., Malone, K.D., Chu, H.Y., and Bloom, J.D. (2021). Comprehensive mapping of mutations to the SARS-CoV-2 receptor-binding domain that affect recognition by polyclonal human serum antibodies. bioRxiv, 2020.2012.2031.425021.; Wang, Z., Schmidt, F., Weisblum, Y., Muecksch, F., Barnes, C.O., Finkin, S., Schaefer-Babajew, D., Cipolla, M., Gaebler, C., Lieberman, J.A., et al. (2021b). mRNA vaccine-elicited antibodies to SARS-CoV-2 and circulating variants. bioRxiv, 2021.2001.2015.426911.; Weisblum, Y., Schmidt, F., Zhang, F., DaSilva, J., Poston, D., Lorenzi, J.C., Muecksch, F., Rutkowska, M., Hoffmann, H.H., Michailidis, E., et al. (2020). Escape from neutralizing antibodies by SARS-CoV-2 spike protein variants. Elife 9.). Fittingly, the neutralising activity of convalescent or vaccinated plasma is reduced against this variant (Cele, S., Gazy, I., Jackson, L., Hwa, S.-H., Tegally, H., Lustig, G., Giandhari, J., Pillay, S., Wilkinson, E., Naidoo, Y., et al. (2021). Escape of SARS-CoV-2 501Y.V2 from neutralization by convalescent plasma. medRxiv, 2021.2001.2026.21250224.; Wang, P., Nair, M.S., Liu, L., Iketani, S., Luo, Y., Guo, Y., Wang, M., Yu, J., Zhang, B., Kwong, P.D., et al. (2021a). Antibody Resistance of SARS-CoV-2 Variants B.1.351 and B.1.1.7. Nature.; Wibmer, C.K., Ayres, F., Hermanus, T., Madzivhandila, M., Kgagudi, P., Oosthuysen, B., Lambson, B.E., de Oliveira, T., Vermeulen, M., van der Berg, K., et al. (2021). SARS-CoV-2 501Y.V2 escapes neutralization by South African COVID-19 donor plasma. Nature Medicine.); there is also evidence that reinfections with beta (B.1.351) can occur after passed infection with a previously circulating SARS-CoV-2 variant (Shinde, V., Bhikha, S., Hoosain, Z., Archary, M., Bhorat, Q., Fairlie, L., Lalloo, U., Masilela, M.S.L., Moodley, D., Hanley, S., et al. (2021). Preliminary Efficacy of the NVX-CoV2373 Covid-19 Vaccine Against the B.1.351 Variant. medRxiv, 2021.2002.2025.21252477.). Phase 3 clinical studies of vaccine efficacy also suggest reduced efficacy of certain vaccines against infection by this variant (Madhi, S.A., Baillie, V., Cutland, C.L., Voysey, M., Koen, A.L., Fairlie, L., Padayachee, S.D., Dheda, K., Barnabas, S.L., Bhorat, Q.E., et al. (2021). Efficacy of the ChAdOx1 nCoV-19 Covid-19 Vaccine against the B.1.351 Variant. N Engl J Med.).

With regard to escape from natural immunity, the 501Y.V2 variant showed complete escape from neutralizing antibodies in 48% of convalescent serum samples obtained from patients who had previously had Covid-19. In vitro studies have shown that antibodies induced by all available vaccines (Pfizer-BioNTech, Moderna, AstraZeneca-Oxford, Johnson and Johnson, Novavax) yield decreased neutralization activity on this variant in vitro. Clinical studies conducted by Novavax, Johnson and Johnson and AstraZeneca have confirmed a decreased efficacy. Specifically, Novavax clinical trials showed 89% efficacy in the UK and 60% efficacy in South Africa. Johnson and Johnson clinical trials showed that the level of protection against moderate to severe COVID-19 infection was 72% in the US, 66% in Latin America and 57% in South Africa, 28 days post-vaccination.

AstraZeneca vaccination was halted since clinical trials did not show protection against mild or moderate illness caused by the more contagious Beta virus variant. Overall vaccine efficacy for Covid-19 of any degree of severity more than 14 days after the first dose was 33.5%.

### Gamma (P1)

"Gamma", "P1", "B.1.1.28.1", "501Y.V3", "VOC202101/02", "GR/501Y.V3", "20J/501Y.V3" or "Brazilian variant" as used herein, is a SARS-CoV-2 variant defined by mutations in the spike protein comprising L18F, T20N, P26S, D138Y, R190S, K417T, E484K, N501Y, D614G, H655Y, T1027I, V1176F (SEQ ID NO 5). N501Y, K417N, and E484K are located in the receptor binding domain of spike protein, which can enhance its affinity to the human ACE2 receptor. Increased transmissibility, reduced effectiveness of neutralising antibodies and increased virulence are also discussed for this variant (Faria, N.R., Mellan, T.A., Whittaker, C., Claro, I.M., Candido, D.D.S., Mishra, S., Crispim, M.A.E., Sales, F.C.S., Hawryluk, I., McCrone, J.T., et al. (2021a). Genomics and epidemiology of the P.1 SARS-CoV-2 lineage in Manaus, Brazil. Science.; Faria, N.R., Morales Claro, I., Candido, D., Moyses Franco, L.A., Andrade, P.S., Coletti, T.M., Silva, C.A.M., Sales, F.C., Manuli, E.R., Aguiar, R.S., et al. (2021b). Genomic characterisation of an emergent SARS-CoV-2 lineage in Manaus: preliminary findings.).

Gamma variant was first detected in Brazil. It has caused widespread infection in the city of Manaus. As shown with other UK and South African variants, the Brazilian variant has shown to enhanced transmissibility and is spreading globally. P1 variant has shown to be as much as 2.5 times more contagious than the original coronavirus.

Gamma variant includes the potential immune escape mutation (E484K). The variant has shown to be relatively resistant to neutralization by convalescent plasma and vaccinee sera after Moderna or Pfizer vaccination. However, the magnitude of the loss was modest (3.8-4.8 fold). The study was conducted among nearly 70,000 health care workers in Manaus, which was the epicenter for the emergence of the P.1 variant.

### B1.617.2 (Delta) and B.1.617.1 (Delta-Plus)

"B1.617.2", "Delta", "VOC202104/01", "G/452R.V3", "21A/S:478K" or "Indian variant", as used herein, is a SARS-CoV-2 variant defined by mutations in the spike protein comprising T19R, del157-158, L452R, T478K, D614G, P681R, D950N (SEQ ID NO 4). Several of these mutations may affect immune responses targeting key antigenic regions of the receptor binding protein (452 and 478) and deletion of part of the N-terminal domain (157 and 158). The single L452R amino acid substitution has been shown to increase ACE2 receptor affinity and viral particle infectivity in vitro (Motozono et al., 2021). Laboratory data also suggest that this mutation involves a change in antigenic properties (Motozono, C., Toyoda, M., Zahradnik, J., Saito, A., Nasser, H., Tan, T.S., Ngare, I., Kimura, I., Uriu, K., Kosugi, Y., et al. (2021). SARS-CoV-2 spike L452R variant evades cellular immunity and increases infectivity. Cell Host Microbe 29, 1124-1136 e1111.; Public Health England (2021b). SARS-CoV-2 variants of concern and variants under investigation in England. Technical briefing 10.. In available online at https://assetspublishingservicegovuk/government/uploads/system/uploads/attachment). The P681R exchange, at a site immediately adjacent to the furin cleavage site, is associated in vitro with increased cleavage of the S protein at the S1/S2 interface, which may increase viral infectivity and promote transmissibility (Peacock, T.P., Sheppard, C.M., Brown, J.C., Goonawardane, N., Zhou, J., Whiteley, M., Consortium, P.V., de Silva, T.I., and Barclay, W.S. (2021). The SARS-CoV-2 variants associated with infections in India, B.1.617, show enhanced spike cleavage by furin. bioRxiv, 2021.2005.2028.446163.).

The delta variant was first detected in India in December 2020 and could be more than twice as transmissible as the original strain WUHAN of SARS-CoV-2. The variant was first detectable in infected persons four days after exposure, compared with an average of six days in infected persons with the original virus, suggesting increased replication of this variant. Individuals infected with this variant also had viral loads up to 1,260 times higher than individuals infected with the original virus. The delta variant is now contributing to a wave of cases around the world and delta variant already accounts for more than 90% of all infections. There has been a rapid increase in cases with the variant, which has been linked to travel to India and transmission in the community. Growth rates show that the delta variant is growing faster than the alpha variant. A modeling study predicts that there is a "realistic possibility" that B.1.617.2 is 60% more transmissible than the alpha variant (B.1.1.7).

Laboratory experiments have shown a reduction in neutralisability by convalescent sera for the delta variant and for vaccine sera, especially after administration of only one vaccine dose (Liu, C., Ginn, H.M., Dejnirattisai, W., Supasa, P., Wang, B., Tuekprakhon, A., Nutalai, R., Zhou, D., Mentzer, A.J., Zhao, Y., et al. (2021). Reduced neutralization of SARS-CoV-2 B.1.617 by vaccine and convalescent serum. Cell.; Planas, D., Veyer, D., Baidaliuk, A., Staropoli, I., Guivel-Benhassine, F., Rajah, M.M., Planchais, C., Porrot, F., Robillard, N., Puech, J., et al. (2021). Reduced sensitivity of SARS-CoV-2 variant Delta to antibody neutralization. Nature.). It was demonstrated that neutralising activity against neither beta nor delta in ∼50% of those recovered one year after illness.

First observational studies on clinical vaccine efficacy show that the clinical protective efficacy of vaccination is significantly reduced after administration of only one vaccine dose compared to the delta variant (Lopez Bernal, J., Andrews, N., Gower, C., Gallagher, E., Simmons, R., Thelwall, S., Stowe, J., Tessier, E., Groves, N., Dabrera, G., et al. (2021). Effectiveness of Covid-19 Vaccines against the B.1.617.2 (Delta) Variant. N Engl J Med.; Nasreen, S., Chung, H., He, S., Brown, K.A., Gubbay, J.B., Buchan, S.A., Fell, D.B., Austin, P.C., Schwartz, K.L., Sundaram, M.E., et al. (2021). Effectiveness of COVID-19 vaccines against variants of concern in Ontario, Canada. medRxiv, 2021.2006.2028.21259420.). According to Lopez-Bernal et al. the protective effect against symptomatic delta (B.1.617.2) infections after one vaccine dose (mRNA or vector vaccine) was 36% (95%Cl: 23-46%), which is below the protective effect against symptomatic alpha (B.1.1.7) infections, which is 48% (42-53%). After complete vaccination, the m-RNA vaccine was found to be 88% (85-90%) protective, slightly below the 94% (92-95%) protective effect of the same vaccine against symptomatic alpha (B.1.1.7) infections. The protective efficacy of the vector vaccine used in the UK against symptomatic B.1.617.2 infections is 67% (61-72%), also below the 75% (68-79%)% protective efficacy of this vaccine against symptomatic B.1.1.7 infections (Lopez Bernal, J., Andrews, N., Gower, C., Gallagher, E., Simmons, R., Thelwall, S., Stowe, J., Tessier, E., Groves, N., Dabrera, G., et al. (2021). Effectiveness of Covid-19 Vaccines against the B.1.617.2 (Delta) Variant. N Engl J Med.). The B.1.617.2 (delta) variant has been detected in Germany since March 2021 and is now the predominant variant in Germany.

A sub-variant of Delta is "Delta-Plus", also called "B.1.617.1", and was first identified in India in April 2021. "Delta-Plus" includes an additional mutation in the spike protein comprising K417N (SEQ ID NO 11). It has been suggested that this change slightly decreases the binding affinity to ACE2. A recent study showed that hamsters infected with B.1.617.1 had increased body weight loss, higher viral load in the lungs, and marked lung lesions compared with the B.1 variant.

The use of an infectious virus assay revealed that the B.1.617.1 variant was 6.8 times more resistant to neutralization by sera from COVID-19 convalescents and from individuals vaccinated with Moderna and Pfizer. In a UK study, the efficacy of Pfizer's 2-dose vaccine against this variant was 87.9% (93.4% against B.1.1.7); AstraZeneca's 2-dose vaccine showed 59.8% efficacy against this variant and 66.1% against B.1.1.7. (Lopez Bernal J, Andrews N, Gower C, Gallagher E, Simmons R, Thelwall S, Stowe J, Tessier E, Groves N, Dabrera G, Myers R, Campbell CNJ, Amirthalingam G, Edmunds M, Zambon M, Brown KE, Hopkins S, Chand M, Ramsay M. Effectiveness of Covid-19 Vaccines against the B.1.617.2 (Delta) Variant. N Engl J Med. 2021 Aug 12;385(7):585-594.)

### C.37 (Lambda)

"Lambda variant", "C.37 variant" or "Peru variant" is defined by mutations in the spike protein comprising D614G, T859N, F490S, L452Q or L452R, T76I, G75V, del246/252 (SEQ ID NO 6). Mutations L452Q and F490S locate in the Spike protein's receptor-binding domain. L452R is known to be associated with increased affinity for the ACE2 receptor. F490S has been associated with reduced in vitro susceptibility to antibody neutralization.

"Lambda variant" was first detected in Peru in December 2020 and currently is causing eighty percent of new cases of COVID-19 in Peru has become a dominant variant in South America and found in more than 30 countries. Lambda variant has been categorized as a "variant of interest" by the World Health Organization. The researchers found that while pseudotyped virus expressing the C.37 viral spike protein was less susceptible to vaccine-elicited neutralizing antibodies.

### Spike and ACE2 proteins

The Coronavirus Spike protein, also known as S protein, is a glycoprotein trimer, wherein each monomer of the trimeric S protein is about 180 kDa, and contains two subunits, S1 and S2, mediating attachment and membrane fusion, respectively. As described in Xiuyuan Ou et al (Nature Communications, 11, 1620, 2020), Coronaviruses (CoV) use the Spike glycoprotein to bind ACE2 and mediate membrane fusion and virus entry. In the S protein structure, N- and C- terminal portions of S1 fold as two independent domains, N-terminal domain (NTD) and C-terminal domain (C-domain). Depending on the virus, either the NTD or C-domain can serve as the receptor-binding domain (RBD). While RBD of mouse hepatitis virus (MHV) is located at the NTD14, most of other CoVs, including SARS-CoV and MERS-CoV use C-domain to bind their receptors.

Like other coronaviruses, SARS-CoV-2 has four structural proteins, known as the S (spike), E (envelope), M (membrane), and N (nucleocapsid) proteins; the N protein holds the RNA genome, and the S, E, and M proteins together create the viral envelope. The spike protein, which has been imaged at the atomic level using cryogenic electron microscopy, is the protein responsible for allowing the virus to attach to and fuse with the membrane of a host cell.

The receptors for SARS-CoV and MERS-CoV are human angiotensin-converting enzyme 2 (hACE2) and human dipeptidyl peptidase 4 (hDPP4), respectively. CoV S proteins are typical class I viral fusion proteins, and protease cleavage is required for activation of the fusion potential of S protein. A two-step sequential protease cleavage model has been proposed for activation of S proteins of SARS-CoV and MERS-CoV, priming cleavage between S1 and S2 and activating cleavage on S2' site. Depending on virus strains and cell types, CoV S proteins may be cleaved by one or several host proteases, including furin, trypsin, cathepsins, transmembrane protease serine protease-2 (TMPRSS-2), TMPRSS-4, or human airway trypsin-like protease (HAT). Availability of these proteases on target cells largely determines whether CoVs enter cells through plasma membrane or endocytosis.

Angiotensin-converting enzyme 2 (ACE2) is a cell membrane linked carboxypeptidase presented on the outer surface (cell membranes) of cells in the vascular endothelia, kidney, bladder, heart, nasal mucosa, bronchus and lung. ACE2 has the function of lowering blood pressure by catalyzing the hydrolysis of angiotensin II into angiotensin. ACE2 counters the activity of the related angiotensin-converting enzyme (ACE) making it a drug target for treating cardiovascular diseases. ACE2 is a which is expressed in. ACE2 also serves as the entry point into cells for some coronaviruses including Severe acute respiratory syndrome coronavirus 2.

ACE2 is a zinc containing metalloenzyme that contains an N-terminal peptidase M2 domain and a C-terminal collectrin renal amino acid transporter domain. ACE2 is a single-pass type I membrane protein, with its enzymatically active domain exposed on the surface of cells. The extracellular domain of ACE2 is cleaved from the transmembrane domain by another enzyme known as sheddase, and the resulting soluble protein is released into the blood stream and ultimately excreted into urine.

### Spike like protein

The term "spike like protein", "mutated spike protein" or "spike variant" refers to amino acid sequences of spike protein in SARS-CoV-2 variants differing to the WUHAN spike protein sequence of the original SARS-CoV-2 strain according to SEQ ID No: 1. In embodiments, the spike-like protein differs in at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or more amino acids from the WUHAN spike protein (SEQ ID NO: 1) and/or has identity to the WUHAN sequence (SEQ ID NO. : 1) of or less than 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% or less.

### Antibodies

As used herein, an "antibody" generally refers to a protein consisting of one or more polypeptides substantially encoded by immunoglobulin genes or fragments of immunoglobulin genes. Where the term "antibody" is used, the term "antibody fragment" may also be considered to be referred to.

"Specific binding" is to be understood as via one skilled in the art, whereby the skilled person is clearly aware of various experimental procedures that can be used to test binding and binding specificity. Some cross-reaction or background binding may be inevitable in many protein-protein interactions; this is not to detract from the "specificity" of the binding between antibody and epitope. The term "directed against" is also applicable when considering the term "specificity" in understanding the interaction between antibody and epitope.

### Antigen

An "antigen" is a substance that is capable of being recognized (typically via its epitope(s)) by the immune system, preferably the adaptive immune system, and is capable of eliciting an antigen-specific immune response, e.g., by producing antibodies and/or antigen-specific T cells as part of an adaptive immune response. In the context of the present invention, "antigens" are preferably peptides, polypeptides or proteins comprising or consisting of a SARS coronavirus protein or fragment or variant thereof, as defined herein. A peptide is also a polypeptide.

An "antigen" as used in the present invention may further be an "antigenic peptide or protein" as defined herein. The term "antigenic peptide or protein" as used herein also generally refers to a (poly)peptide or protein derived from a protein, preferably a SARS-CoV-2 protein such as the SARS-CoV-2 spike protein or SARS-CoV-2 spike like protein, that is capable of eliciting an (adaptive) immune response. Therefore, an antigenic peptide or protein preferably comprises or provides at least one epitope of the protein from which it is derived. The expression comprises (poly)peptides or proteins without being restricted in length, composition or structure. The term "epitope" or "antigenic determinant" usually refers to the part of an antigen that is recognized by the adaptive immune system.. Typically, an antigen comprises a peptide or protein that can be presented to (antigen-specific) T cells on MHC surface molecules through the MHC complex. For purposes of the present invention, an antigenic peptide or protein is typically the product of translation of a provided nucleic acid molecule, preferably an mRNA or vector DNA, as defined herein. As used herein, the term "epitope" specifically refers to a portion or fragment of an antigen presented on an MHC surface molecule. Such a fragment comprising or consisting of a (functional epitope) may typically comprise from about 5 to about 20 amino acids. Epitopes can be divided into T-cell epitopes and B-cell epitopes. T-cell epitopes or portions of proteins within the scope of the present invention may comprise fragments that are preferably from about 6 to about 20 or even more amino acids in length, e.g., fragments as processed and presented by MHC class I molecules, preferably from about 8 to about 10 amino acids in length, e.g. 8, 9, or 10 (or even 11 or 12 amino acids), or fragments as processed and presented by MHC class II molecules, preferably having a length of about 13 or more amino acids, e.g., 13, 14, 15, 16, 17, 18, 19, 20, or even more amino acids, wherein these fragments may be selected from any part of the amino acid sequence. These fragments are typically recognized by T cells in the form of a complex consisting of the peptide fragment and an MHC surface molecule, i.e., the fragments are typically not recognized in their native form. B-cell epitopes are typically fragments located on the outer surface of (native) protein or peptide antigens as defined herein, preferably with 5 to 15 amino acids, more preferably with 5 to 12 amino acids, even more preferably with 6 to 9 amino acids that can be recognized by antibodies, i.e., in their native form. The term "epitope" includes "conformational" (or "discontinuous") epitopes, which consist of discontinuous sequences of the amino acids of the antigen but are joined in the three-dimensional structure, and "linear" epitopes, which are formed by a continuous sequence of amino acids of the antigen.

### Sequence Variatio:

Sequence variants of the claimed nucleic acids, proteins and antibodies, for example defined by the claimed % sequence identity, that maintain the said properties of the invention are also included in the scope of the invention. Such variants, which show alternative sequences, but maintain essentially the same binding properties, such as target specificity, as the specific sequences provided are known as functional analogues, or as functionally analogous. Sequence identity relates to the percentage of identical nucleotides or amino acids when carrying out a sequence alignment.

It will be appreciated by those of ordinary skill in the art that, as a result of the degeneracy of the genetic code, there are many nucleotide sequences that encode a polypeptide as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions and other changes in sequence that fall under the described sequence identity are also encompassed in the invention.

Protein sequence modifications, which may occur through substitutions, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein, preferably without significantly altering the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, Ile and Leu; the non-polar aromatic amino acids Phe, Trp and Tyr; the neutral polar amino acids Ser, Thr, Cys, Gln, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

Substitution variants have at least one amino acid residue in the antibody molecule removed and a different residue inserted in its place. The sites of greatest interest for substitutional mutagenesis include the hypervariable regions, but FR alterations are also contemplated. If such substitutions result in a change in biological activity, then more substantial changes, denominated "exemplary substitutions" in the table immediately below, or as further described below in reference to amino acid classes, may be introduced and the products screened.

**Potential Amino Acid Substitutions:**

| **Original residue** | **Preferred conservative substitutions** | **Examples of exemplary substitutions** |
|---|---|---|
| Ala (A) | Val | Val; Leu; Ile |
| Asg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain.

Conservative amino acid substitutions are not limited to naturally occurring amino acids, but also include synthetic amino acids. Commonly used synthetic amino acids are omega amino acids of various chain lengths and cyclohexyl alanine which are neutral non-polar analogs; citrulline and methionine sulfoxide which are neutral non-polar analogs, phenylglycine which is an aromatic neutral analog; cysteic acid which is a negatively charged analog and ornithine which is a positively charged amino acid analog. Like the naturally occurring amino acids, this list is not exhaustive, but merely exemplary of the substitutions that are well known in the art.

### Protein variants

Variants of proteins: "Variants" of proteins or peptides, as defined in the context of the present invention, may be generated that have an amino acid sequence that differs from the original sequence in one or more mutation(s), such as one or more substituted , inserted and/or deleted amino acid(s).

Preferably, these fragments and/or variants have the same biological function or specific activity compared to the native full length protein, e.g. its specific antigenic property. "Variants" of proteins or peptides, as defined in the context of the present invention, may comprise one or more conservative amino acid substitution(s) compared to their native, i.e. non-mutated, physiological sequence. These amino acid sequences, as well as their coding nucleotide sequences, more particularly fall within the term "variants" as defined herein. Substitutions in which amino acids originating from the same class are exchanged for each other are called conservative substitutions. These are in particular amino acids with aliphatic side chains, positively or negatively charged side chains, aromatic groups in the side chains or amino acids whose side chains can form hydrogen bonds, e.g. side chains that have a hydroxyl function. This means that, for example, an amino acid with a polar side chain is replaced by another amino acid with a side chain that is also polar, or, for example, an amino acid characterised by a hydrophobic side chain is replaced by another amino acid with a side chain that is also hydrophobic (e.g. serine (threonine) by threonine (serine) or leucine (isoleucine) by isoleucine (leucine)).

Insertions and substitutions are possible especially at such sequence positions that do not cause a change in the three-dimensional structure or do not affect the binding region. Modifications to a three-dimensional structure by insertion(s) or deletion(s) can be easily determined, e.g. using circular dichroism spectra (CD spectra) (Urry, 1985, Absorption, Circular Dichroism and ORD of Polypeptides, in: Modern Physical Methods in Biochemistry, Neuberger et al., (eds.), Elsevier, Amsterdam).

Furthermore, variants of proteins or peptides as defined herein that may be encoded by a nucleic acid molecule may also comprise such sequences, wherein nucleotides of the encoding nucleic acid sequence are exchanged according to the degeneracy of the genetic code without any change in the respective amino acid sequence of the protein or peptide, i.e. the amino acid sequence or at least a part thereof may not differ from the original sequence in one or more mutation(s) as defined above.

Fragments of proteins: "Fragments" of proteins or peptides in the context of the present invention may typically comprise a sequence of a protein or peptide as defined herein, that is, with respect to its amino acid sequence (or its encoded nucleic acid) molecule), N-terminally and/or C-terminally truncated compared to the amino acid sequence of the original (native) protein (or its encoded nucleic acid molecule).

In this context, a fragment of a protein may typically comprise an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98% or 99%, preferably of at least 70%, more preferably of at least 80%, even more preferably of at least 85%, even more preferably of at least 90% and most preferably of at least 95% or even 97%, having an amino acid sequence of the respective naturally occurring full-length protein.

### Vectors

The term "vector" is used herein to refer to a nucleic acid molecule capable of transferring or transporting another nucleic acid molecule, also termed as "gene delivery system". Classically, vectors are divided into viral and non-viral vectors. The transferred nucleic acid is generally linked to, e.g., inserted into, the vector nucleic acid molecule. A vector may include sequences that direct autonomous replication in a cell, or may include sequences sufficient to allow integration into host cell DNA. Useful vectors include, for example, plasmids (e.g., DNA plasmids or RNA plasmids), transposons, cosmids, bacterial artificial chromosomes, inorganic nanoparticles, polymers, dendrimers, cationic lipids, liposomes, cationic lipid vesicles, solid lipid nanoparticle, lipid nano emulsions, peptide-based vectors, and viral vectors. Useful viral vectors include, e.g., replication defective retroviruses and lentiviruses. Useful inorganic nanoparticle vectors include, e.g. gold, iron oxide, carbon nanotubes, silica, calcium phosphate, and graphene. Useful polymer vectors include, e.g., poly (DL- Lactide), poly ( DL-Lactide- co- glycoside), polyethylenimine, chitosan, polyaminoester, polymethacrylate, polyspermine, and cyclodextrin. Useful dendrimer vector include, e.g., polyamidoamine, polyethylene glycol-poly-L-lysin, and butylenediamine polypropylenimine. Useful cationic lipid vectors include, e.g., DOTMA (N-(1-(2,3-dioleyloxy)propyl)-N,N,N-trimethylammonium), DOPE (di-oleoylphosphatidyl-ethanolamine), DOGS (ioctadecylamidoglycyl-spermine), DOSPA (2,3-dioleyloxy-N-[2-(sperminecarboxamido)ethyl]-N,N-dimethyl -1-propanaminium trifluoroacetate), DC-cholsterol, DOTPA (N-[1-(2,3-dioleoyloxy)propyl]-N,N,N-trimethylammonium methylsulfate), DMRIE (1,2-dimyristyloxy-propyl-3-dimethyl-hydroxy ethyl ammonium bromide), and DTPA (dodecatungstophosphoric acid). Useful peptide vectors include, e.g. protamine sulphate, poly-L-lysine, poly-L-arginines, and CPP (cell penetrating peptides).

In non-viral gene transfer, the nucleic acid is condensed by interaction with e.g. a polymer or lipid, or encapsulated in e.g. a liposome, to form a polyplex or lipoplex and pass the cell membrane by endocytosis. Once the endosome escapes, the complex is released into the cytosol, and the released nucleic acid is transported to the perinuclear region via the microtubule system. Translocation of an exogenous DNA into the nucleus can be achieved by passing through the nuclear pore complex in nondividing cells.

Both viral and non-viral vectors used for gene transfer in gene therapy rely on the fact that genetic material must be transported to the cell, across the cell membrane, and eventually into the nucleus. Vectors facilitate transport, uptake into the cell by e.g. endocytosis, and protect the harbored nucleic acid from damaging environmental influences, including enzymatic degradation. Accordingly, any selected vector is capable of increasing the stability of the nucleic acid it contains. The vector may allow the encapsulated nucleic acid to reach the target cell and/or may preferably allow the encapsulated nucleic acid to reach the target cell, or, alternatively, limit the delivery of that mRNA to other sites or cells where the presence of the administered mRNA may be useless or undesirable. Further, incorporation of the nucleic acid into a vector also facilitates delivery of that nucleic acid to a target cell.

The aim of gene therapy is to accomplish intracellular delivery of nucleic acids (also named as transgene) into specific cells to achieve a therapeutic effect, wherein the therapeutic effect includes, for example, correcting an existing abnormality, providing cells with a new function, or replacing a defective or missing gene by a single administration of an appropriate vector and transfer method.

A recombinant adenovirus, MVA, CMV, or recombinant adeno-associated virus or recombinant adeno-associated virus is selected from the group of replication-deficient adeno-associated viral vectors and replication-deficient adenoviral vectors, including serotype 1, serotype 2, serotype 5, serotype 6, serotype 26, serotype 35, serotype 41, or e.g. the human replication-deficient E1/E3 deleted adenovirus serotype 6. In some embodiments, the recombinant adenovirus or recombinant adeno-associated virus comprise a nucleotide sequence encoding a variant spike protein of a corona virus, wherein said nucleotide sequence is a vaccine gene. In particular embodiments, a host cell in subject is infected with a viral vector comprising the vaccine gene.

As will be evident to one of skill in the art, the term "viral vector" is widely used to refer either to a nucleic acid molecule (e.g., a transfer plasmid) that includes virus-derived nucleic acid elements that typically facilitate transfer of the nucleic acid molecule or integration into the genome of a cell or to a viral particle that mediates nucleic acid transfer. Viral particles will typically include various viral components and sometimes also host cell components in addition to nucleic acid(s).

In one embodiment, the vector used in the composition of the invention is a liposomal vector comprising a lipid nanoparticle. The liposomal vector may be selected and/or prepared to optimize delivery of the mRNA to a target cell. Liposomal vectors are usually characterized as microscopic vesicles with an internal water space that is compartmentalized from an external medium by a membrane of one or more bilayers. Bilayer membranes of liposomes are usually formed by amphiphilic molecules, such as lipids of synthetic or natural origin, comprising spatially separated hydrophilic and hydrophobic domains. The bilayer membranes of liposomes can also be formed by amphiphilic polymers and surfactants (e.g., polymerosomes, niosomes, etc.).

For present invention purposes, liposomal vectors may be used, wherein liposomal vectors are produced to contain at least one desired nucleic acid. The incorporation of a nucleic acid into a liposome may also be referred to as "encapsulation", wherein the nucleic acid is completely enclosed within the interior of the liposome. The purpose of incorporating an mRNA into a liposomal vector, such as a liposome, is often to protect the nucleic acid from an environment that may contain enzymes or chemicals that degrade nucleic acids and/or systems or receptors that cause rapid shedding of the nucleic acids. Accordingly, any liposomal vector selected is capable of increasing the stability of the mRNA it contains. The liposome may allow the encapsulated mRNA to reach the target cell and/or may preferably allow the encapsulated mRNA to reach the target cell. Further, incorporation of the mRNA into a cationic liposome also facilitates delivery of that mRNA to a target cell. In one embodiment, lipid nanoparticle comprise one or more cationic lipids, non-cationic lipids, and PEG-modified lipids. Preferably, the lipid nanoparticles are formulated to deliver one or more mRNA to one or more target cells.

### Vaccine protein production:

The vaccine may comprise the spike like protein (vaccine protein), mRNA encoding the spike like protein or a vector DNA encoding the spike like protein (vaccine gene). SARS-CoV-2 coronavirus infection vaccine or SARS-CoV-2 coronavirus variant infection SARS-CoV-2 variant vaccine: A vaccine directed against a SARS-CoV-2 coronavirus is referred to herein as a SARS-CoV-2 coronavirus infection vaccine or SARS-CoV-2 coronavirus variant infection SARS-CoV-2 variant vaccine.

The vaccine comprising the vaccine protein as antigen supplied as a pharmaceutical product form of a protein of the present invention may be produced by synthesize of the polypeptide or by transfection of a host cell with an expression vector comprising the coding sequence for the vaccine protein of the invention. An expression vector or recombinant plasmid is produced by placing these coding sequences for the antibody in operative association with conventional regulatory control sequences capable of controlling the replication and expression in, and/or secretion from, a host cell. Regulatory sequences include promoter sequences, e.g., CMV promoter, and signal sequences which can be derived from other known antibodies.

A selected host cell is co-transfected by conventional techniques with vectors (or simply transfected by a single vector) to create the transfected host cell of the invention comprising the recombinant vaccine protein. The transfected cell is then cultured by conventional techniques to produce the engineered vaccine protein of the invention.

Suitable vectors for the cloning and subcloning steps employed in the methods and construction of the compositions of this invention may be selected by one of skill in the art. For example, the conventional pUC series of cloning vectors may be used. One vector, pUC19, is commercially available. The components of such vectors, e.g. replicons, selection genes, enhancers, promoters, signal sequences and the like, may be obtained from commercial or natural sources or synthesized by known procedures for use in directing the expression and/or secretion of the product of the recombinant DNA in a selected host. Other appropriate expression vectors of which numerous types are known in the art for mammalian, bacterial, insect, yeast, and fungal expression may also be selected for this purpose.

The present invention also encompasses a cell line transfected with a recombinant plasmid containing the coding sequences of the vaccine protein of the present invention. Host cells useful for the cloning and other manipulations of these cloning vectors are also conventional.

Suitable host cells or cell lines for the expression of the vaccine proteins of the invention include mammalian cells such as NS0, Sp2/0, CHO (e.g. DG44), COS, HEK, a fibroblast cell (e.g., 3T3), and myeloma cells, for example it may be expressed in a CHO or a myeloma cell. Human cells may be used, thus enabling the molecule to be modified with human glycosylation patterns. Alternatively, other prokaryotic or eukaryotic cell lines may be employed. The selection of suitable mammalian host cells and methods for transformation, culture, amplification, screening and product production and purification are known in the art.

In accordance with the present invention there is provided a method of producing an vaccine protein of the present invention which leads to antibody formation, immunity, and/or neutralizing activity of the virus in the subject, which method comprises the steps of; (a) providing a vector encoding vaccine protein, preferably the Spike like protein, (b) transforming a mammalian host cell (e.g. CHO) with said vector; (c) culturing the host cell under conditions conducive to the secretion of the vaccine protein from said host cell into said culture media; (d) recovering the secreted vaccine protein of step (c) or isolating the vaccine protein from cells of step (c) by protein isolation and purification methods known in the art. Once expressed, the vaccine protein can be assessed for the desired binding properties using methods as described herein.

A vaccine gene comprising a polypeptide can be also produced synthetically.

"Immune system": The immune system can protect organisms from infection. If a pathogen breaks through a physical barrier of an organism and enters that organism, the innate immune system delivers an immediate but non-specific response. If pathogens escape this innate response, vertebrates have a second layer of protection, the adaptive immune system. Here, the immune system adapts its response during an infection to improve recognition of the pathogen.

This improved response is then retained after the pathogen is eliminated in the form of an immunological memory, enabling the adaptive immune system to launch faster and stronger attacks each time the pathogen appears. Accordingly, the immune system comprises the innate immune system and the adaptive immune system. Each of these two parts contains so-called humoral and cellular components.

"Immune response": an immune response can typically be either a specific response of the adaptive immune system to a particular antigen (so-called specific or adaptive immune response) or a non-specific response of the innate immune system (so-called non-specific or innate immune response). The invention relates to the core to specific responses (adaptive immune responses) of the adaptive immune system. In particular, it relates to adaptive immune responses to infections by viruses such as SARS-CoV-2 coronaviruses. However, this specific response may be supported by an additional non-specific response (innate immune response).

Therefore, the invention also relates to a compound for simultaneously stimulating the innate and adaptive immune systems to elicit an efficient adaptive immune response. Adaptive immune system: The adaptive immune system consists of highly specialised systemic cells and processes that eliminate or prevent pathogenic growth. The adaptive immune response gives the vertebrate immune system the ability to recognise and remember specific pathogens (to generate immunity) and to launch stronger attacks each time the pathogen appears.

### Pharmaceutical compositions:

In some embodiments, the invention relates to a pharmaceutical composition in the form of a vaccine comprising an isolated nucleic acid molecule encoding a variant spike (spike-like) protein of a corona virus or fragment thereof of the invention together with a pharmaceutically acceptable carrier. A pharmaceutically acceptable carrier in the sense of the present invention may be any non-toxic material, such as an adiuvant or adjuvant component that does not significantly interfere in a detrimental sense with the effectiveness of the biological activity of the isolated nucleic acid molecule of the present invention.

Evidently, the characteristics of the carrier will depend on the route of administration. Such a composition may contain, in addition to the active substance and carrier, diluents, fillers, salts, buffers, stabilizers, solubilizers, and other materials well known in the art. Formulation of pharmaceutically-acceptable excipients and carrier solutions is well-known to those of skill in the art, as is the development of suitable dosing and treatment regimens for using the particular compositions described herein in a variety of treatment regimens, including, without limitation, oral, parenteral, intravenous, sub-cutaneous, intranasal, inhalation, and intramuscular administration.

Adiuvant/adjuvant component: An adjuvant or adjuvant component in the broadest sense is typically an agent or composition (e.g. pharmacological or immunological) which, for example, enhances the efficacy of other active ingredients, such as a drug or vaccine.

Conventionally, in the context of the invention, the term refers to a compound or composition that serves as a carrier or excipient for immunogens and/or other pharmaceutically active compounds. It is to be interpreted broadly to refer to a wide range of substances capable of enhancing the immunogenicity of antigens incorporated into or co-administered with an adjuvant in question. In the context of the present invention, an adjuvant will preferably enhance the specific immunogenic effect of the active agents of the present invention. Typically, "adjuvant" or "adjuvant component" have the same meaning and may be used interchangeably. Adjuvants may be divided into, for example, immunopotentiators, antigenic delivery systems or even combinations thereof.

The term "adjuvant" is not typically intended to include agents that confer immunity themselves.

An adjuvant assists the immune system non-specifically in enhancing the antigen-specific immune response by, for example, promoting the presentation of an antigen to the immune system or inducing a non-specific innate immune response. Further, an adjuvant may preferably e.g. modulate the antigen-specific immune response, e.g. shift the dominant Th2-based antigen-specific response to a more Th1-based antigen-specific response or vice versa. Accordingly, an adjuvant may favourably modulate cytokine expression/secretion, antigen presentation, type of immune response, etc.

*Vaccine:* A vaccine is typically understood to be a prophylactic or therapeutic material that provides at least one antigen or antigenic function. The antigen or antigen function may stimulate the body's adaptive immune system to provide an adaptive immune response. Antigen-delivering mRNA: An antigen-delivering mRNA in the context of the invention can typically be an mRNA that has at least one open reading frame that can be translated by a cell or organism provided with that mRNA. The product of this translation is a peptide or protein that can act as an antigen, preferably an immunogen. The product may also be a fusion protein consisting of more than one immunogen, e.g. a fusion protein consisting of two or more epitopes, peptides or proteins derived from the same or different viral proteins, wherein the epitopes, peptides or proteins may be linked by linker sequences.

*Antigen-delivering peptide:* Peptide or protein capable of acting as an antigen, preferably as an immunogen.

*Antigen-delivering vector DNA:* An antigen-delivering vector DNA in the context of the invention can typically be a vector DNA, e.g. plasmid or viral vector, which has at least one open reading frame that can be transcribed into mRNA by a cell or organism provided with this vector DNA and subsequently the resulting mRNA can be translated. The product of this translation is a peptide or protein that can act as an antigen, preferably as an immunogen.

The vaccine, otherwise known as a pharmaceutical composition, containing the active ingredient (isolated nucleic acid molecule encoding a variant spike (spike-like) protein of a corona virus or fragment thereof or polypeptide encoding a variant spike (spike-like) protein) may be in a form suitable for intramuscular, cutaneous, subcutaneous, intravenous or muconasal application. A skilled person is aware of the carriers and additives required for particular application forms.

The vaccine, otherwise known as a pharmaceutical composition, containing the active ingredient (isolated nucleic acid molecule encoding a variant spike (spike-like) protein of a corona virus or fragment thereof or polypeptide encoding a variant spike (spike-like) protein) may be in a form suitable for injection. Modes of administration involving injection comprise, without limitation, Subcutaneous (under the skin), Intramuscular (in a muscle), Intravenous (in a vein) or Intrathecal (around the spinal cord). Antibody therapies are typically administered using intravenous administration.

Excipients, as an example of pharmaceutically acceptable carrier, for liquid formulations intended for injection are known in the art and can be selected appropriately by a skilled person. Excipients have been used to increase the stability of a wide range of protein and peptide-based formulations by reducing protein dynamics and motion, increasing the conformational stability of vaccine gene or vaccine protein especially at high concentrations and inhibiting interface-dependent aggregation. Excipients usually inhibit aggregation and protects the protein by adsorbing to the air-liquid interface; for example, the use of surfactants (e.g., polysorbate 20 and 80), carbohydrates (e.g., cyclodextrin derivatives) and amino acids (e.g., arginine and histidine) can help prevent aggregation by this mechanism. Cyclodextrin has been reported to stabilize commercially available antibody-based drugs in a hydrogel formulation. Some of the generally recognized as safe (GRAS) excipients include pluronic F68, trehalose, glycine and amino acids such as arginine, glycine, glutamate and histidine, which are found in a number of commercial protein therapeutic products. By way of example, bevacizumab, 25 mg/mL, contains trehalose dehydrate, sodium phosphate and polysorbate 20. Excipients in subcutaneous trastuzumab, 600 mg, are rHuPH20, histidine hydrochloride, histidine, trehalose dehydrate, polysorbate 20, methionine and water for injection.

When a therapeutically effective amount of the active substance (isolated nucleic acid molecule encoding a variant spike (spike-like) protein of a corona virus or fragment thereof or polypeptide encoding a variant spike (spike-like) protein) of the invention is administered by intramuscular, intravenous, cutaneous or subcutaneous injection, the active substance may be in the form of a solution, preferably a pyrogen-free, parenterally acceptable aqueous solution.

The preparation of such parenterally acceptable solutions, having due regard to pH, isotonicity, stability, and the like, is within the skill in the art. A preferred pharmaceutical composition for intramuscular, intravenous, cutaneous, or subcutaneous injection should contain, in addition to the active substance, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art.

The invention also relates to administration of a therapeutically relevant amount of active substance (isolated nucleic acid molecule encoding a variant spike (spike-like) protein of a corona virus or fragment thereof or polypeptide encoding a variant spike (spike-like) protein) as described herein in the prevention of a subject who is at risk receiving the medical disorders as disclosed herein. As used herein, the term "therapeutically effective amount" means the total amount of each active component of the pharmaceutical composition or method that is sufficient to show a meaningful patient benefit. The amount of active substance in the pharmaceutical composition of the present invention will depend upon the nature and severity of the condition being treated, and on the nature of prior treatments which the patient has undergone. Larger doses may be administered until the optimal preventing effect is obtained for the patient, and at that point the dosage is not increased further.

The dose of the vaccine administered evidently depends on numerous factors well-known in the art such as, e.g., the chemical nature and pharmaceutical formulation of the active substance, and of body weight, body surface, age and sex of the patient, as well as the time and route of administration. For an adult, the dose may exemplarily be between 0.001 µg and 1 g per day, preferably between 0.1 µg and 100 mg per day, more preferably between 1 µg and 100 mg per day, even more preferably between 5 µg and 10 mg per day. In a continuous infusion, the dose may exemplarily be between 0.01 µg and 100 mg, preferably between 1 µg and 10 mg per kilogram body mass per minute.

### Embodiments relating to analyzing patient sample and diagnostic:

In another advantageous embodiment an immunoassay is used in the detection of Coronavirus using the antibodies or fragments thereof of the present invention, to which end binding of the antibodies or fragments thereof of the present invention to a solid phase is envisaged.

In another advantageous embodiment an immunoassay is used in the detection of Coronavirus using the antibodies or fragments thereof of the present invention, to which end binding of the antibodies or fragments thereof of the present invention to a solid phase is envisaged.

Following addition of sample solution, virus in the patient's sample binds to the solid phase bound antibodies or fragments thereof of the present invention. The virus which is obtained e.g. from the serum of a patient and bound to the solid phase is subsequently detected using a label, or labelled reagent and optionally quantified, preferably using a further antibody directed against the Coronavirus, as disclosed herein. Thus, according to the invention, detection of the virus in this method is achieved using labelled reagents according to the well-known ELISA (Enzyme-Linked Immunosorbent Assay) technology. Labels according to the invention therefore comprise enzymes catalysing a chemical reaction which can be determined by optical means, especially by means of chromogenic substrates, chemiluminescent methods or fluorescent dyes. In another preferred embodiment the autoantibodies are detected by labelling with weakly radioactive substances in radioimmunoassays (RIA) wherein the resulting radioactivity is measured.

The term immunoassay encompasses techniques including, without limitation, enzyme immunoassays (EIA) such as enzyme multiplied immunoassay technique (EMIT), enzyme-linked immunosorbent assay (ELISA), antigen capture ELISA, sandwich ELISA, IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); lateral flow assays (LFA) and chemiluminescence assays (CL).

In another preferred embodiment of the invention, soluble or solid phase-bound antibodies or fragments thereof of the present invention are used to bind the virus. In a second reaction step, further antibodies, such as those of the present invention, directed against the virus are employed, said further antibodies being detectably labelled. The advantage of this embodiment lies in the use of ELISA technology usually available in laboratory facilities so that detection according to the invention can be established in a cost-effective manner. The further antibodies may be detectably coupled to fluorescein isothiocyanate (FITC). Much like the above-mentioned ELISA, the FITC technology represents a system that is available in many places and therefore allows smooth and low-cost establishment of the inventive detection in laboratory routine.

Indirect labels include various enzymes well-known in the art, such as horseradish peroxidase (HRP), alkaline phosphatase (AP), β-gaiactosidase, urease, and the like. A horseradish-peroxidase detection system can be used, for example, with the chromogenic substrate tetramethylbenzidine (TMB), which yields a soluble product in the presence of hydrogen peroxide that is detectable at 450 nm. An alkaline phosphatase detection system can be used with the chromogenic substrate p-nitrophenyl phosphate, for example, which yields a soluble product readily detectable at 405 nm. Similarly, a β-gaiactosidase detection system can be used with the chromogenic substrate o-nitrophenyl-β-D-galactopyranoside (ONPG), which yields a soluble product detectable at 410 nm.

In another embodiment of the invention the antibodies or fragments thereof of the present invention in accordance with one or more of the sequences disclosed herein is bound to a solid phase. Binding of antibodies or fragments thereof of the present invention in accordance with one or more of the sequences disclosed herein to the solid phase can be effected via a spacer. All those chemical compounds having suitable structural and functional preconditions for spacer function can be used as spacers as long as they do not modify the binding behavior in such a way that binding of the virus to antibodies or fragments thereof of the present invention is adversely affected. In another preferred embodiment of the invention the molecule comprises a linker or spacer selected from the group of α-aminocarboxylic acids as well as homo- and heterooligomers thereof, α,ω-aminocarboxylic acids and branched homo- or heterooligomers thereof, other amino acids, as well as linear and branched homo- or heterooligomers; amino-oligoalkoxyalkylamines; maleinimidocarboxylic acid derivatives; oligomers of alkylamines; 4-alkylphenyl derivatives; 4-oligoalkoxyphenyl or 4-oligoalkoxyphenoxy derivatives; 4-oligoalkylmercaptophenyl or 4-oligoalkylmercaptophenoxy derivatives; 4-oligoalkylaminophenyl or 4-oligoalkylaminophenoxy derivatives; (oligoalkylbenzyl)phenyl or 4-(oligoalkylbenzyl)phenoxy derivatives, as well as 4-(oligoalkoxybenzyl)phenyl or 4-(oligoalkoxybenzyl)phenoxy derivatives; trityl derivatives; benzyloxyaryl or benzyloxyalkyl derivatives; xanthen-3-yloxyalkyl derivatives; (4-alkylphenyl)- or ω-(4-alkylphenoxy)alkanoic acid derivatives; oligoalkylphenoxyalkyl or oligo-alkoxyphenoxyalkyl derivatives; carbamate derivatives; amines; trialkylsilyl or dialkylalkoxysilyl derivatives; alkyl or aryl derivatives or combinations thereof.

In another embodiment of the invention, the antibodies or fragments thereof of the present invention are immobilized. More specifically, the solid phase-bound antibodies or fragments thereof of the present invention is bound to organic, inorganic, synthetic and/or mixed polymers, preferably agarose, cellulose, silica gel, polyamides and/or polyvinyl alcohols. In the meaning of the invention, immobilization is understood to involve various methods and techniques to fix the antibodies or fragments thereof of the present invention on specific carriers, e.g. according to WO 99/56126 or WO 02/26292. For example, immobilization can serve to stabilize the peptides so that their activity would not be reduced or adversely modified by biological, chemical or physical exposure, especially during storage or in single-batch use. Immobilization of the antibodies or fragments thereof of the present invention allows repeated use under technical or clinical routine conditions; furthermore, a sample - preferably blood components - can be reacted with at least one of the antibodies or fragments thereof of the present invention in a continuous fashion. In the meaning of the invention, three basic methods can be used for immobilization:
(i) Crosslinking: in crosslinking, the antibodies are fixed to one another without adversely affecting their activity. Advantageously, they are no longer soluble as a result of such crosslinking.
(ii) Binding to a carrier: binding to a carrier proceeds via adsorption, ionic binding or covalent binding, for example. Such binding may also take place inside microbial cells or liposomes or other membranous, closed or open structures. Advantageously, the antibodies are not adversely affected by such fixing. For example, multiple or continuous use of carrier-bound antibodies is possible with advantage in clinical diagnosis or therapy.
(iii) Inclusion: inclusion in the meaning of the invention especially proceeds in a semipermeable membrane in the form of gels, fibrils or fibers. Advantageously, encapsulated antibodies are separated from the surrounding sample solution by a semipermeable membrane in such a way that interaction with the virus still is possible. Various methods are available for immobilization, such as adsorption on an inert or electrically charged inorganic or organic carrier.

Another method is covalent binding to carrier materials. In addition, the carriers may have reactive groups forming homopolar bonds with amino acid side chains. Suitable groups in antibodies are carboxy, hydroxy and sulfide groups and especially the terminal amino groups of lysines. Aromatic groups offer the possibility of diazo coupling. Advantageously, a large number of antibodies can undergo direct covalent binding with polyacrylamide resins.

The invention also relates to a diagnostic kit for the determination of a Coronavirus infection, comprising antibodies in accordance with one or more of the sequences as disclosed herein. The diagnostic kit optionally includes instructions concerning combining the contents of the kit and/or providing a formulation for the detection of viral infection. For example, the instruction can be in the form of an instruction leaflet or other medium providing the user with information as to the type of method wherein the substances mentioned are to be used. Obviously, the information need not necessarily be in the form of an instruction leaflet, and the information may also be imparted via the Internet, for example. To a patient, one advantageous effect of such a kit is, for instance, that he or she, without directly addressing a physician, can determine the actual state of a disease.

### SEQUENCES

**Preferred amino acid sequences of the invention:** Each highlighted (bold and large type in the table) amino acid difference over SEQ ID NO: 1 (Wuhan sequence) may be absent or present in the invented product as disclosed herein. Amino acids that may be deleted in the invented product as disclosed herein and therefore absent in the amino acid sequences listed below, said amino acids may also be present in the invented product as disclosed herein.

| **SEQ ID** | **Info** | **Amino Acid Sequence (N-C)** |
|---|---|---|
| 1 | WUHAN Sequence | >YP_009724390.1 surface glycoprotein [Severe acute respiratory syndrome coronavirus 2] |
| | | |
| 2 | B.1.1.7 (Alpha) | >QQQ47833.1 surface glycoprotein [Severe acute respiratory syndrome coronavirus 2] |
| | | MFVFLVLLPLVSSQCVNLTTRTQLPPAYTNSFTRGVYYPDKVFRSSVLHST QDLFLPFFSNVTWFHAISGTNGTKRFDNPVLPFNDGVYFASTEKSNIIRGWI FGTTLDSKTQSLLIVNNATNVVIKVCEFQFCNDPFLGVYHKNNKSWMESEF |
| | | RVYSSANNCTFEYVSQPFLMDLEGKQGNFKNLREFVFKNIDGYFKIYSKHT PINLVRDLPQGFSALEPLVDLPIGINITRFQTLLALHRSYLTPGDSSSGWTAG AAAYYVGYLQPRTFLLKYNENGTITDAVDCALDPLSETKCTLKSFTVEKGIY QTSNFRVQPTESIVRFPNITNLCPFGEVFNATRFASVYAWNRKRISNCVAD YSVLYNSASFSTFKCYGVSPTKLNDLCFTNVYADSFVIRGDEVRQIAPGQT GKIADYNYKLPDDFTGCVIAWNSNNLDSKVGGNYNYLYRLFRKSNLKPFER DISTEIYQAGSTPCNGVEGFNCYFPLQSYGFQPTYGVGYQPYRVVVLSF ELLHAPATVCGPKKSTNLVKNKCVNFNFNGLTGTGVLTESNKKFLPFQQFG RDIDDTTDAVRDPQTLEILDITPCSFGGVSVITPGTNTSNQVAVLYQGV NCTEVPVAIHADQLTPTWRVYSTGSNVFQTRAGCLIGAEHVNNSYECDIPI GAGICASYQTQTNSHRRARSVASQSIIAYTMSLGAENSVAYSNNSIAIPIN FTISVTTEILPVSMTKTSVDCTMYICGDSTECSNLLLQYGSFCTQLNRALTGI AVEQDKNTQEVFAQVKQIYKTPPIKDFGGFNFSQILPDPSKPSKRSFIEDLL FNKVTLADAGFIKQYGDCLGDIAARDLICAQKFNGLTVLPPLLTDEMIAQYT SALLAGTITSGWTFGAGAALQIPFAMQMAYRFNGIGVTQNVLYENQKLIAN QFNSAIGKIQDSLSSTASALGKLQDVVNQNAQALNTLVKQLSSNFGAISSVL NDILARLDKVEAEVQIDRLITGRLQSLQTYVTQQLIRAAEIRASANLAATKM SECVLGQSKRVDFCGKGYHLMSFPQSAPHGVVFLHVTYVPAQEKNFTTAP AICHDGKAHFPREGVFVSNGTHWFVTQRNFYEPQIITTHNTFVSGNCDV VIGIVNNTVYDPLQPELDSFKEELDKYFKNHTSPDVDLGDISGINASVVNIQK EIDRLNEVAKNLNESLIDLQELGKYEQYIKWPWYIWLGFIAGLIAIVMVTIMLC CMTSCCSCLKGCCSCGSCCKFDEDDSEPVLKGVKLHYT |
| 3 | B.1.351 (Beta) | >QWW93436.1 surface glycoprotein [Severe acute respiratory syndrome coronavirus 2] |
| | | MFVFLVLLPLVSSQCVN F TTRTQLPPAYTNSFTRGVYYPDKVFRSSVLHS TQDLFLPFFSNVTWFHAIHVSGTNGTKRF A NPVLPFNDGVYFASTEKSNII RGWIFGTTLDSKTQSLLIVNNATNVVIKVCEFQFCNDPFLGVYYHKNNKSW MESEFRVYSSANNCTFEYVSQPFLMDLEGKQGNFKNLREFVFKNIDGYFKI YSKHTPINLVR G LPQGFSALEPLVDLPIGINITRFQTLHRSYLTPGDSSSG WTAGAAAYYVGYLQPRTFLLKYNENGTITDAVDCALDPLSETKCTLKSFTV EKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIA PGQTG N IADYNYKLPDDFTGCVIAWNSNNLDSKVGGNYNYLYRLFRKSN LKPFERDISTEIYQAGSTPCNGV K GFNCYFPLQSYGFQPT Y GVGYQPY RVVVLSFELLHAPATVCGPKKSTNLVKNKCVNFNFNGLTGTGVLTESNKKF LPFQQFGRDIADTTDAVRDPQTLEILDITPCSFGGVSVITPGTNTSNQVAVL YQ G VNCTEVPVAIHADQLTPTWRVYSTGSNVFQTRAGCLIGAEHVNNSY ECDIPIGAGICASYQTQTNSPRRARSVASQSIIAYTMSLG V ENSVAYSNNS IAIPTNFTISVTTEILPVSMTKTSVDCTMYICGDSTECSNLLLQYGSFCTQLN RALTGIAVEQDKNTQEVFAQVKQIYKTPPIKDFGGFNFSQILPDPSKPSKRS FIEDLLFNKVTLADAGFIKQYGDCLGDIAARDLICAQKFNGLTVLPPLLTDEM IAQYTSALLAGTITSGWTFGAGAALQIPFAMQMAYRFNGIGVTQNVLYENQ |
| | | KLIANQFNSAIGKIQDSLSSTASALGKLQDVVNQNAQALNTLVKQLSSNFGA ISSVLNDILSRLDKVEAEVQIDRLITGRLQSLQTYVTQQLIRAAEIRASANLAA TKMSECVLGQSKRVDFCGKGYHLMSFPQSAPHGVVFLHVTYVPAQEKNF TTAPAICHDGKAHFPREGVFVSNGTHWFVTQRNFYEPQIITTDNTFVSGNC DVVIGIVNNTVYDPLQPELDSFKEELDKYFKNHTSPDVDLGDISGINASVVNI QKEIDRLNEVAKNLNESLIDLQELGKYEQYIKWPWYIWLGFIAGLIAIVMVTI MLCCMTSCCSCLKGCCSCGSCCKFDEDDSEPVLKGVKLHYT |
| 4 | B.1.617.2 (Delta) | MFVFLVLLPLVSSQCVNLRTRTQLPPAYTNSFTRGVYYPDKVFRSSVLHS TQDLFLPFFSNVTWFHAIHVSGTNGTKRFDNPVLPFNDGVYFASTEKSNIIR GWIFGTTLDSKTQSLLIVNNATNVVIKVCEFQFCNDPFLGVYYHKNNKSWM ESEVYSSANNCTFEYVSQPFLMDLEGKQGNFKNLREFVFKNIDGYFKIYSK HTPINLVRDLPQGFSALEPLVDLPIGINITRFQTLLALHRSYLTPGDSSSGWT AGAAAYYVGYLQPRTFLLKYNENGTITDAVDCALDPLSETKCTLKSFTVEK GIYQTSNFRVQPTESIVRFPNITNLCPFGEVFNATRFASVYAWNRKRISNCV ADYSVLYNSASFSTFKCYGVSPTKLNDLCFTNVYADSFVIRGDEVRQIAPG QTGKIADYNYKLPDDFTGCVIAWNSNNLDSKVGGNYNYRYRLFRKSNLK PFERDISTEIYQAGSKPCNGVEGFNCYFPLQSYGFQPTNGVGYQPYRVV VLSFELLHAPATVCGPKKSTNLVKNKCVNFNFNGLTGTGVLTESNKKFLPF QQFGRDIADTTDAVRDPQTLEILDITPCSFGGVSVITPGTNTSNQVAVLYQ GVNCTEVPVAIHADQLTPTWRVYSTGSNVFQTRAGCLIGAEHVNNSYEC DIPIGAGICASYQTQTNSRRRARSVASQSIIAYTMSLGAENSVAYSNNSIAI PTNFTISVTTEILPVSMTKTSVDCTMYICGDSTECSNLLLQYGSFCTQLNRA LTGIAVEQDKNTQEVFAQVKQIYKTPPIKDFGGFNFSQILPDPSKPSKRSFIE DLLFNKVTLADAGFIKQYGDCLGDIAARDLICAQKFNGLTVLPPLLTDEMIAQ YTSALLAGTITSGWTFGAGAALQIPFAMQMAYRFNGIGVTQNVLYENQKLIA NQFNSAIGKIQDSLSSTASALGKLQNVVNQNAQALNTLVKQLSSNFGAIS SVLNDILSRLDKVEAEVQIDRLITGRLQSLQTYVTQQLIRAAEIRASANLAAT KMSECVLGQSKRVDFCGKGYHLMSFPQSAPHGVVFLHVTYVPAQEKNFT TAPAICHDGKAHFPREGVFVSNGTHWFVTQRNFYEPQIITTDNTFVSGNCD VVIGIVNNTVYDPLQPELDSFKEELDKYFKNHTSPDVDLGDISGINASVVNIQ KEIDRLNEVAKNLNESLIDLQELGKYEQYIKWPWYIWLGFIAGLIAIVMVTIML CCMTSCCSCLKGCCSCGSCCKFDEDDSEPVLKGVKLHYT |
| 5 | B1.128 P1 (Gamma) | MFVFLVLLPLVSSQCVNFTNRTQLPSAYTNSFTRGVYYPDKVFRSSV LHSTQDLFLPFFSNVTWFHAIHVSGTNGTKRFDNPVLPFNDGVYFASTEKS NIIRGWIFGTTLDSKTQSLLIVNNATNVVIKVCEFQFCNYPFLGVYYHKNN KSWMESEFRVYSSANNCTFEYVSQPFLMDLEGKQGNFKNLSEFVFKNID GYFKIYSKHTPINLVRDLPQGFSALEPLVDLPIGINITRFQTLLALHRSYLTPG DSSSGWTAGAAAYYVGYLQPRTFLLKYNENGTITDAVDCALDPLSETKCTL KSFTVEKGIYQTSNFRVQPTESIVRFPNITNLCPFGEVFNATRFASVYAWNR KRISNCVADYSVLYNSASFSTFKCYGVSPTKLNDLCFTNVYADSFVIRGDE VRQIAPGQTGTIADYNYKLPDDFTGCVIAWNSNNLDSKVGGNYNYLYRLF RKSNLKPFERDISTEIYQAGSTPCNGVKGFNCYFPLQSYGFQPTYGVG YQPYRVVVLSFELLHAPATVCGPKKSTNLVKNKCVNFNFNGLTGTGVLTES NKKFLPFQQFGRDIADTTDAVRDPQTLEILDITPCSFGGVSVITPGTNTSNQ |
| | | VAVLYQGVNCTEVPVAIHADQLTPTWRVYSTGSNVFQTRAGCLIGAEY VNNSYECDIPIGAGICASYQTQTNSPRRARSVASQSIIAYTMSLGAENSVAY SNNSIAIPTNFTISVTTEILPVSMTKTSVDCTMYICGDSTECSNLLLQYGSFC TQLNRALTGIAVEQDKNTQEVFAQVKQIYKTPPIKDFGGFNFSQILPDPSKP SKRSFIEDLLFNKVTLADAGFIKQYGDCLGDIAARDLICAQKFNGLTVLPPLL TDEMIAQYTSALLAGTITSGWTFGAGAALQIPFAMQMAYRFNGIGVTQNVL YENQKLIANQFNSAIGKIQDSLSSTASALGKLQDVVNQNAQALNTLVKQLSS NFGAISSVLNDILSRLDKVEAEVQIDRLITGRLQSLQTYVTQQLIRAAEIRASA NLAAIKMSECVLGQSKRVDFCGKGYHLMSFPQSAPHGVVFLHVTYVPAQ EKNFTTAPAICHDGKAHFPREGVFVSNGTHWFVTQRNFYEPQIITTDNTFV SGNCDVVIGIVNNTVYDPLQPELDSFKEELDKYFKNHTSPDVDLGDISGINA SFVNIQKEIDRLNEVAKNLNESLIDLQELGKYEQYIKWPWYIWLGFIAGLIAI VMVTIMLCCMTSCCSCLKGCCSCGSCCKFDEDDSEPVLKGVKLHYT |
| 6 | C.37 (Lambda) | >QVU28103.1 surface glycoprotein [Severe acute respiratory syndrome coronavirus 2]. |
| | Position 445 can also be substituted with an R, wherein position 445 in C.37 Lambda refers to position 452 in WUHAN Sequence (SEQ ID No 1). | MFVFLVLLPLVSSQCVNLTTRTQLPPAYTNSFTRGVYYPDKVFRSSVLHST QDLFLPFFSNVTWFHAIHVSGTNVIKRFDNPVLPFNDGVYFASTEKSNIIR GWIFGTTLDSKTQSLLIVNNATNVVIKVCEFQFCNDPFLGVYYHKNNKSWM ESEFRVYSSANNCTFEYVSQPFLMDLEGKQGNFKNLREFVFKNIDGYFKIY SKHTPINLVRDLPQGFSALEPLVDLPIGINITRFQTLLALHNSSSGWTAGA AAYYVGYLQPRTFLLKYNENGTITDAVDCALDPLSETKCTLKSFTVEKGIYQ TSNFRVQPTESIVRFPNITNLCPFGEVFNATRFASVYAWNRKRISNCVADY SVLYNSASFSTFKCYGVSPTKLNDLCFTNVYADSFVIRGDEVRQIAPGQTG KIADYNYKLPDDFTGCVIAWNSNNLDSKVGGNYNYQYRLFRKSNLKPFE RDISTEIYQAGSTPCNGVEGFNCYSPLQSYGFQPTNGVGYQPYRVVVLS FELLHAPATVCGPKKSTNLVKNKCVNFNFNGLTGTGVLTESNKKFLPFQQF GRDIADTTDAVRDPQTLEILDITPCSFGGVSVITPGTNTSNQVAVLYQGV NCTEVPVAIHADQLTPTWRVYSTGSNVFQTRAGCLIGAEHVNNSYECDIPI GAGICASYQTQTNSPRRARSVASQSIIAYTMSLGAENSVAYSNNSIAIPTNF TISVTTEILPVSMTKTSVDCTMYICGDSTECSNLLLQYGSFCTQLNRALTGIA VEQDKNTQEVFAQVKQIYKTPPIKDFGGFNFSQILPDPSKPSKRSFIEDLLF NKVTLADAGFIKQYGDCLGDIAARDLICAQKFNGLNVLPPLLTDEMIAQYT SALLAGTITSGWTFGAGAALQIPFAMQMAYRFNGIGVTQNVLYENQKLIAN QFNSAIGKIQDSLSSTASALGKLQDVVNQNAQALNTLVKQLSSNFGAISSVL NDILSRLDKVEAEVQIDRLITGRLQSLQTYVTQQLIRAAEIRASANLAATKMS ECVLGQSKRVDFCGKGYHLMSFPQSAPHGVVFLHVTYVPAQEKNFTTAPA ICHDGKAHFPREGVFVSNGTHWFVTQRNFYEPQIITTDNTFVSGNCDVVIGI VNNTVYDPLQPELDSFKEELDKYFKNHTSPDVDLGDISGINASVVNIQKEID RLNEVAKNLNESLIDLQELGKYEQYIKWPWYIWLGFIAGLIAIVMVTIMLCCM TSCCSCLKGCCSCGSCCKFDEDDSEPVLKGVKLHYT |
| | This sequence comprises a 7AA deletion compared to SEQ IQ No 1, at position 246-252. In some databases, this deletion is characterized by the alternative numbering of 247-253. | |
| 7 | Vaccine gene ABG comprising SARS-CoV-2 variants alpha, beta, and gamma | MFVFLVLLPLVSSQCVNFTNRTQLPSAYTNSFTRGVYYPDKVFRSSV LHSTQDLFLPFFSNVTWFHAISGTNGTKRFDNPVLPFNDGVYFASTEKSNII RGWIFGTTLDSKTQSLLIVNNATNVVIKVCEFQFCNYPFLGVYHKNNKSW |
| | | MESEFRVYSSANNCTFEYVSQPFLMDLEGKQGNFKNLSEFVFKNIDGYF KIYSKHTPINLVRDLPQGFSALEPLVDLPIGINITRFQTLLALHRSYLTPGDSS SGWTAGAAAYYVGYLQPRTFLLKYNENGTITDAVDCALDPLSETKCTLKSF TVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQ IAPGQTGTIADYNYKLPDDFTGCVIAWNSNNLDSKVGGNYNYLYRLFRKS NLKPFERDISTEIYQAGSTPCNGVKGFNCYFPLQSYGFQPTYGVGYQP YRVVVLSFELLHAPATVCGPKKSTNLVKNKCVNFNFNGLTGTGVLTESNKK FLPFQQFGRDIDDTTDAVRDPQTLEILDITPCSFGGVSVITPGTNTSNQVA VLYQGVNCTEVPVAIHADQLTPTWRVYSTGSNVFQTRAGCLIGAEYVN NSYECDIPIGAGICASYQTQTNSHRRARSVASQSIIAYTMSLGAENSVAYS NNSIAIPINFTISVTTEILPVSMTKTSVDCTMYICGDSTECSNLLLQYGSFCT QLNRALTGIAVEQDKNTQEVFAQVKQIYKTPPIKDFGGFNFSQILPDPSKPS KRSPIEDLLFNKVTLADAGFIKQYGDCLGDIAARDLICAQKFNGLTVLPPLL TDEMIAQYTSALLAGTITSGWTFGAGPALQIPFPMQMAYRFNGIGVTQN VLYENQKLIANQFNSAIGKIQDSLSSTPSALGKLQDVVNQNAQALNTLVK QLSSNFGAISSVLNDILARLDPPEAEVQIDRLITGRLQSLQTYVTQQLI RAAEIRASANLAAIKMSECVLGQSKRVDFCGKGYHLMSFPQSAPHGVVFL HVTYVPAQEKNFTTAPAICHDGKAHFPREGVFVSNGTHWFVTQRNFYEPQ IITTHNTFVSGNCDVVIGIVNNTVYDPLQPELDSFKEELDKYFKNHTSPDV DLGDISGINASVVNIQKEIDRLNEVAKNLNESLIDLQELGKYEQYIKWPWYIW LGFIAGLIAIVMVTIMLCCMTSCCSCLKGCCSCGSCCKFDEDDSEPVLKGV KLHYT |
| 8 | Vaccine gene ABGD comprising SARS-CoV-2 variants alpha, beta, gamma, and delta | MFVFLVLLPLVSSQCVNFTNRTQLPSAYTNSFTRGVYYPDKVFRSSV LHSTQDLFLPFFSNVTWFHAISGTNGTKRFDNPVLPFNDGVYFASTEKSNII RGWIFGTTLDSKTQSLLIVNNATNVVIKVCEFQFCNYPFLGVYHKNNKSW MESEFRVYSSANNCTFEYVSQPFLMDLEGKQGNFKNLSEFVFKNIDGYF KIYSKHTPINLVRDLPQGFSALEPLVDLPIGINITRFQTLLALHRSYLTPGDSS SGWTAGAAAYYVGYLQPRTFLLKYNENGTITDAVDCALDPLSETKCTLKSF TVEKGIYQTSNFRVQPTESIVRFPNITNLCPFGEVFNATRFASVYAWNRKRI SNCVADYSVLYNSASFSTFKCYGVSPTKLNDLCFTNVYADSFVIRGDEVRQ IAPGQTGTIADYNYKLPDDFTGCVIAWNSNNLDSKVGGNYNYLYRLFRKS NLKPFERDISTEIYQAGSTPCNGVKGFNCYFPLQSYGFQPTYGVGYQP YRVVVLSFELLHAPATVCGPKKSTNLVKNKCVNFNFNGLTGTGVLTESNKK FLPFQQFGRDIDDTTDAVRDPQTLEILDITPCSFGGVSVITPGTNTSNQVA |
| | | VLYQGVNCTEVPVAIHADQLTPTWRVYSTGSNVFQTRAGCLIGAEYVN NSYECDIPIGAGICASYQTQTNSHRRARSVASQSIIAYTMSLGAENSVAYS NNSIAIPINFTISVTTEILPVSMTKTSVDCTMYICGDSTECSNLLLQYGSFCT QLNRALTGIAVEQDKNTQEVFAQVKQIYKTPPIKDFGGFNFSQILPDPSKPS KRSPIEDLLFNKVTLADAGFIKQYGDCLGDIAARDLICAQKFNGLTVLPPLL TDEMIAQYTSALLAGTITSGWTFGAGPALQIPFPMQMAYRFNGIGVTQN VLYENQKLIANQFNSAIGKIQDSLSSTPSALGKLQDVVNQNAQALNTLVK QLSSNFGAISSVLNDILARLDPPEAEVQIDRLITGRLQSLQTYVTQQLI RAAEIRASANLAAIKMSECVLGQSKRVDFCGKGYHLMSFPQSAPHGVVFL HVTYVPAQEKNFTTAPAICHDGKAHFPREGVFVSNGTHWFVTQRNFYEPQ IITTHNTFVSGNCDVVIGIVNNTVYDPLQPELDSFKEELDKYFKNHTSPDV DLGDISGINASVVNIQKEIDRLNEVAKNLNESLIDLQELGKYEQYIKWPWYIW LGFIAGLIAIVMVTIMLCCMTSCCSCLKGCCSCGSCCKFDEDDSEPVLKGV KLHYT |
| 9 | Vaccine gene ABGDL comprising SARS-CoV-2 variants alpha, beta, gamma, delta and lambda | MFVFLVLLPLVSSQCVNFRNRTQLPSAYTNSFTRGVYYPDKVFRSS VLHSTQDLFLPFFSNVTWFHAISGTNVIKRFDNPVLPFNDGVYFASTEKS NIIRGWIFGTTLDSKTQSLLIVNNATNVVIKVCEFQFCNYPFLGVYHKNNK SWMESGVYSSANNCTFEYVSQPFLMDLEGKQGNFKNLSEFVFKNIDG YFKIYSKHTPINLVRGLPQGFSALEPLVDLPIGINITRFQTLLALHNSSSG WTAGAAAYYVGYLQPRTFLLKYNENGTITDAVDCALDPLSETKCTLKSFTV EKGIYQTSNFRVQPTESIVRFPNITNLCPFGEVFNATRFASVYAWNRKRISN CVADYSVLYNSASFSTFKCYGVSPTKLNDLCFTNVYADSFVIRGDEVRQIA PGQTGTIADYNYKLPDDFTGCVIAWNSNNLDSKVGGNYNYRYRLFRKS NLKPFERDISTEIYQAGSKPCNGVQGFNCYSPLQSYGFQPTYGVG YQPYRVVVLSFELLHAPATVCGPKKSTNLVKNKCVNFNFNGLTGTGVLTES NKKFLPFQQFGRDIADTTDAVRDPQTLEILDITPCSFGGVSVITPGTNTSNQ VAVLYQGVNCTEVPVAIHADQLTPTWRVYSTGSNVFQTRAGCLIGAEY VNNSYECDIPIGAGICASYQTQTNSRRRARSVASQSIIAYTMSLGAENSV AYSNNSIAIPINFTISVTTEILPVSMTKTSVDCTMYICGDSTECSNLLLQYGS FCTQLNRALTGIAVEQDKNTQEVFAQVKQIYKTPPIKDFGGFNFSQILPDPS KPSKRSPIEDLLFNKVTLADAGFIKQYGDCLGDIAARDLICAQKFNGLNV LPPLLTDEMIAQYTSALLAGTITSGWTFGAGPALQIPFPMQMAYRFNGI |
| | | GVTQNVLYENQKLIANQFNSAIGKIQDSLSSTPSALGKLQNVVNQNAQA LNTLVKQLSSNFGAISSVLNDILARLDPPEAEVQIDRLITGRLQSLQTY VTQQLIRAAEIRASANLAAIKMSECVLGQSKRVDFCGKGYHLMSFPQSAP HGVVFLHVTYVPAQEKNFTTAPAICHDGKAHFPREGVFVSNGTHWFVTQR NFYEPQIITTHNTFVSGNCDVVIGIVNNTVYDPLQPELDSFKEELDKYFKN HTSPDVDLGDISGINASFVNIQKEIDRLNEVAKNLNESLIDLQELGKYEQYI KWPWYIWLGFIAGLIAIVMVTIMLCCMTSCCSCLKGCCSCGSCCKFDEDDS EPVLKGVKLHYT |
| 10 | Vaccine gene ABGD ver2 comprising SARS-CoV-2 variants alpha, beta, gamma, and delta | MFVFLVLLPLVSSQCVNFRNRTQLPSAYTNSFTRGVYYPDKVFRSS VLHSTQDLFLPFFSNVTWFHAISGTNGTKRFDNPVLPFNDGVYFASTEKSN IIRGWIFGTTLDSKTQSLLIVNNATNVVIKVCEFQFCNYPFLGVYHKNNKS WMESEVYSSANNCTFEYVSQPFLMDLEGKQGNFKNLSEFVFKNIDGYFK IYSKHTPINLVRDLPQGFSALEPLVDLPIGINITRFQTLLALHRSYLTPGDSSS GWTAGAAAYYVGYLQPRTFLLKYNENGTITDAVDCALDPLSETKCTLKSFT VEKGIYQTSNFRVQPTESIVRFPNITNLCPFGEVFNATRFASVYAWNRKRIS NCVADYSVLYNSASFSTFKCYGVSPTKLNDLCFTNVYADSFVIRGDEVRQI APGQTGTIADYNYKLPDDFTGCVIAWNSNNLDSKVGGNYNYRYRLFRK SNLKPFERDISTEIYQAGSKPCNGVKGFNCYFPLQSYGFQPTYGVGY QPYRVVVLSFELLHAPATVCGPKKSTNLVKNKCVNFNFNGLTGTGVLTESN KKFLPFQQFGRDIDDTTDAVRDPQTLEILDITPCSFGGVSVITPGTNTSNQ VAVLYQGVNCTEVPVAIHADQLTPTWRVYSTGSNVFQTRAGCLIGAEY VNNSYECDIPIGAGICASYQTQTNSHRRARSVASQSIIAYTMSLGAENSV AYSNNSIAIPINFTISVTTEILPVSMTKTSVDCTMYICGDSTECSNLLLQYGS FCTQLNRALTGIAVEQDKNTQEVFAQVKQIYKTPPIKDFGGFNFSQILPDPS KPSKRSPIEDLLFNKVTLADAGFIKQYGDCLGDIAARDLICAQKFNGLTVL PPLLTDEMIAQYTSALLAGTITSGWTFGAGPALQIPFPMQMAYRFNGIG VTQNVLYENQKLIANQFNSAIGKIQDSLSSTPSALGKLQNVVNQNAQAL NTLVKQLSSNFGAISSVLNDILARLDPPEAEVQIDRLITGRLQSLQTYV TQQLIRAAEIRASANLAAIKMSECVLGQSKRVDFCGKGYHLMSFPQSAPH GVVFLHVTYVPAQEKNFTTAPAICHDGKAHFPREGVFVSNGTHWFVTQRN FYEPQIITTHNTFVSGNCDVVIGIVNNTVYDPLQPELDSFKEELDKYFKNH TSPDVDLGDISGINASVVNIQKEIDRLNEVAKNLNESLIDLQELGKYEQYIKW |
| | | PWYIWLGFIAGLIAIVMVTIMLCCMTSCCSCLKGCCSCGSCCKFDEDDSEP VLKGVKLHYT |
| 11 | B1.617.1 (Delta Plus) | MFVFLVLLPLVSSQCVNLRTRTQLPPAYTNSFTRGVYYPDKVFRSSVLHS TQDLFLPFFSNVTWFHAIHVSGTNGTKRFDNPVLPFNDGVYFASTEKSNIIR GWIFGTTLDSKTQSLLIVNNATNVVIKVCEFQFCNDPFLGVYYHKNNKSWM ESEVYSSANNCTFEYVSQPFLMDLEGKQGNFKNLREFVFKNIDGYFKIYSK HTPINLVRDLPQGFSALEPLVDLPIGINITRFQTLLALHRSYLTPGDSSSGWT AGAAAYYVGYLQPRTFLLKYNENGTITDAVDCALDPLSETKCTLKSFTVEK GIYQTSNFRVQPTESIVRFPNITNLCPFGEVFNATRFASVYAWNRKRISNCV ADYSVLYNSASFSTFKCYGVSPTKLNDLCFTNVYADSFVIRGDEVRQIAPG QTGNIADYNYKLPDDFTGCVIAWNSNNLDSKVGGNYNYRYRLFRKSNL KPFERDISTEIYQAGSKPCNGVEGFNCYFPLQSYGFQPTNGVGYQPYRV VVLSFELLHAPATVCGPKKSTNLVKNKCVNFNFNGLTGTGVLTESNKKFLP FQQFGRDIADTTDAVRDPQTLEILDITPCSFGGVSVITPGTNTSNQVAVLYQ GVNCTEVPVAIHADQLTPTWRVYSTGSNVFQTRAGCLIGAEHVNNSYEC DIPIGAGICASYQTQTNSRRRARSVASQSIIAYTMSLGAENSVAYSNNSIAI PTNFTISVTTEILPVSMTKTSVDCTMYICGDSTECSNLLLQYGSFCTQLNRA LTGIAVEQDKNTQEVFAQVKQIYKTPPIKDFGGFNFSQILPDPSKPSKRSFIE DLLFNKVTLADAGFIKQYGDCLGDIAARDLICAQKFNGLTVLPPLLTDEMIAQ YTSALLAGTITSGWTFGAGAALQIPFAMQMAYRFNGIGVTQNVLYENQKLIA NQFNSAIGKIQDSLSSTASALGKLQNVVNQNAQALNTLVKQLSSNFGAIS SVLNDILSRLDKVEAEVQIDRLITGRLQSLQTYVTQQLIRAAEIRASANLAAT KMSECVLGQSKRVDFCGKGYHLMSFPQSAPHGVVFLHVTYVPAQEKNFT TAPAICHDGKAHFPREGVFVSNGTHWFVTQRNFYEPQIITTDNTFVSGNCD VVIGIVNNTVYDPLQPELDSFKEELDKYFKNHTSPDVDLGDISGINASVVNIQ KEIDRLNEVAKNLNESLIDLQELGKYEQYIKWPWYIWLGFIAGLIAIVMVTIML CCMTSCCSCLKGCCSCGSCCKFDEDDSEPVLKGVKLHYT |

**Preferred nucleic acid sequences of the invention:**

| **SEQ ID** | **Info** | **Data base entry (NCBI Genome)** |
|---|---|---|
| 12 | Spike protein coding WUHAN Sequence nucleotides | NC_045512.2 Severe acute respiratory syndrome coronavirus 2 isolate Wuhan-Hu-1, complete genome : [Spike protein: 21563..25384] |
| | | ATGTTTGTTTTTCTTGTTTTATTGCCACTAGTCTCTAGTCAGTGTGTTAATCTTAC AACCAGAACTCAATTACCCCCTGCATACACTAATTCTTTCACACGTGGTGTTTA TTACCCTGACAAAGTTTTCAGATCCTCAGTTTTACATTCAACTCAGGACTTGTTC TTACCTTTCTTTTCCAATGTTACTTGGTTCCATGCTATACATGTCTCTGGGACCA ATGGTACTAAGAGGTTTGATAACCCTGTCCTACCATTTAATGATGGTGTTTATTT TGCTTCCACTGAGAAGTCTAACATAATAAGAGGCTGGATTTTTGGTACTACTTT AGATTCGAAGACCCAGTCCCTACTTATTGTTAATAACGCTACTAATGTTGTTATT AAAGTCTGTGAATTTCAATTTTGTAATGATCCATTTTTGGGTGTTTATTACCACA AAAACAACAAAAGTTGGATGGAAAGTGAGTTCAGAGTTTATTCTAGTGCGAATA ATTGCACTTTTGAATATGTCTCTCAGCCTTTTCTTATGGACCTTGAAGGAAAACA GGGTAATTTCAAAAATCTTAGGGAATTTGTGTTTAAGAATATTGATGGTTATTTT AAAATATATTCTAAGCACACGCCTATTAATTTAGTGCGTGATCTCCCTCAGGGT TTTTCGGCTTTAGAACCATTGGTAGATTTGCCAATAGGTATTAACATCACTAGG |
| | | TTTCAAACTTTACTTGCTTTACATAGAAGTTATTTGACTCCTGGTGATTCTTCTT CAGGTTGGACAGCTGGTGCTGCAGCTTATTATGTGGGTTATCTTCAACCTAGG ACTTTTCTATTAAAATATAATGAAAATGGAACCATTACAGATGCTGTAGACTGTG CACTTGACCCTCTCTCAGAAACAAAGTGTACGTTGAAATCCTTCACTGTAGAAA AAGGAATCTATCAAACTTCTAACTTTAGAGTCCAACCAACAGAATCTATTGTTAG ATTTCCTAATATTACAAACTTGTGCCCTTTTGGTGAAGTTTTTAACGCCACCAGA TTTGCATCTGTTTATGCTTGGAACAGGAAGAGAATCAGCAACTGTGTTGCTGAT TATTCTGTCCTATATAATTCCGCATCATTTTCCACTTTTAAGTGTTATGGAGTGT CTCCTACTAAATTAAATGATCTCTGCTTTACTAATGTCTATGCAGATTCATTTGT AATTAGAGGTGATGAAGTCAGACAAATCGCTCCAGGGCAAACTGGAAAGATTG CTGATTATAATTATAAATTACCAGATGATTTTACAGGCTGCGTTATAGCTTGGAA TTCTAACAATCTTGATTCTAAGGTTGGTGGTAATTATAATTACCTGTATAGATTG TTTAGGAAGTCTAATCTCAAACCTTTTGAGAGAGATATTTCAACTGAAATCTATC AGGCCGGTAGCACACCTTGTAATGGTGTTGAAGGTTTTAATTGTTACTTTCCTT TACAATCATATGGTTTCCAACCCACTAATGGTGTTGGTTACCAACCATACAGAG TAGTAGTACTTTCTTTTGAACTTCTACATGCACCAGCAACTGTTTGTGGACCTAA AAAGTCTACTAATTTGGTTAAAAACAAATGTGTCAATTTCAACTTCAATGGTTTA ACAGGCACAGGTGTTCTTACTGAGTCTAACAAAAAGTTTCTGCCTTTCCAACAA TTTGGCAGAGACATTGCTGACACTACTGATGCTGTCCGTGATCCACAGACACT TGAGATTCTTGACATTACACCATGTTCTTTTGGTGGTGTCAGTGTTATAACACC AGGAACAAATACTTCTAACCAGGTTGCTGTTCTTTATCAGGATGTTAACTGCAC AGAAGTCCCTGTTGCTATTCATGCAGATCAACTTACTCCTACTTGGCGTGTTTA TTCTACAGGTTCTAATGTTTTTCAAACACGTGCAGGCTGTTTAATAGGGGCTGA ACATGTCAACAACTCATATGAGTGTGACATACCCATTGGTGCAGGTATATGCGC TAGTTATCAGACTCAGACTAATTCTCCTCGGCGGGCACGTAGTGTAGCTAGTC AATCCATCATTGCCTACACTATGTCACTTGGTGCAGAAAATTCAGTTGCTTACT CTAATAACTCTATTGCCATACCCACAAATTTTACTATTAGTGTTACCACAGAAAT TCTACCAGTGTCTATGACCAAGACATCAGTAGATTGTACAATGTACATTTGTGG TGATTCAACTGAATGCAGCAATCTTTTGTTGCAATATGGCAGTTTTTGTACACAA TTAAACCGTGCTTTAACTGGAATAGCTGTTGAACAAGACAAAAACACCCAAGAA GTTTTTGCACAAGTCAAACAAATTTACAAAACACCACCAATTAAAGATTTTGGTG GTTTTAATTTTTCACAAATATTACCAGATCCATCAAAACCAAGCAAGAGGTCATT TATTGAAGATCTACTTTTCAACAAAGTGACACTTGCAGATGCTGGCTTCATCAA ACAATATGGTGATTGCCTTGGTGATATTGCTGCTAGAGACCTCATTTGTGCACA AAAGTTTAACGGCCTTACTGTTTTGCCACCTTTGCTCACAGATGAAATGATTGC TCAATACACTTCTGCACTGTTAGCGGGTACAATCACTTCTGGTTGGACCTTTGG TGCAGGTGCTGCATTACAAATACCATTTGCTATGCAAATGGCTTATAGGTTTAA TGGTATTGGAGTTACACAGAATGTTCTCTATGAGAACCAAAAATTGATTGCCAA CCAATTTAATAGTGCTATTGGCAAAATTCAAGACTCACTTTCTTCCACAGCAAGT GCACTTGGAAAACTTCAAGATGTGGTCAACCAAAATGCACAAGCTTTAAACACG CTTGTTAAACAACTTAGCTCCAATTTTGGTGCAATTTCAAGTGTTTTAAATGATA TCCTTTCACGTCTTGACAAAGTTGAGGCTGAAGTGCAAATTGATAGGTTGATCA CAGGCAGACTTCAAAGTTTGCAGACATATGTGACTCAACAATTAATTAGAGCTG CAGAAATCAGAGCTTCTGCTAATCTTGCTGCTACTAAAATGTCAGAGTGTGTAC TTGGACAATCAAAAAGAGTTGATTTTTGTGGAAAGGGCTATCATCTTATGTCCT TCCCTCAGTCAGCACCTCATGGTGTAGTCTTCTTGCATGTGACTTATGTCCCTG CACAAGAAAAGAACTTCACAACTGCTCCTGCCATTTGTCATGATGGAAAAGCAC ACTTTCCTCGTGAAGGTGTCTTTGTTTCAAATGGCACACACTGGTTTGTAACAC AAAGGAATTTTTATGAACCACAAATCATTACTACAGACAACACATTTGTGTCTGG TAACTGTGATGTTGTAATAGGAATTGTCAACAACACAGTTTATGATCCTTTGCAA CCTGAATTAGACTCATTCAAGGAGGAGTTAGATAAATATTTTAAGAATCATACAT CACCAGATGTTGATTTAGGTGACATCTCTGGCATTAATGCTTCAGTTGTAAACA TTCAAAAAGAAATTGACCGCCTCAATGAGGTTGCCAAGAATTTAAATGAATCTC TCATCGATCTCCAAGAACTTGGAAAGTATGAGCAGTATATAAAATGGCCATGGT ACATTTGGCTAGGTTTTATAGCTGGCTTGATTGCCATAGTAATGGTGACAATTA TGCTTTGCTGTATGACCAGTTGCTGTAGTTGTCTCAAGGGCTGTTGTTCTTGTG GATCCTGCTGCAAATTTGATGAAGACGACTCTGAGCCAGTGCTCAAAGGAGTC AAATTACATTACACATA |
| 13 | B1.1.7 (Alpha) nucleotides | MW487270.1 Severe acute respiratory syndrome coronavirus 2 isolate SARS-CoV-2/human/USA/NYI.B1-7.01-21/2021, complete genome : [Spike protein: 21503..25315] |
| | | ATGTTTGTTTTTCTTGTTTTATTGCCACTAGTCTCTAGTCAGTGTGTTAATCTTAC AACCAGAACTCAATTACCCCCTGCATACACTAATTCTTTCACACGTGGTGTTTA TTACCCTGACAAAGTTTTCAGATCCTCAGTTTTACATTCAACTCAGGACTTGTTC TTACCTTTCTTTTCCAATGTTACTTGGTTCCATGCTATCTCTGGGACCAATGGTA CTAAGAGGTTTGATAACCCTGTCCTACCATTTAATGATGGTGTTTATTTTGCTTC CACTGAGAAGTCTAACATAATAAGAGGCTGGATTTTTGGTACTACTTTAGATTC GAAGACCCAGTCCCTACTTATTGTTAATAACGCTACTAATGTTGTTATTAAAGTC TGTGAATTTCAATTTTGTAATGATCCATTTTTGGGTGTTTACCACAAAAACAACA AAAGTTGGATGGAAAGTGAGTTCAGAGTTTATTCTAGTGCGAATAATTGCACTT TTGAATATGTCTCTCAGCCTTTTCTTATGGACCTTGAAGGAAAACAGGGTAATT TCAAAAATCTTAGGGAATTTGTGTTTAAGAATATTGATGGTTATTTTAAAATATAT TCTAAGCACACGCCTATTAATTTAGTGCGTGATCTCCCTCAGGGTTTTTCGGCT TTAGAACCATTGGTAGATTTGCCAATAGGTATTAACATCACTAGGTTTCAAACTT TACTTGCTTTACATAGAAGTTATTTGACTCCTGGTGATTCTTCTTCAGGTTGGAC AGCTGGTGCTGCAGCTTATTATGTGGGTTATCTTCAACCTAGGACTTTTCTATT AAAATATAATGAAAATGGAACCATTACAGATGCTGTAGACTGTGCACTTGACCC TCTCTCAGAAACAAAGTGTACGTTGAAATCCTTCACTGTAGAAAAAGGAATCTA TCAAACTTCTAACTTTAGAGTCCAACCAACAGAATCTATTGTTAGATTTCCTAAT ATTACAAACTTGTGCCCTTTTGGTGAAGTTTTTAACGCCACCAGATTTGCATCT GTTTATGCTTGGAACAGGAAGAGAATCAGCAACTGTGTTGCTGATTATTCTGTC CTATATAATTCCGCATCATTTTCCACTTTTAAGTGTTATGGAGTGTCTCCTACTA AATTAAATGATCTCTGCTTTACTAATGTCTATGCAGATTCATTTGTAATTAGAGG TGATGAAGTCAGACAAATCGCTCCAGGGCAAACTGGAAAGATTGCTGATTATA ATTATAAATTACCAGATGATTTTACAGGCTGCGTTATAGCTTGGAATTCTAACAA TCTTGATTCTAAGGTTGGTGGTAATTATAATTACCTGTATAGATTGTTTAGGAAG TCTAATCTCAAACCTTTTGAGAGAGATATTTCAACTGAAATCTATCAGGCCGGT AGCACACCTTGTAATGGTGTTGAAGGTTTTAATTGTTACTTTCCTTTACAATCAT ATGGTTTCCAACCCACTTATGGTGTTGGTTACCAACCATACAGAGTAGTAGTAC TTTCTTTTGAACTTCTACATGCACCAGCAACTGTTTGTGGACCTAAAAAGTCTAC TAATTTGGTTAAAAACAAATGTGTCAATTTCAACTTCAATGGTTTAACAGGCACA GGTGTTCTTACTGAGTCTAACAAAAAGTTTCTGCCTTTCCAACAATTTGGCAGA GACATTGATGACACTACTGATGCTGTCCGTGATCCACAGACACTTGAGATTCTT GACATTACACCATGTTCTTTTGGTGGTGTCAGTGTTATAACACCAGGAACAAAT ACTTCTAACCAGGTTGCTGTTCTTTATCAGGGTGTTAACTGCACAGAAGTCCCT GTTGCTATTCATGCAGATCAACTTACTCCTACTTGGCGTGTTTATTCTACAGGTT CTAATGTTTTTCAAACACGTGCAGGCTGTTTAATAGGGGCTGAACATGTCAACA ACTCATATGAGTGTGACATACCCATTGGTGCAGGTATATGCGCTAGTTATCAGA CTCAGACTAATTCTCATCGGCGGGCACGTAGTGTAGCTAGTCAATCCATCATT GCCTACACTATGTCACTTGGTGCAGAAAATTCAGTTGCTTACTCTAATAACTCT ATTGCCATACCCATAAATTTTACTATTAGTGTTACCACAGAAATTCTACCAGTGT CTATGACCAAGACATCAGTAGATTGTACAATGTACATTTGTGGTGATTCAACTG AATGCAGCAATCTTTTGTTGCAATATGGCAGTTTTTGTACACAATTAAACCGTG CTTTAACTGGAATAGCTGTTGAACAAGACAAAAACACCCAAGAAGTTTTTGCAC AAGTCAAACAAATTTACAAAACACCACCAATTAAAGATTTTGGTGGTTTTAATTT TTCACAAATATTACCAGATCCATCAAAACCAAGCAAGAGGTCATTTATTGAAGA TCTACTTTTCAACAAAGTGACACTTGCAGATGCTGGCTTCATCAAACAATATGG TGATTGCCTTGGTGATATTGCTGCTAGAGACCTCATTTGTGCACAAAAGTTTAA CGGCCTTACTGTTTTGCCACCTTTGCTCACAGATGAAATGATTGCTCAATACAC TTCTGCACTGTTAGCGGGTACAATCACTTCTGGTTGGACCTTTGGTGCAGGTG CTGCATTACAAATACCATTTGCTATGCAAATGGCTTATAGGTTTAATGGTATTGG AGTTACACAGAATGTTCTCTATGAGAACCAAAAATTGATTGCCAACCAATTTAAT AGTGCTATTGGCAAAATTCAAGACTCACTTTCTTCCACAGCAAGTGCACTTGGA AAACTTCAAGATGTGGTCAACCAAAATGCACAAGCTTTAAACACGCTTGTTAAA CAACTTAGCTCCAATTTTGGTGCAATTTCAAGTGTTTTAAATGATATCCTTGCAC GTCTTGACAAAGTTGAGGCTGAAGTGCAAATTGATAGGTTGATCACAGGCAGA |
| | | CTTCAAAGTTTGCAGACATATGTGACTCAACAATTAATTAGAGCTGCAGAAATC AGAGCTTCTGCTAATCTTGCTGCTACTAAAATGTCAGAGTGTGTACTTGGACAA TCAAAAAGAGTTGATTTTTGTGGAAAGGGCTATCATCTTATGTCCTTCCCTCAG TCAGCACCTCATGGTGTAGTCTTCTTGCATGTGACTTATGTCCCTGCACAAGAA AAGAACTTCACAACTGCTCCTGCCATTTGTCATGATGGAAAAGCACACTTTCCT CGTGAAGGTGTCTTTGTTTCAAATGGCACACACTGGTTTGTAACACAAAGGAAT TTTTATGAACCACAAATCATTACTACACACAACACATTTGTGTCTGGTAACTGTG ATGTTGTAATAGGAATTGTCAACAACACAGTTTATGATCCTTTGCAACCTGAATT AGACTCATTCAAGGAGGAGTTAGATAAATATTTTAAGAATCATACATCACCAGA TGTTGATTTAGGTGACATCTCTGGCATTAATGCTTCAGTTGTAAACATTCAAAAA GAAATTGACCGCCTCAATGAGGTTGCCAAGAATTTAAATGAATCTCTCATCGAT CTCCAAGAACTTGGAAAGTATGAGCAGTATATAAAATGGCCATGGTACATTTGG CTAGGTTTTATAGCTGGCTTGATTGCCATAGTAATGGTGACAATTATGCTTTGC TGTATGACCAGTTGCTGTAGTTGTCTCAAGGGCTGTTGTTCTTGTGGATCCTGC TGCAAATTTGATGAAGACGACTCTGAGCCAGTGCTCAAAGGAGTCAAATTACAT TACACATAA |
| 14 | B1.351 (Beta) nucleotides | MZ433432.1 Severe acute respiratory syndrome coronavirus 2 isolate SARS-CoV-2/Vero E6 cells/DEU/B.1.351/2021, complete genome : [Spike protein: 21544-25356] |
| | | ATGTTTGTTTTTCTTGTTTTATTGCCACTAGTCTCTAGTCAGTGTGTTAATTTTAC AACCAGAACTCAATTACCCCCTGCATACACTAATTCTTTCACACGTGGTGTTTA TTACCCTGACAAAGTTTTCAGATCCTCAGTTTTACATTCAACTCAGGACTTGTTC TTACCTTTCTTTTCCAATGTTACTTGGTTCCATGCTATACATGTCTCTGGGACCA ATGGTACTAAGAGGTTTGCTAACCCTGTCCTACCATTTAATGATGGTGTTTATTT TGCTTCCACTGAGAAGTCTAACATAATAAGAGGCTGGATTTTTGGTACTACTTT AGATTCGAAGACCCAGTCCCTACTTATTGTTAATAACGCTACTAATGTTGTTATT AAAGTCTGTGAATTTCAATTTTGTAATGATCCATTTTTGGGTGTTTATTACCACA AAAACAACAAAAGTTGGATGGAAAGTGAGTTCAGAGTTTATTCTAGTGCGAATA ATTGCACTTTTGAATATGTCTCTCAGCCTTTTCTTATGGACCTTGAAGGAAAACA GGGTAATTTCAAAAATCTTAGGGAATTTGTGTTTAAGAATATTGATGGTTATTTT AAAATATATTCTAAGCACACGCCTATTAATTTAGTGCGTGGTCTCCCTCAGGGT TTTTCGGCTTTAGAACCATTGGTAGATTTGCCAATAGGTATTAACATCACTAGG TTTCAAACTTTACATAGAAGTTATTTGACTCCTGGTGATTCTTCTTCAGGTTGGA CAGCTGGTGCTGCAGCTTATTATGTGGGTTATCTTCAACCTAGGACTTTTCTAT TAAAATATAATGAAAATGGAACCATTACAGATGCTGTAGACTGTGCACTTGACC CTCTCTCAGAAACAAAGTGTACGTTGAAATCCTTCACTGTAGAAAAAGGAATCT ATCAAACTTCTAACTTTAGAGTCCAACCAACAGAATCTATTGTTAGATTTCCTAA TATTACAAACTTGTGCCCTTTTGGTGAAGTTTTTAACGCCACCAGATTTGCATCT GTTTATGCTTGGAACAGGAAGAGAATCAGCAACTGTGTTGCTGATTATTCTGTC CTATATAATTCCGCATCATTTTCCACTTTTAAGTGTTATGGAGTGTCTCCTACTA AATTAAATGATCTCTGCTTTACTAATGTCTATGCAGATTCATTTGTAATTAGAGG TGATGAAGTCAGACAAATCGCTCCAGGGCAAACTGGAAATATTGCTGATTATAA TTATAAATTACCAGATGATTTTACAGGCTGCGTTATAGCTTGGAATTCTAACAAT CTTGATTCTAAGGTTGGTGGTAATTATAATTACCTGTATAGATTGTTTAGGAAGT CTAATCTCAAACCTTTTGAGAGAGATATTTCAACTGAAATCTATCAGGCCGGTA GCACACCTTGTAATGGTGTTAAAGGTTTTAATTGTTACTTTCCTTTACAATCATA TGGTTTCCAACCCACTTATGGTGTTGGTTACCAACCATACAGAGTAGTAGTACT TTCTTTTGAACTTCTACATGCACCAGCAACTGTTTGTGGACCTAAAAAGTCTACT AATTTGGTTAAAAACAAATGTGTCAATTTCAACTTCAATGGTTTAACAGGCACAG GTGTTCTTACTGAGTCTAACAAAAAGTTTCTGCCTTTCCAACAATTTGGCAGAG ACATTGCTGACACTACTGATGCTGTCCGTGATCCACAGACACTTGAGATTCTTG ACATTACACCATGTTCTTTTGGTGGTGTCAGTGTTATAACACCAGGAACAAATA CTTCTAACCAGGTTGCTGTTCTTTATCAGGGTGTTAACTGCACAGAAGTCCCTG TTGCTATTCATGCAGATCAACTTACTCCTACTTGGCGTGTTTATTCTACAGGTTC TAATGTTTTTCAAACACGTGCAGGCTGTTTAATAGGGGCTGAACATGTCAACAA CTCATATGAGTGTGACATACCCATTGGTGCAGGTATATGCGCTAGTTATCAGAC TCAGACTAATTCTCCTCGGCGGGCACGTAGTGTAGCTAGTCAATCCATCATTG |
| | | CCTACACTATGTCACTTGGTGTAGAAAATTCAGTTGCTTACTCTAATAACTCTAT TGCCATACCCACAAATTTTACTATTAGTGTTACCACAGAAATTCTACCAGTGTCT ATGACCAAGACATCAGTAGATTGTACAATGTACATTTGTGGTGATTCAACTGAA TGCAGCAATCTTTTGTTGCAATATGGCAGTTTTTGTACACAATTAAACCGTGCTT TAACTGGAATAGCTGTTGAACAAGACAAAAACACCCAAGAAGTTTTTGCACAAG TCAAACAAATTTACAAAACACCACCAATTAAAGATTTTGGTGGTTTTAATTTTTC ACAAATATTACCAGATCCATCAAAACCAAGCAAGAGGTCATTTATTGAAGATCT ACTTTTCAACAAAGTGACACTTGCAGATGCTGGCTTCATCAAACAATATGGTGA TTGCCTTGGTGATATTGCTGCTAGAGACCTCATTTGTGCACAAAAGTTTAACGG CCTTACTGTTTTGCCACCTTTGCTCACAGATGAAATGATTGCTCAATACACTTCT GCACTGTTAGCGGGTACAATCACTTCTGGTTGGACCTTTGGTGCAGGTGCTGC ATTACAAATACCATTTGCTATGCAAATGGCTTATAGGTTTAATGGTATTGGAGTT ACACAGAATGTTCTCTATGAGAACCAAAAATTGATTGCCAACCAATTTAATAGT GCTATTGGCAAAATTCAAGACTCACTTTCTTCCACAGCAAGTGCACTTGGAAAA CTTCAAGATGTGGTCAACCAAAATGCACAAGCTTTAAACACGCTTGTTAAACAA CTTAGCTCCAATTTTGGTGCAATTTCAAGTGTTTTAAATGATATCCTTTCACGTC TTGACAAAGTTGAGGCTGAAGTGCAAATTGATAGGTTGATCACAGGCAGACTT CAAAGTTTGCAGACATATGTGACTCAACAATTAATTAGAGCTGCAGAAATCAGA GCTTCTGCTAATCTTGCTGCTACTAAAATGTCAGAGTGTGTACTTGGACAATCA AAAAGAGTTGATTTTTGTGGAAAGGGCTATCATCTTATGTCCTTCCCTCAGTCA GCACCTCATGGTGTAGTCTTCTTGCATGTGACTTATGTCCCTGCACAAGAAAAG AACTTCACAACTGCTCCTGCCATTTGTCATGATGGAAAAGCACACTTTCCTCGT GAAGGTGTCTTTGTTTCAAATGGCACACACTGGTTTGTAACACAAAGGAATTTT TATGAACCACAAATCATTACTACAGACAACACATTTGTGTCTGGTAACTGTGAT GTTGTAATAGGAATTGTCAACAACACAGTTTATGATCCTTTGCAACCTGAATTA GACTCATTCAAGGAGGAGTTAGATAAATATTTTAAGAATCATACATCACCAGAT GTTGATTTAGGTGACATCTCTGGCATTAATGCTTCAGTTGTAAACATTCAAAAA GAAATTGACCGCCTCAATGAGGTTGCCAAGAATTTAAATGAATCTCTCATCGAT CTCCAAGAACTTGGAAAGTATGAGCAGTATATAAAATGGCCATGGTACATTTGG CTAGGTTTTATAGCTGGCTTGATTGCCATAGTAATGGTGACAATTATGCTTTGC TGTATGACCAGTTGCTGTAGTTGTCTCAAGGGCTGTTGTTCTTGTGGATCCTGC TGCAAATTTGATGAAGACGACTCTGAGCCAGTGCTCAAAGGAGTCAAATTACAT TACACATAA |
| 15 | Vaccine gene ABGDL comprising SARS-CoV-2 variants alpha, beta, gamma, delta, and lambda nucleotides | ATGTTCGTGTTCCTGGTGCTGCTGCCTCTGGTGTCCAGCCAGTGCGTGAACTT CCGGAACAGAACCCAGCTGCCTAGCGCCTACACCAACAGCTTTACCAGAGGC GTGTACTACCCCGACAAGGTGTTCAGATCCAGCGTGCTGCACTCTACCCAGGA CCTGTTCCTGCCTTTCTTCAGCAACGTGACCTGGTTCCACGCCATCAGCGGCA CCAACGTGATCAAGAGATTCGACAACCCCGTGCTGCCCTTCAACGACGGGGT GTACTTTGCCAGCACCGAGAAGTCCAACATCATCAGAGGCTGGATCTTCGGCA CCACACTGGACAGCAAGACCCAGAGCCTGCTGATCGTGAACAACGCCACCAA CGTCGTGATCAAAGTGTGCGAGTTCCAGTTCTGCAACTACCCCTTCCTGGGCG TCTACCACAAGAACAACAAGAGCTGGATGGAAAGCGGCGTGTACAGCAGCGC CAACAACTGCACCTTCGAGTACGTGTCCCAGCCTTTCCTGATGGACCTGGAAG GCAAGCAGGGCAACTTCAAGAACCTGTCCGAGTTCGTGTTTAAGAACATCGAC GGCTACTTCAAGATCTACAGCAAGCACACCCCTATCAACCTCGTGCGGGATCT GCCTCAGGGCTTCTCTGCTCTGGAACCCCTGGTGGATCTGCCCATCGGCATCA ACATCACCCGGTTTCAGACACTGCTGGCCCTGCACAATAGCAGCTCTGGATGG ACTGCTGGCGCCGCTGCCTACTATGTGGGATACCTGCAGCCTCGGACCTTCCT GCTGAAGTACAACGAGAACGGCACCATCACCGACGCCGTGGATTGTGCTCTG GACCCTCTGAGCGAGACAAAGTGCACCCTGAAGTCCTTCACCGTGGAAAAGG GCATCTACCAGACCAGCAACTTCCGGGTGCAGCCCACCGAATCCATCGTGCG GTTCCCCAATATCACCAATCTGTGCCCCTTCGGCGAGGTGTTCAATGCCACCA GATTCGCCTCTGTGTACGCCTGGAACCGGAAGCGGATCAGCAATTGCGTGGC CGACTACTCCGTGCTGTACAACTCCGCCAGCTTCAGCACCTTCAAGTGCTACG GCGTGTCCCCTACCAAGCTGAACGACCTGTGCTTCACAAACGTGTACGCCGAC AGCTTCGTGATCCGGGGAGATGAAGTGCGGCAGATTGCCCCTGGACAGACAG GCACAATCGCCGACTACAACTACAAGCTGCCCGACGACTTCACCGGCTGTGTG ATTGCCTGGAACAGCAACAACCTGGACTCCAAAGTCGGCGGCAACTACAATTA CCGGTACAGGCTGTTTCGGAAGTCCAATCTGAAGCCCTTCGAGCGGGACATCT |
| | | CCACCGAGATCTATCAGGCCGGCAGCAAGCCTTGCAATGGCGTGCAGGGCTT CAACTGCTACAGCCCACTGCAGAGCTACGGCTTCCAGCCAACATACGGCGTG GGCTACCAGCCTTACAGAGTGGTGGTGCTGAGCTTCGAGCTGCTGCATGCTC CTGCCACAGTGTGCGGCCCTAAGAAAAGCACCAATCTCGTGAAGAACAAATGC GTCAACTTCAATTTCAACGGCCTGACCGGCACCGGCGTGCTGACAGAGAGCA ACAAGAAGTTCCTGCCATTCCAGCAGTTCGGCCGGGATATCGCCGATACCACA GATGCCGTCAGAGATCCCCAGACACTGGAAATCCTGGACATCACCCCTTGCAG CTTCGGCGGAGTGTCTGTGATCACCCCTGGCACCAACACCAGCAATCAGGTG GCAGTGCTGTACCAGGGCGTTAACTGTACCGAAGTGCCCGTGGCCATTCACG CCGATCAGCTGACACCTACATGGCGGGTGTACTCCACCGGCTCCAACGTGTTC CAGACAAGAGCCGGCTGTCTGATCGGAGCCGAGTATGTGAACAACAGCTACG AGTGCGACATCCCCATCGGCGCTGGCATCTGCGCCTCTTACCAGACACAGAC CAACAGCAGGCGGAGAGCCAGATCTGTGGCCAGCCAGAGCATCATTGCCTAC ACAATGTCTCTGGGCGCCGAGAACAGCGTGGCCTACTCCAACAACTCTATCGC TATCCCCATCAACTTCACCATCAGCGTGACCACAGAGATCCTGCCTGTGTCCA TGACCAAGACCAGCGTGGACTGCACCATGTACATCTGCGGCGATAGCACCGA GTGCTCCAACCTGCTGCTGCAGTACGGCAGCTTCTGCACCCAGCTGAATAGAG CCCTGACCGGAATCGCCGTGGAACAGGACAAGAACACCCAAGAGGTGTTCGC CCAAGTGAAGCAGATCTACAAGACCCCTCCTATCAAGGACTTCGGCGGCTTTA ACTTCAGCCAGATTCTGCCCGATCCTAGCAAGCCCAGCAAGCGGAGCCCTATC GAGGACCTGCTGTTCAACAAAGTGACACTGGCCGACGCCGGCTTCATCAAGC AGTATGGCGATTGTCTGGGCGACATTGCCGCCAGGGATCTGATTTGCGCCCA GAAGTTCAACGGACTGAACGTGCTGCCTCCTCTGCTGACCGATGAGATGATCG CCCAGTACACATCTGCCCTGCTGGCCGGAACAATCACCAGCGGCTGGACATTT GGAGCTGGACCCGCTCTGCAGATCCCATTTCCAATGCAGATGGCCTACCGGTT CAACGGCATCGGAGTGACCCAGAATGTGCTGTACGAGAACCAGAAGCTGATC GCCAACCAGTTCAACAGCGCCATCGGCAAGATCCAGGACAGCCTGAGCAGCA CACCTAGCGCTCTGGGCAAGCTGCAGAACGTGGTCAACCAGAATGCCCAGGC ACTGAACACCCTGGTCAAGCAGCTGTCCAGCAATTTCGGCGCCATCTCTAGCG TGCTGAACGATATCCTGGCCAGACTGGACCCACCTGAGGCCGAGGTGCAGAT CGACAGACTGATCACAGGACGGCTGCAGTCCCTGCAGACCTACGTTACACAG CAGCTGATCAGAGCCGCCGAGATTAGAGCCTCTGCCAATCTGGCCGCCATCA AGATGAGCGAGTGTGTGCTGGGCCAGAGCAAGAGAGTGGACTTTTGCGGCAA GGGCTACCACCTGATGAGCTTCCCTCAGTCTGCTCCTCACGGCGTGGTGTTTC TGCACGTGACATACGTGCCCGCTCAAGAGAAGAATTTCACCACCGCTCCAGCC ATCTGCCACGACGGCAAAGCCCACTTTCCTAGAGAAGGCGTGTTCGTGTCCAA CGGCACCCATTGGTTCGTGACCCAGCGGAACTTCTACGAGCCCCAGATCATCA CCACACACAACACCTTCGTGTCCGGCAACTGTGACGTTGTGATCGGCATTGTG AACAATACCGTGTACGACCCTCTGCAGCCCGAGCTGGACTCCTTCAAAGAGGA ACTGGACAAGTACTTTAAGAACCACACAAGCCCCGACGTGGACCTGGGCGATA TCAGCGGAATCAACGCCTCCTTCGTGAACATCCAGAAAGAGATCGACCGGCTG AACGAGGTGGCCAAGAATCTGAACGAGAGCCTGATCGACCTGCAAGAACTGG GGAAGTACGAGCAGTACATCAAGTGGCCTTGGTACATCTGGCTGGGCTTTATC GCCGGACTGATTGCCATCGTGATGGTCACAATCATGCTGTGCTGCATGACCAG CTGCTGCTCTTGCCTGAAGGGCTGCTGTAGCTGTGGCTCCTGCTGCAAGTTCG ACGAGGACGATTCTGAGCCCGTGCTGAAGGGCGTGAAACTGCACTACACATG A |

As described above, any one or more of the individual amino acids highlighted above (bold and large type in the table), indicated as different from the SEQ ID NO 1 reference sequence, may be combined with any other one or more of highlighted amino acids, in order to arrive at a sequence of the invention. Thus, any combination of any one or more of the highlighted amino acid differences over SEQ ID NO 1 are encompassed by the invention and considered as disclosed herein. Furthermore, changes at any one or more of the indicated positions within any one or more of the indicated sequences, and any combination thereof, with reference to SEQ ID NO 1, without limitation to the specific resulting amino acid, are also considered encompassed by the invention and disclosed herein.

For several viruses, whose fusion activity is activated by low pH, have been shown that specific lipid or ion requirements may affect the specific endosomal fusion site. These requirements are either at the level of the initial interaction between the exposed fusion peptide/loop and the target membrane or at a later stage of the fusion process. For example, SFV requires ∼33 mol% cholesterol and ∼1-2% sphingomyelin in the target membrane for optimal fusion. The cholesterol prerequisite is the stable insertion of the fusion loop (within its fusion protein E1) into the target membrane before the refolding steps. Similarly, hantaviruses, like the Andean virus, require high cholesterol levels in the target membrane (in addition to a low pH) for fusion.

Some virus types require priming of viral fusion proteins by binding to a receptor, followed by the action of a protease, which may or may not be low pH-dependent. An exemplarily list of virus with pH triggered endosomal viral fusion is shown in Table 2. Such triggering mechanisms are used by many coronaviruses and the paramyxovirus respiratory syncytial virus (RSV). SARS-CoV has been extensively studied in this respect. After its interaction with the ACE2 receptor, SARS-CoV is activated by proteolytic cleavage; cathepsin L activates the virus in late endosomes pH-dependent, but the virus can also be activated pH-independently by trypsin-like proteases TMPRSS2 on the cell surface. As first identified with SARS-CoV 208, coronaviruses are somewhat unusual in that they have two different cleavage sites within their spike proteins and S2', which are known as S1/S2. In the case of SARS-CoV, either cathepsin L or trypsin-like enzymes cleave at both positions, but probably in a sequential manner (S1/S2 followed by S2'). The use of each protease may be different for each cell type (e.g. vero cells versus respiratory epithelial cells).

### FIGURES

The invention is demonstrated by way of the example through the figures disclosed herein. The figures provided represent particular, non-limiting embodiments and are not intended to limit the scope of the invention.

Short description of the figures:
**Figure 1** **Neutralizing antibodies after vaccination of mice with the vaccine gene encoding the SARS-CoV-2 spike protein comprising mutations occurring in the SARS-CoV-2 variants alpha, beta, and gamma.**
**Figure 2****: Neutralizing antibodies after vaccination of mice with the vaccine gene encoding the SARS-CoV-2 spike protein comprising mutations occurring in the SARS-CoV-2 variants alpha, beta, gamma, and delta.**

Detailed description of the figures:

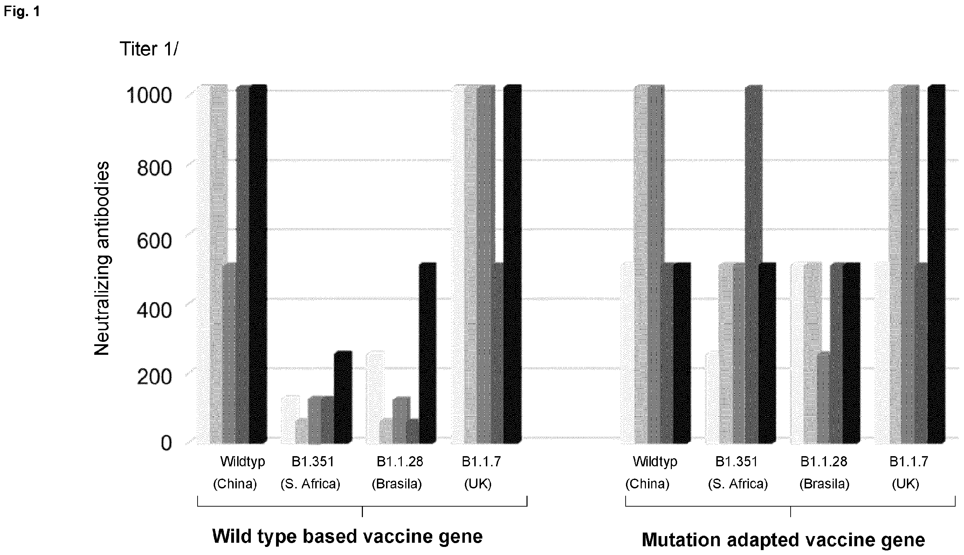

**Figure 1****: Neutralizing antibodies after vaccination of mice with the vaccine gene encoding the SARS-CoV-2 spike protein comprising mutations occurring in the SARS-CoV-2 variants alpha, beta, and gamma.** Each bar represents the neutralizing antibody titer in mice (n=5) immunized once with an adenoviral vaccine carrying the 'old' vaccine gene (left) or the mutation-adapted vaccine gene (right). The 'old' vaccine gene, which originates from the CoV2 strain (Wuhan), has an up to 20-fold lower efficacy in terms of the formation of neutralizing antibodies compared to the South African and Brazilian variants, while the mutation-adapted vaccine gene offers significantly improved immunogenicity against mutated virus strains overall.

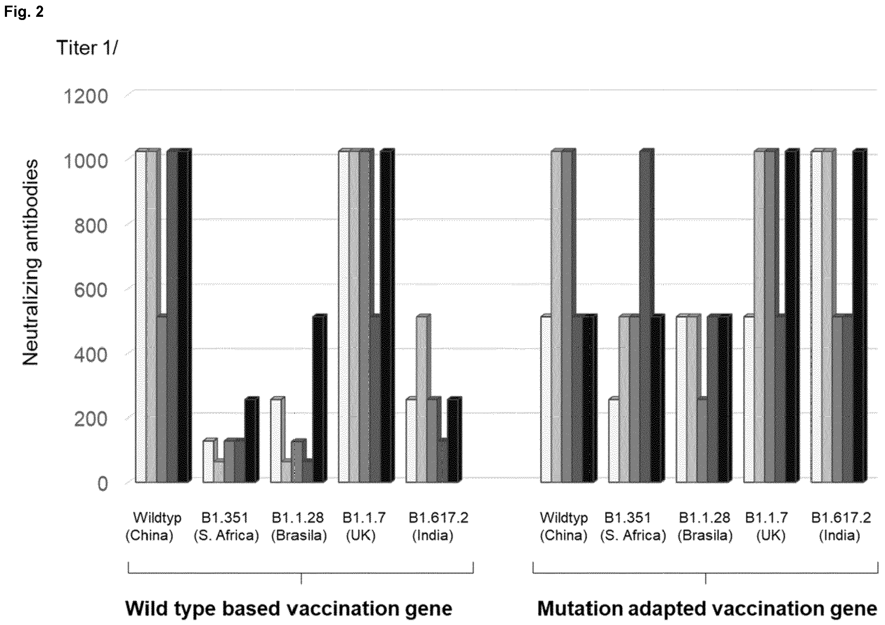

**Figure 2****: Neutralizing antibodies after vaccination of mice with the vaccine gene encoding the SARS-CoV-2 spike protein comprising mutations occurring in the SARS-CoV-2 variants alpha, beta, gamma, and delta.** Each bar represents the neutralizing antibody titer in mice (n=5) immunized once with an adenoviral vaccine carrying the 'old' vaccine gene (left) or the mutation-adapted vaccine gene (right). The 'old' vaccine gene, which originates from the CoV2 strain (Wuhan), has an up to 20-fold lower efficacy in terms of the formation of neutralizing antibodies compared to the South African and Brazilian variants, while the mutation-adapted vaccine gene offers significantly improved immunogenicity against mutated virus strains overall.

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

The examples below present the design, in vivo test and characterization of modified (mutation-adapted) vaccine gene encoding the SARS-CoV-2 spike protein suitable for the reducing the risk for an infection with a coronavirus and prophylaxis of Coronavirus-mediated disease, such as COVID-19.

The isolation of these antibodies and their corresponding binding properties distinguish the monoclonal antibodies characterized herein from developments of other research groups (for example Ju 2020). Described in more detail below is:
**Example 1: Design and test of a modified (mutation-adapted) vaccine gene encoding the SARS-CoV-2 spike protein comprising mutations occurring in the SARS-CoV-2 variants alpha, beta, gamma, and delta.**

The spike genes of the original SARS-CoV-2 strain (CoV2-S; WUHAN strain) and the mutation-adapted vaccine gene (CoV2-mut.S) were cloned into an expression cassette, inserted into an adenoviral vector (human replication-deficient E1/E3 deleted adenovirus serotype 6) and vaccinated in mice. Three weeks after vaccination, blood samples were collected from these mice and serum was isolated. The serum was tested in neutralization assays for its ability to neutralize the SARS-CoV-2 virus of the original strain (WUHAN) as well as several SARS-CoV-2 variants (Fig. 1).

It was shown that the mutation-adapted vaccine gene (SEQ ID NO 7) not only did not lose its ability to induce neutralising antibodies, but developed a significantly better efficacy against individual mutants than the original vaccine, which is currently used in all vaccines worldwide.

Point mutations and deletions occurring in the SARS-CoV-2 variants alpha, beta, gamma, and delta were included in the modified (mutation-adapted) vaccine gene encoding the SARS-CoV-2 spike protein by modifying the spike gene sequence of the CoV2 original strain (Wuhan). In addition, six pre-fusion stabilising mutations were inserted into the vaccine gene to ensure improved antigenic presentation.

| | |
|---|---|
| Vaccine gene comprising SARS-CoV-2 variants alpha, beta, and gamma | L18F, T20N, P26S, HV69-70del, D138Y, Y144del, R190S, K417T, E484K, N501Y, A570D, D614G, H655Y, P681H, T716I, S982A, T1027I, D1118H |
| pre-fusion stabilising mutations | F817P, A892P, A899P, A942P, K986P, V987P |

**Example 2: Design and test of a modified (mutation-adapted) vaccine gene encoding the SARS-CoV-2 spike protein comprising mutations occurring in the SARS-CoV-2 variants alpha, beta, gamma, and delta.**

The spike genes of the original SARS-CoV-2 strain (CoV2-S; WUHAN strain) and the mutation-adapted vaccine gene (CoV2-mut.S) are cloned into an expression cassette, inserted into an adenoviral vector (human replication-deficient E1/E3 deleted adenovirus serotype 6) and vaccinated into mice. Three weeks after vaccination, blood samples are collected from these mice and serum is isolated. The serum is tested in neutralization assays for its ability to neutralize the SARS-CoV-2 virus of the original strain (WUHAN) as well as several SARS-CoV-2 variants.

The mutation-adapted vaccine gene is not losing its ability to induce neutralizing antibodies, and is developing a significantly better efficacy against individual mutants than the original vaccine, which is currently used in all vaccines worldwide.

Point mutations and deletions occurring in the SARS-CoV-2 variants alpha, beta, gamma, and delta are included in the modified (mutation-adapted) vaccine gene encoding the SARS-CoV-2 spike protein by modifying the spike gene sequence of the CoV2 original strain (Wuhan). In addition, six pre-fusion stabilising mutations are inserted into the vaccine gene to ensure improved antigenic presentation.

| | |
|---|---|
| Vaccine gene comprising SARS-CoV-2 variants alpha, beta, gamma, and delta | L18F, T20N, P26S, HV69-70del, D138Y, Y144del, R190S K417T, E484K, N501Y, A570D, D614G, H655Y, P681H, T716I, S982A, T1027I, D1118H, T19R, del 157-158, L452R, T478K, D614G, P681R, D950N |
| pre-fusion stabilising mutations | F817P, A892P, A899P, A942P, K986P, V987P |

**Example 3: Design of a modified (mutation-adapted) vaccine gene encoding the SARS-CoV-2 spike protein comprising mutations occurring in the SARS-CoV-2 variants alpha, beta, gamma, delta, and lambda.**

The spike genes of the original SARS-CoV-2 strain (CoV2-S; WUHAN strain) and the mutation-adapted vaccine gene (CoV2-mut.S) are cloned into an expression cassette, inserted into an adenoviral vector (human replication-deficient E1/E3 deleted adenovirus serotype 6) and vaccinated into mice. Three weeks after vaccination, blood samples are collected from these mice and serum is isolated. The serum is tested in neutralization assays for its ability to neutralize the SARS-CoV-2 virus of the original strain (WUHAN) as well as several SARS-CoV-2 variants. (Fig 2)

The mutation-adapted vaccine gene is not losing its ability to induce neutralizing antibodies, and is developing a significantly better efficacy against individual mutants than the original vaccine, which is currently used in all vaccines worldwide.

Point mutations and deletions occurring in the SARS-CoV-2 variants alpha, beta, gamma, delta, and lambda are included in the modified (mutation-adapted) vaccine gene encoding the SARS-CoV-2 spike protein by modifying the spike gene sequence of the CoV2 original strain (Wuhan). In addition, six pre-fusion stabilising mutationsare inserted into the vaccine gene to ensure improved antigenic presentation.

| | |
|---|---|
| Vaccine gene comprising SARS-CoV-2 variants alpha, beta, gamma, delta, and lambda | L18F, T19R, T20N, P26S, HV-del 69-70, G75V, T76I, D138Y, Y144del, E156G, FR-del 157-158, R190S, D215G, RSYLTPG246-252del, D253N, K417T, L452R, T478K, E484Q, F490S, N501Y, D614G, H655Y, P681R, T716I, T859N, D950N, S982A, T1027I, D1118H, V1176F |
| pre-fusion stabilising mutations | F817P, A892P, A899P, A942P, K986P, V987P |

### References

1. Wu, Z. & McGoogan, J. M. Characteristics of and important lessons from the coronavirusdisease 2019 (COVID-19) outbreak in China: summary of a report of 72314 cases from the Chinese Center for Disease Control and Prevention. JAMA 323, 1239-1242 (2020).
2. Guan, W. J. et al. Clinical characteristics of coronavirus disease 2019 in China. N. Engl. J.Med. 382, 1708-1720 (2020).
3. Huang, C. et al. Clinical features of patients infected with 2019 novel coronavirus in Wuhan,China. Lancet 395, 497-506 (2020).
4. Oh, D.-Y., Kröger, S., Wedde, M., Hartkopf, F., Budt, M., Seifried, J., Radonic, A., Belarbi, E., Hölzer, M., Böttcher, S., et al. (2021). SARS-CoV-2-Varianten: Evolution im Zeitraffer. Deutsches Arzteblatt 118, A-460 / B-388.
5. World Health Organization (2021a). COVID-19 Weekly Epidemiological Update - 11 May 2021 (WHO).
6. World Health Organization (2021b). COVID-19 Weekly Epidemiological Update (Suppl. 25 February 2021), Special Edition: Proposed working definitions of SARS-CoV-2 Variants of Interest and Variants of Concern (World Health Organization).
7. World Health Organization (2021c). Tracking SARS-CoV-2 variants (World Health Organization).

## Claims

1. An isolated nucleic acid molecule encoding a variant spike protein (spike-like protein) of a corona virus or fragment thereof, wherein the variant spike protein comprises an amino acid sequence with at least two amino acid differences over SEQ ID NO 1 (Wuhan Sequence), and wherein said variant spike protein comprises amino acid differences over SEQ ID NO 1 from at least two of the B.1.1.7 (SEQ ID NO 2), B.1.351 (SEQ ID NO 3), B1.617.2 (SEQ ID NO 4), B.1.1.28 (SEQ ID NO 5) and C.37 (SEQ ID NO 6) spike proteins.

2. The nucleic acid molecule according to claim 1, wherein the variant spike (spike-like) protein comprises an amino acid sequence comprising at least four amino acid differences over SEQ ID NO 1 (Wuhan Sequence) at positions 18, 19, 20, 26, 69, 70, 75, 76, 138, 144, 145, , 242-244, 246-252, 253, 417, 452, 478, 484, 490, 501, 570, 614, 655, 681, 716, 859, 950, 982, 1027, 1118 and/or 1176, with reference to SEQ ID NO 1.

3. The nucleic acid molecule according to claim 1 or 2, wherein the variant comprises amino acid differences over SEQ ID NO 1 (Wuhan Sequence) located in two or more coronavirus spike protein domains, selected from the group consisting of an N-terminal domain (NTD), a receptor binding domain (RBP), a fusion peptide (FP), a heptapeptide repeat sequence 1 (HR1), a heptapeptide repeat sequence 2 (HR2), a transmembrane domain (TM) or cytoplasm domain (CT) and a protein sequence linking said domains.

4. The nucleic acid molecule according to any one of the preceding claims, wherein the variant comprises an amino acid sequence comprising at least four amino acids differences over SEQ ID NO 1 (Wuhan sequence), and wherein the variant comprises amino acid differences over SEQ ID NO 1 of at least two of B.1.1.7 (SEQ ID NO 2), B.1 .351 (SEQ ID NO 3), B1.617.2 (SEQ ID NO 4), B.1.1.28 (SEQ ID NO 5), and C.37 (SEQ ID NO 6) having spike proteins at positions 18, 19, 20, 26, 69, 70, 75, 76, 138, 144, 145, 156, 157, 158, 190, 242-244, 246-252, 253, 417, 452, 478, 484, 490, 501, 570, 614, 655, 681, 716, 859, 950, 982, 1027, 1118 and/or 1176, with reference to SEQ ID NO 1.

5. The nucleic acid molecule according to any one of the preceding claims, wherein the variant comprises an amino acid sequence (SEQ ID NO 9) comprising L18F, T19R, T20N, P26S, HV69-70 deletion, G75V, T76I, D138Y, Y144 deletion, E156G, FR157-158 deletion, R190S, D215G, RSYLTPG246-252 deletion, D253N, K417T, L452R, T478K, E484Q, F490S, N501Y, D614G, H655Y, P681R, T716I, T859N, D950N, S982A, T1027I, D1118H, and V1176F, with reference to SEQ ID NO 1.

6. The nucleic acid molecule according to any one of the preceding claims, wherein the variant additionally comprises one or more prefusion stabilizing amino acids, preferably at any amino acid position after 810, with reference to SEQ ID NO 1.

7. The nucleic acid molecule according to the preceding claim, wherein the prefusion stabilizing mutations occur at amino acid positions 817, 892, 899, 942, 986 and 987, with reference to SEQ ID NO 1, preferably F817P, A892P, A899P, A942P, K986P, and V987P.

8. The nucleic acid molecule according to any one of the preceding claims, wherein the nucleic acid molecule encodes a variant spike protein comprising or consisting of a sequence with at least 80% sequence identity, preferably 90%, more preferably 95% sequence identity, to SEQ ID NO 1.

9. The nucleic acid molecule according to any one of the preceding claims, wherein the nucleic acid molecule encodes a variant spike protein comprising or consisting of an amino acid sequence according to SEQ ID NO 7, SEQ ID NO 8, SEQ ID NO 9 or SEQ ID NO 10.

10. A recombinant variant spike protein of a corona virus encoded by the nucleic acid molecule according to any one of the preceding claims, preferably comprising or consisting of an amino acid sequence according to SEQ ID NO 7, SEQ ID NO 8, SEQ ID NO 9 or SEQ ID NO 10.

11. A pharmaceutical composition in the form of a vaccine for use in the prevention and/or reduction of risk of a human coronavirus infection, wherein said vaccine comprises nucleic acid molecule according to any of claims 1 to 10 or a recombinant protein according to claim 11, with a pharmaceutically acceptable carrier.

12. The pharmaceutical composition according to the preceding claim, wherein the composition comprises:
i) A messenger ribonucleic acid (mRNA) molecule encoding the variant spike protein of a corona virus according to any of claims 1 to 10, or
ii) A recombinant viral vector (such as adenovirus, MVA, CMV, adeno-associated virus) comprising a nucleotide sequence encoding a variant spike protein of a corona virus according to any of claims 1 to 10,
iii) A polypeptide of the variant spike protein of a corona virus comprising an amino acid sequence encoded by a nucleotide sequence according to any of claims 1 to 10.

13. The pharmaceutical composition according to claim 12 or 13, wherein the composition induces an immunogenic response in a human subject against a human coronavirus, wherein the human coronavirus is a SARS corona virus, preferably SARS-CoV-2, more preferably a mutant of SARS-CoV-2 including alpha, beta, gamma, delta, and lambda or any combination thereof

14. The pharmaceutical composition according to claims 12 to 14, wherein the recombinant adenovirus or recombinant adeno-associated virus is selected from the group of replication-deficient adeno-associated viral vectors and replication-deficient adenoviral vectors, including serotype 1, serotype 2, serotype 5, serotype 6, serotype 26, serotype 35, serotype 41, e.g. or the human replication-deficient E1/E3 deleted adenovirus serotype 6.

15. The pharmaceutical composition according to claim 13, wherein the mRNA comprises
i) A 5'-cap structure,
ii) A 5'-UTR,
iii) An open reading frame encoding the variant spike protein of a corona virus according to any of claims 1 to 10,
iv) A 3'-UTR, and
v) A Poly-A-region of at least 50 nucleotides in length.


SEQUENCE LISTING

<110>  Charité - Universitätsmedizin Berlin

<120>  VACCINE WITH IMPROVED IMMUNOGENICITY AGAINST MUTANT CORONAVIRUSES

<130>  2197-21EP

<160>  15

<170>  PatentIn version 3.5

<210>  1
<211>  1273
<212>  PRT
<213>  Severe acute respiratory syndrome-related coronavirus

<400>  1

<210> 2
<211> 1270
<212> PRT
<213> Severe acute respiratory syndrome-related coronavirus

<220>
<221> VARIANT
<222> (678)..(678)
<223> Position 678 can also be substituted with an R, wherein position
678 in B.1.1.7 (Alpha) refers to position 681 in WUHAN Sequence
(SEQ ID No 1).

<400> 2

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile Ser Gly Thr Asn Gly Thr Lys Arg Phe Asp Asn Pro
65                  70                  75                  80

Val Leu Pro Phe Asn Asp Gly Val Tyr Phe Ala Ser Thr Glu Lys Ser
                85                  90                  95

Asn Ile Ile Arg Gly Trp Ile Phe Gly Thr Thr Leu Asp Ser Lys Thr
            100                 105                 110

Gln Ser Leu Leu Ile Val Asn Asn Ala Thr Asn Val Val Ile Lys Val
        115                 120                 125

Cys Glu Phe Gln Phe Cys Asn Asp Pro Phe Leu Gly Val Tyr His Lys
    130                 135                 140

Asn Asn Lys Ser Trp Met Glu Ser Glu Phe Arg Val Tyr Ser Ser Ala
145                 150                 155                 160

Asn Asn Cys Thr Phe Glu Tyr Val Ser Gln Pro Phe Leu Met Asp Leu
                165                 170                 175

Glu Gly Lys Gln Gly Asn Phe Lys Asn Leu Arg Glu Phe Val Phe Lys
            180                 185                 190

Asn Ile Asp Gly Tyr Phe Lys Ile Tyr Ser Lys His Thr Pro Ile Asn
        195                 200                 205

Leu Val Arg Asp Leu Pro Gln Gly Phe Ser Ala Leu Glu Pro Leu Val
    210                 215                 220

Asp Leu Pro Ile Gly Ile Asn Ile Thr Arg Phe Gln Thr Leu Leu Ala
225                 230                 235                 240

Leu His Arg Ser Tyr Leu Thr Pro Gly Asp Ser Ser Ser Gly Trp Thr
                245                 250                 255

Ala Gly Ala Ala Ala Tyr Tyr Val Gly Tyr Leu Gln Pro Arg Thr Phe
            260                 265                 270

Leu Leu Lys Tyr Asn Glu Asn Gly Thr Ile Thr Asp Ala Val Asp Cys
        275                 280                 285

Ala Leu Asp Pro Leu Ser Glu Thr Lys Cys Thr Leu Lys Ser Phe Thr
    290                 295                 300

Val Glu Lys Gly Ile Tyr Gln Thr Ser Asn Phe Arg Val Gln Pro Thr
305                 310                 315                 320

Glu Ser Ile Val Arg Phe Pro Asn Ile Thr Asn Leu Cys Pro Phe Gly
                325                 330                 335

Glu Val Phe Asn Ala Thr Arg Phe Ala Ser Val Tyr Ala Trp Asn Arg
        340                 345                 350

Lys Arg Ile Ser Asn Cys Val Ala Asp Tyr Ser Val Leu Tyr Asn Ser
    355                 360                 365

Ala Ser Phe Ser Thr Phe Lys Cys Tyr Gly Val Ser Pro Thr Lys Leu
    370                 375                 380

Asn Asp Leu Cys Phe Thr Asn Val Tyr Ala Asp Ser Phe Val Ile Arg
385                 390                 395                 400

Gly Asp Glu Val Arg Gln Ile Ala Pro Gly Gln Thr Gly Lys Ile Ala
                405                 410                 415

Asp Tyr Asn Tyr Lys Leu Pro Asp Asp Phe Thr Gly Cys Val Ile Ala
            420                 425                 430

Trp Asn Ser Asn Asn Leu Asp Ser Lys Val Gly Gly Asn Tyr Asn Tyr
        435                 440                 445

Leu Tyr Arg Leu Phe Arg Lys Ser Asn Leu Lys Pro Phe Glu Arg Asp
    450                 455                 460

Ile Ser Thr Glu Ile Tyr Gln Ala Gly Ser Thr Pro Cys Asn Gly Val
465                 470                 475                 480

Glu Gly Phe Asn Cys Tyr Phe Pro Leu Gln Ser Tyr Gly Phe Gln Pro
                485                 490                 495

Thr Tyr Gly Val Gly Tyr Gln Pro Tyr Arg Val Val Val Leu Ser Phe
            500                 505                 510

Glu Leu Leu His Ala Pro Ala Thr Val Cys Gly Pro Lys Lys Ser Thr
        515                 520                 525

Asn Leu Val Lys Asn Lys Cys Val Asn Phe Asn Phe Asn Gly Leu Thr
    530                 535                 540

Gly Thr Gly Val Leu Thr Glu Ser Asn Lys Lys Phe Leu Pro Phe Gln
545                 550                 555                 560

Gln Phe Gly Arg Asp Ile Asp Asp Thr Thr Asp Ala Val Arg Asp Pro
                565                 570                 575

Gln Thr Leu Glu Ile Leu Asp Ile Thr Pro Cys Ser Phe Gly Gly Val
            580                 585                 590

Ser Val Ile Thr Pro Gly Thr Asn Thr Ser Asn Gln Val Ala Val Leu
        595                 600                 605

Tyr Gln Gly Val Asn Cys Thr Glu Val Pro Val Ala Ile His Ala Asp
    610                 615                 620

Gln Leu Thr Pro Thr Trp Arg Val Tyr Ser Thr Gly Ser Asn Val Phe
625                 630                 635                 640

Gln Thr Arg Ala Gly Cys Leu Ile Gly Ala Glu His Val Asn Asn Ser
                645                 650                 655

Tyr Glu Cys Asp Ile Pro Ile Gly Ala Gly Ile Cys Ala Ser Tyr Gln
            660                 665                 670

Thr Gln Thr Asn Ser His Arg Arg Ala Arg Ser Val Ala Ser Gln Ser
        675                 680                 685

Ile Ile Ala Tyr Thr Met Ser Leu Gly Ala Glu Asn Ser Val Ala Tyr
    690                 695                 700

Ser Asn Asn Ser Ile Ala Ile Pro Ile Asn Phe Thr Ile Ser Val Thr
705                 710                 715                 720

Thr Glu Ile Leu Pro Val Ser Met Thr Lys Thr Ser Val Asp Cys Thr
                725                 730                 735

Met Tyr Ile Cys Gly Asp Ser Thr Glu Cys Ser Asn Leu Leu Leu Gln
            740                 745                 750

Tyr Gly Ser Phe Cys Thr Gln Leu Asn Arg Ala Leu Thr Gly Ile Ala
        755                 760                 765

Val Glu Gln Asp Lys Asn Thr Gln Glu Val Phe Ala Gln Val Lys Gln
    770                 775                 780

Ile Tyr Lys Thr Pro Pro Ile Lys Asp Phe Gly Gly Phe Asn Phe Ser
785                 790                 795                 800

Gln Ile Leu Pro Asp Pro Ser Lys Pro Ser Lys Arg Ser Phe Ile Glu
                805                 810                 815

Asp Leu Leu Phe Asn Lys Val Thr Leu Ala Asp Ala Gly Phe Ile Lys
            820                 825                 830

Gln Tyr Gly Asp Cys Leu Gly Asp Ile Ala Ala Arg Asp Leu Ile Cys

        835                 840                 845

Ala Gln Lys Phe Asn Gly Leu Thr Val Leu Pro Pro Leu Leu Thr Asp
    850                 855                 860

Glu Met Ile Ala Gln Tyr Thr Ser Ala Leu Leu Ala Gly Thr Ile Thr
865                 870                 875                 880

Ser Gly Trp Thr Phe Gly Ala Gly Ala Ala Leu Gln Ile Pro Phe Ala
                885                 890                 895

Met Gln Met Ala Tyr Arg Phe Asn Gly Ile Gly Val Thr Gln Asn Val
            900                 905                 910

Leu Tyr Glu Asn Gln Lys Leu Ile Ala Asn Gln Phe Asn Ser Ala Ile
        915                 920                 925

Gly Lys Ile Gln Asp Ser Leu Ser Ser Thr Ala Ser Ala Leu Gly Lys
    930                 935                 940

Leu Gln Asp Val Val Asn Gln Asn Ala Gln Ala Leu Asn Thr Leu Val
945                 950                 955                 960

Lys Gln Leu Ser Ser Asn Phe Gly Ala Ile Ser Ser Val Leu Asn Asp
                965                 970                 975

Ile Leu Ala Arg Leu Asp Lys Val Glu Ala Glu Val Gln Ile Asp Arg
            980                 985                 990

Leu Ile Thr Gly Arg Leu Gln Ser Leu Gln Thr Tyr Val Thr Gln Gln
        995                 1000                1005

Leu Ile Arg Ala Ala Glu Ile Arg Ala Ser Ala Asn Leu Ala Ala
    1010                1015                1020

Thr Lys Met Ser Glu Cys Val Leu Gly Gln Ser Lys Arg Val Asp
    1025                1030                1035

Phe Cys Gly Lys Gly Tyr His Leu Met Ser Phe Pro Gln Ser Ala
    1040                1045                1050

Pro His Gly Val Val Phe Leu His Val Thr Tyr Val Pro Ala Gln
    1055                1060                1065

Glu Lys Asn Phe Thr Thr Ala Pro Ala Ile Cys His Asp Gly Lys
    1070                1075                1080

Ala His Phe Pro Arg Glu Gly Val Phe Val Ser Asn Gly Thr His
    1085                1090                1095

Trp Phe Val Thr Gln Arg Asn Phe Tyr Glu Pro Gln Ile Ile Thr
    1100                1105                1110

Thr His Asn Thr Phe Val Ser Gly Asn Cys Asp Val Val Ile Gly
    1115                1120                1125

Ile Val Asn Asn Thr Val Tyr Asp Pro Leu Gln Pro Glu Leu Asp
    1130                1135                1140

Ser Phe Lys Glu Glu Leu Asp Lys Tyr Phe Lys Asn His Thr Ser
    1145                1150                1155

Pro Asp Val Asp Leu Gly Asp Ile Ser Gly Ile Asn Ala Ser Val
    1160                1165                1170

Val Asn Ile Gln Lys Glu Ile Asp Arg Leu Asn Glu Val Ala Lys
    1175                1180                1185

Asn Leu Asn Glu Ser Leu Ile Asp Leu Gln Glu Leu Gly Lys Tyr
    1190                1195                1200

Glu Gln Tyr Ile Lys Trp Pro Trp Tyr Ile Trp Leu Gly Phe Ile
    1205                1210                1215

Ala Gly Leu Ile Ala Ile Val Met Val Thr Ile Met Leu Cys Cys
    1220                1225                1230

Met Thr Ser Cys Cys Ser Cys Leu Lys Gly Cys Cys Ser Cys Gly
    1235                1240                1245

Ser Cys Cys Lys Phe Asp Glu Asp Asp Ser Glu Pro Val Leu Lys
    1250                1255                1260

Gly Val Lys Leu His Tyr Thr
    1265                1270

<210>  3
<211>  1270
<212>  PRT
<213>  Severe acute respiratory syndrome-related coronavirus

<220>
<221>  VARIANT
<222>  (481)..(481)
<223>  Position 481 can also be substituted with an Q, wherein position
       481 in B.1.351 (Beta) refers to position 681 in WUHAN Sequence

(SEQ ID No 1).

<400> 3

Met Phe Val Phe Leu Val Leu Leu Pro Leu Val Ser Ser Gln Cys Val
1               5                   10                  15

Asn Phe Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Ala
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Gly Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu His Arg Ser Tyr Leu Thr Pro Gly Asp Ser Ser Ser Gly Trp Thr
                245                 250                 255

Ala Gly Ala Ala Ala Tyr Tyr Val Gly Tyr Leu Gln Pro Arg Thr Phe
            260                 265                 270

Leu Leu Lys Tyr Asn Glu Asn Gly Thr Ile Thr Asp Ala Val Asp Cys
        275                 280                 285

Ala Leu Asp Pro Leu Ser Glu Thr Lys Cys Thr Leu Lys Ser Phe Thr
    290                 295                 300

Val Glu Lys Gly Ile Tyr Gln Thr Ser Asn Phe Arg Val Gln Pro Thr
305                 310                 315                 320

Glu Ser Ile Val Arg Phe Pro Asn Ile Thr Asn Leu Cys Pro Phe Gly
                325                 330                 335

Glu Val Phe Asn Ala Thr Arg Phe Ala Ser Val Tyr Ala Trp Asn Arg
            340                 345                 350

Lys Arg Ile Ser Asn Cys Val Ala Asp Tyr Ser Val Leu Tyr Asn Ser
        355                 360                 365

Ala Ser Phe Ser Thr Phe Lys Cys Tyr Gly Val Ser Pro Thr Lys Leu
    370                 375                 380

Asn Asp Leu Cys Phe Thr Asn Val Tyr Ala Asp Ser Phe Val Ile Arg
385                 390                 395                 400

Gly Asp Glu Val Arg Gln Ile Ala Pro Gly Gln Thr Gly Asn Ile Ala
                405                 410                 415

Asp Tyr Asn Tyr Lys Leu Pro Asp Asp Phe Thr Gly Cys Val Ile Ala
            420                 425                 430

Trp Asn Ser Asn Asn Leu Asp Ser Lys Val Gly Gly Asn Tyr Asn Tyr
        435                 440                 445

Leu Tyr Arg Leu Phe Arg Lys Ser Asn Leu Lys Pro Phe Glu Arg Asp
    450                 455                 460

Ile Ser Thr Glu Ile Tyr Gln Ala Gly Ser Thr Pro Cys Asn Gly Val
465                 470                 475                 480

Lys Gly Phe Asn Cys Tyr Phe Pro Leu Gln Ser Tyr Gly Phe Gln Pro
                485                 490                 495

Thr Tyr Gly Val Gly Tyr Gln Pro Tyr Arg Val Val Val Leu Ser Phe
            500                 505                 510

Glu Leu Leu His Ala Pro Ala Thr Val Cys Gly Pro Lys Lys Ser Thr
        515                 520                 525

Asn Leu Val Lys Asn Lys Cys Val Asn Phe Asn Phe Asn Gly Leu Thr
    530                 535                 540

Gly Thr Gly Val Leu Thr Glu Ser Asn Lys Lys Phe Leu Pro Phe Gln
545                 550                 555                 560

Gln Phe Gly Arg Asp Ile Ala Asp Thr Thr Asp Ala Val Arg Asp Pro
                565                 570                 575

Gln Thr Leu Glu Ile Leu Asp Ile Thr Pro Cys Ser Phe Gly Gly Val
            580                 585                 590

Ser Val Ile Thr Pro Gly Thr Asn Thr Ser Asn Gln Val Ala Val Leu
        595                 600                 605

Tyr Gln Gly Val Asn Cys Thr Glu Val Pro Val Ala Ile His Ala Asp
    610                 615                 620

Gln Leu Thr Pro Thr Trp Arg Val Tyr Ser Thr Gly Ser Asn Val Phe
625                 630                 635                 640

Gln Thr Arg Ala Gly Cys Leu Ile Gly Ala Glu His Val Asn Asn Ser
                645                 650                 655

Tyr Glu Cys Asp Ile Pro Ile Gly Ala Gly Ile Cys Ala Ser Tyr Gln
            660                 665                 670

Thr Gln Thr Asn Ser Pro Arg Arg Ala Arg Ser Val Ala Ser Gln Ser
        675                 680                 685

Ile Ile Ala Tyr Thr Met Ser Leu Gly Val Glu Asn Ser Val Ala Tyr
    690                 695                 700

Ser Asn Asn Ser Ile Ala Ile Pro Thr Asn Phe Thr Ile Ser Val Thr
705                 710                 715                 720

Thr Glu Ile Leu Pro Val Ser Met Thr Lys Thr Ser Val Asp Cys Thr
                725                 730                 735

Met Tyr Ile Cys Gly Asp Ser Thr Glu Cys Ser Asn Leu Leu Leu Gln

740                 745                 750

Tyr Gly Ser Phe Cys Thr Gln Leu Asn Arg Ala Leu Thr Gly Ile Ala
        755                 760                 765

Val Glu Gln Asp Lys Asn Thr Gln Glu Val Phe Ala Gln Val Lys Gln
    770                 775                 780

Ile Tyr Lys Thr Pro Pro Ile Lys Asp Phe Gly Gly Phe Asn Phe Ser
785                 790                 795                 800

Gln Ile Leu Pro Asp Pro Ser Lys Pro Ser Lys Arg Ser Phe Ile Glu
                805                 810                 815

Asp Leu Leu Phe Asn Lys Val Thr Leu Ala Asp Ala Gly Phe Ile Lys
            820                 825                 830

Gln Tyr Gly Asp Cys Leu Gly Asp Ile Ala Ala Arg Asp Leu Ile Cys
        835                 840                 845

Ala Gln Lys Phe Asn Gly Leu Thr Val Leu Pro Pro Leu Leu Thr Asp
    850                 855                 860

Glu Met Ile Ala Gln Tyr Thr Ser Ala Leu Leu Ala Gly Thr Ile Thr
865                 870                 875                 880

Ser Gly Trp Thr Phe Gly Ala Gly Ala Ala Leu Gln Ile Pro Phe Ala
                885                 890                 895

Met Gln Met Ala Tyr Arg Phe Asn Gly Ile Gly Val Thr Gln Asn Val
            900                 905                 910

Leu Tyr Glu Asn Gln Lys Leu Ile Ala Asn Gln Phe Asn Ser Ala Ile
        915                 920                 925

Gly Lys Ile Gln Asp Ser Leu Ser Ser Thr Ala Ser Ala Leu Gly Lys
    930                 935                 940

Leu Gln Asp Val Val Asn Gln Asn Ala Gln Ala Leu Asn Thr Leu Val
945                 950                 955                 960

Lys Gln Leu Ser Ser Asn Phe Gly Ala Ile Ser Ser Val Leu Asn Asp
                965                 970                 975

Ile Leu Ser Arg Leu Asp Lys Val Glu Ala Glu Val Gln Ile Asp Arg
            980                 985                 990

Leu Ile Thr Gly Arg Leu Gln Ser Leu Gln Thr Tyr Val Thr Gln Gln
        995                 1000                1005

Leu Ile Arg Ala Ala Glu Ile Arg Ala Ser Ala Asn Leu Ala Ala
    1010                1015                1020

Thr Lys Met Ser Glu Cys Val Leu Gly Gln Ser Lys Arg Val Asp
    1025                1030                1035

Phe Cys Gly Lys Gly Tyr His Leu Met Ser Phe Pro Gln Ser Ala
    1040                1045                1050

Pro His Gly Val Val Phe Leu His Val Thr Tyr Val Pro Ala Gln
    1055                1060                1065

Glu Lys Asn Phe Thr Thr Ala Pro Ala Ile Cys His Asp Gly Lys
    1070                1075                1080

Ala His Phe Pro Arg Glu Gly Val Phe Val Ser Asn Gly Thr His
    1085                1090                1095

Trp Phe Val Thr Gln Arg Asn Phe Tyr Glu Pro Gln Ile Ile Thr
    1100                1105                1110

Thr Asp Asn Thr Phe Val Ser Gly Asn Cys Asp Val Val Ile Gly
    1115                1120                1125

Ile Val Asn Asn Thr Val Tyr Asp Pro Leu Gln Pro Glu Leu Asp
    1130                1135                1140

Ser Phe Lys Glu Glu Leu Asp Lys Tyr Phe Lys Asn His Thr Ser
    1145                1150                1155

Pro Asp Val Asp Leu Gly Asp Ile Ser Gly Ile Asn Ala Ser Val
    1160                1165                1170

Val Asn Ile Gln Lys Glu Ile Asp Arg Leu Asn Glu Val Ala Lys
    1175                1180                1185

Asn Leu Asn Glu Ser Leu Ile Asp Leu Gln Glu Leu Gly Lys Tyr
    1190                1195                1200

Glu Gln Tyr Ile Lys Trp Pro Trp Tyr Ile Trp Leu Gly Phe Ile
    1205                1210                1215

Ala Gly Leu Ile Ala Ile Val Met Val Thr Ile Met Leu Cys Cys
    1220                1225                1230

Met Thr Ser Cys Cys Ser Cys Leu Lys Gly Cys Cys Ser Cys Gly
    1235                1240                1245

Ser Cys Cys Lys Phe Asp Glu Asp Asp Ser Glu Pro Val Leu Lys
    1250                1255                1260

Gly Val Lys Leu His Tyr Thr
    1265                1270

<210> 4
<211> 1271
<212> PRT
<213> Severe acute respiratory syndrome-related coronavirus

<220>
<221> VARIANT
<222> (450)..(450)
<223> Position 450 can also be substituted with an Q, wherein position
      450 in B.1.617.2 (Delta) refers to position 452 in WUHAN Sequence
      (SEQ ID No 1).

<220>
<221> VARIANT
<222> (679)..(679)
<223> Position 679 can also be substituted with an H, wherein position
      679 in B.1.617.2 (Delta) refers to position 681 in WUHAN Sequence
      (SEQ ID No 1).

<400> 4

Met Phe Val Phe Leu Val Leu Leu Pro Leu Val Ser Ser Gln Cys Val
1               5                   10                  15

Asn Leu Arg Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Val Tyr Ser Ser
145                 150                 155                 160

Ala Asn Asn Cys Thr Phe Glu Tyr Val Ser Gln Pro Phe Leu Met Asp
                165                 170                 175

Leu Glu Gly Lys Gln Gly Asn Phe Lys Asn Leu Arg Glu Phe Val Phe
            180                 185                 190

Lys Asn Ile Asp Gly Tyr Phe Lys Ile Tyr Ser Lys His Thr Pro Ile
        195                 200                 205

Asn Leu Val Arg Asp Leu Pro Gln Gly Phe Ser Ala Leu Glu Pro Leu
    210                 215                 220

Val Asp Leu Pro Ile Gly Ile Asn Ile Thr Arg Phe Gln Thr Leu Leu
225                 230                 235                 240

Ala Leu His Arg Ser Tyr Leu Thr Pro Gly Asp Ser Ser Ser Gly Trp
                245                 250                 255

Thr Ala Gly Ala Ala Ala Tyr Tyr Val Gly Tyr Leu Gln Pro Arg Thr
            260                 265                 270

Phe Leu Leu Lys Tyr Asn Glu Asn Gly Thr Ile Thr Asp Ala Val Asp
        275                 280                 285

Cys Ala Leu Asp Pro Leu Ser Glu Thr Lys Cys Thr Leu Lys Ser Phe
    290                 295                 300

Thr Val Glu Lys Gly Ile Tyr Gln Thr Ser Asn Phe Arg Val Gln Pro
305                 310                 315                 320

Thr Glu Ser Ile Val Arg Phe Pro Asn Ile Thr Asn Leu Cys Pro Phe
                325                 330                 335

Gly Glu Val Phe Asn Ala Thr Arg Phe Ala Ser Val Tyr Ala Trp Asn
            340                 345                 350

Arg Lys Arg Ile Ser Asn Cys Val Ala Asp Tyr Ser Val Leu Tyr Asn
        355                 360                 365

Ser Ala Ser Phe Ser Thr Phe Lys Cys Tyr Gly Val Ser Pro Thr Lys
    370                 375                 380

Leu Asn Asp Leu Cys Phe Thr Asn Val Tyr Ala Asp Ser Phe Val Ile
385                 390                 395                 400

Arg Gly Asp Glu Val Arg Gln Ile Ala Pro Gly Gln Thr Gly Lys Ile
                405                 410                 415

Ala Asp Tyr Asn Tyr Lys Leu Pro Asp Asp Phe Thr Gly Cys Val Ile
            420                 425                 430

Ala Trp Asn Ser Asn Asn Leu Asp Ser Lys Val Gly Gly Asn Tyr Asn
        435                 440                 445

Tyr Arg Tyr Arg Leu Phe Arg Lys Ser Asn Leu Lys Pro Phe Glu Arg
    450                 455                 460

Asp Ile Ser Thr Glu Ile Tyr Gln Ala Gly Ser Lys Pro Cys Asn Gly
465                 470                 475                 480

Val Glu Gly Phe Asn Cys Tyr Phe Pro Leu Gln Ser Tyr Gly Phe Gln
                485                 490                 495

Pro Thr Asn Gly Val Gly Tyr Gln Pro Tyr Arg Val Val Val Leu Ser
            500                 505                 510

Phe Glu Leu Leu His Ala Pro Ala Thr Val Cys Gly Pro Lys Lys Ser
        515                 520                 525

Thr Asn Leu Val Lys Asn Lys Cys Val Asn Phe Asn Phe Asn Gly Leu
    530                 535                 540

Thr Gly Thr Gly Val Leu Thr Glu Ser Asn Lys Lys Phe Leu Pro Phe
545                 550                 555                 560

Gln Gln Phe Gly Arg Asp Ile Ala Asp Thr Thr Asp Ala Val Arg Asp
                565                 570                 575

Pro Gln Thr Leu Glu Ile Leu Asp Ile Thr Pro Cys Ser Phe Gly Gly
            580                 585                 590

Val Ser Val Ile Thr Pro Gly Thr Asn Thr Ser Asn Gln Val Ala Val
        595                 600                 605

Leu Tyr Gln Gly Val Asn Cys Thr Glu Val Pro Val Ala Ile His Ala
    610                 615                 620

Asp Gln Leu Thr Pro Thr Trp Arg Val Tyr Ser Thr Gly Ser Asn Val
625                 630                 635                 640

Phe Gln Thr Arg Ala Gly Cys Leu Ile Gly Ala Glu His Val Asn Asn
                645                 650                 655

Ser Tyr Glu Cys Asp Ile Pro Ile Gly Ala Gly Ile Cys Ala Ser Tyr
            660                 665                 670

Gln Thr Gln Thr Asn Ser Arg Arg Arg Ala Arg Ser Val Ala Ser Gln
        675                 680                 685

Ser Ile Ile Ala Tyr Thr Met Ser Leu Gly Ala Glu Asn Ser Val Ala
    690                 695                 700

Tyr Ser Asn Asn Ser Ile Ala Ile Pro Thr Asn Phe Thr Ile Ser Val
705                 710                 715                 720

Thr Thr Glu Ile Leu Pro Val Ser Met Thr Lys Thr Ser Val Asp Cys
                725                 730                 735

Thr Met Tyr Ile Cys Gly Asp Ser Thr Glu Cys Ser Asn Leu Leu Leu
            740                 745                 750

Gln Tyr Gly Ser Phe Cys Thr Gln Leu Asn Arg Ala Leu Thr Gly Ile
        755                 760                 765

Ala Val Glu Gln Asp Lys Asn Thr Gln Glu Val Phe Ala Gln Val Lys
    770                 775                 780

Gln Ile Tyr Lys Thr Pro Pro Ile Lys Asp Phe Gly Gly Phe Asn Phe
785                 790                 795                 800

Ser Gln Ile Leu Pro Asp Pro Ser Lys Pro Ser Lys Arg Ser Phe Ile
                805                 810                 815

Glu Asp Leu Leu Phe Asn Lys Val Thr Leu Ala Asp Ala Gly Phe Ile
            820                 825                 830

Lys Gln Tyr Gly Asp Cys Leu Gly Asp Ile Ala Ala Arg Asp Leu Ile
        835                 840                 845

Cys Ala Gln Lys Phe Asn Gly Leu Thr Val Leu Pro Pro Leu Leu Thr
    850                 855                 860

Asp Glu Met Ile Ala Gln Tyr Thr Ser Ala Leu Leu Ala Gly Thr Ile

865                 870                 875                 880

Thr Ser Gly Trp Thr Phe Gly Ala Gly Ala Ala Leu Gln Ile Pro Phe
                885                 890                 895

Ala Met Gln Met Ala Tyr Arg Phe Asn Gly Ile Gly Val Thr Gln Asn
            900                 905                 910

Val Leu Tyr Glu Asn Gln Lys Leu Ile Ala Asn Gln Phe Asn Ser Ala
        915                 920                 925

Ile Gly Lys Ile Gln Asp Ser Leu Ser Ser Thr Ala Ser Ala Leu Gly
    930                 935                 940

Lys Leu Gln Asn Val Val Asn Gln Asn Ala Gln Ala Leu Asn Thr Leu
945                 950                 955                 960

Val Lys Gln Leu Ser Ser Asn Phe Gly Ala Ile Ser Ser Val Leu Asn
                965                 970                 975

Asp Ile Leu Ser Arg Leu Asp Lys Val Glu Ala Glu Val Gln Ile Asp
            980                 985                 990

Arg Leu Ile Thr Gly Arg Leu Gln  Ser Leu Gln Thr Tyr  Val Thr Gln
        995                 1000                1005

Gln Leu  Ile Arg Ala Ala Glu  Ile Arg Ala Ser Ala  Asn Leu Ala
    1010                1015                1020

Ala Thr  Lys Met Ser Glu Cys  Val Leu Gly Gln Ser  Lys Arg Val
    1025                1030                1035

Asp Phe  Cys Gly Lys Gly Tyr  His Leu Met Ser Phe  Pro Gln Ser
    1040                1045                1050

Ala Pro  His Gly Val Val Phe  Leu His Val Thr Tyr  Val Pro Ala
    1055                1060                1065

Gln Glu  Lys Asn Phe Thr Thr  Ala Pro Ala Ile Cys  His Asp Gly
    1070                1075                1080

Lys Ala  His Phe Pro Arg Glu  Gly Val Phe Val Ser  Asn Gly Thr
    1085                1090                1095

His Trp  Phe Val Thr Gln Arg  Asn Phe Tyr Glu Pro  Gln Ile Ile
    1100                1105                1110

Thr Thr Asp Asn Thr Phe Val Ser Gly Asn Cys Asp Val Val Ile
    1115                1120                1125

Gly Ile Val Asn Asn Thr Val Tyr Asp Pro Leu Gln Pro Glu Leu
    1130                1135                1140

Asp Ser Phe Lys Glu Glu Leu Asp Lys Tyr Phe Lys Asn His Thr
    1145                1150                1155

Ser Pro Asp Val Asp Leu Gly Asp Ile Ser Gly Ile Asn Ala Ser
    1160                1165                1170

Val Val Asn Ile Gln Lys Glu Ile Asp Arg Leu Asn Glu Val Ala
    1175                1180                1185

Lys Asn Leu Asn Glu Ser Leu Ile Asp Leu Gln Glu Leu Gly Lys
    1190                1195                1200

Tyr Glu Gln Tyr Ile Lys Trp Pro Trp Tyr Ile Trp Leu Gly Phe
    1205                1210                1215

Ile Ala Gly Leu Ile Ala Ile Val Met Val Thr Ile Met Leu Cys
    1220                1225                1230

Cys Met Thr Ser Cys Cys Ser Cys Leu Lys Gly Cys Cys Ser Cys
    1235                1240                1245

Gly Ser Cys Cys Lys Phe Asp Glu Asp Asp Ser Glu Pro Val Leu
    1250                1255                1260

Lys Gly Val Lys Leu His Tyr Thr
    1265                1270

<210> 5
<211> 1273
<212> PRT
<213> Severe acute respiratory syndrome-related coronavirus

<220>
<221> VARIANT
<222> (484)..(484)
<223> Position 484 can also be substituted with an Q.

<400> 5

Met Phe Val Phe Leu Val Leu Leu Pro Leu Val Ser Ser Gln Cys Val
1               5                   10                  15

Asn Phe Thr Asn Arg Thr Gln Leu Pro Ser Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Tyr Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Ser Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala

275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Thr Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Lys Gly Phe Asn Cys Tyr Phe Pro Leu Gln Ser Tyr Gly
                485                 490                 495

Phe Gln Pro Thr Tyr Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Gly Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu Tyr Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Lys Val Glu Ala Glu Val Gln
            980                 985                 990

Ile Asp Arg Leu Ile Thr Gly Arg  Leu Gln Ser Leu Gln  Thr Tyr Val
        995                 1000                1005

Thr Gln  Gln Leu Ile Arg Ala  Ala Glu Ile Arg Ala  Ser Ala Asn
    1010                1015                1020

Leu Ala  Ala Ile Lys Met Ser  Glu Cys Val Leu Gly  Gln Ser Lys
    1025                1030                1035

Arg Val Asp Phe Cys Gly Lys Gly Tyr His Leu Met Ser Phe Pro
    1040                1045                1050

Gln Ser Ala Pro His Gly Val Val Phe Leu His Val Thr Tyr Val
    1055                1060                1065

Pro Ala Gln Glu Lys Asn Phe Thr Thr Ala Pro Ala Ile Cys His
    1070                1075                1080

Asp Gly Lys Ala His Phe Pro Arg Glu Gly Val Phe Val Ser Asn
    1085                1090                1095

Gly Thr His Trp Phe Val Thr Gln Arg Asn Phe Tyr Glu Pro Gln
    1100                1105                1110

Ile Ile Thr Thr Asp Asn Thr Phe Val Ser Gly Asn Cys Asp Val
    1115                1120                1125

Val Ile Gly Ile Val Asn Asn Thr Val Tyr Asp Pro Leu Gln Pro
    1130                1135                1140

Glu Leu Asp Ser Phe Lys Glu Glu Leu Asp Lys Tyr Phe Lys Asn
    1145                1150                1155

His Thr Ser Pro Asp Val Asp Leu Gly Asp Ile Ser Gly Ile Asn
    1160                1165                1170

Ala Ser Phe Val Asn Ile Gln Lys Glu Ile Asp Arg Leu Asn Glu
    1175                1180                1185

Val Ala Lys Asn Leu Asn Glu Ser Leu Ile Asp Leu Gln Glu Leu
    1190                1195                1200

Gly Lys Tyr Glu Gln Tyr Ile Lys Trp Pro Trp Tyr Ile Trp Leu
    1205                1210                1215

Gly Phe Ile Ala Gly Leu Ile Ala Ile Val Met Val Thr Ile Met
    1220                1225                1230

Leu Cys Cys Met Thr Ser Cys Cys Ser Cys Leu Lys Gly Cys Cys
    1235                1240                1245

Ser Cys Gly Ser Cys Cys Lys Phe Asp Glu Asp Asp Ser Glu Pro
    1250                1255                1260

Val Leu Lys Gly Val Lys Leu His Tyr Thr

1265                1270


<210>  6
<211>  1266
<212>  PRT
<213>  Severe acute respiratory syndrome-related coronavirus



<220>
<221>  VARIANT
<222>  (445)..(445)
<223>  Position 445 can also be substituted with an R, wherein position
       445 in C.37 Lambda refers to position 452 in WUHAN Sequence (SEQ
       ID No 1).

<400>  6

Met Phe Val Phe Leu Val Leu Leu Pro Leu Val Ser Ser Gln Cys Val
1               5                   10                  15


Asn Leu Thr Thr Arg Thr Gln Leu Pro Pro Ala Tyr Thr Asn Ser Phe
            20                  25                  30


Thr Arg Gly Val Tyr Tyr Pro Asp Lys Val Phe Arg Ser Ser Val Leu
        35                  40                  45


His Ser Thr Gln Asp Leu Phe Leu Pro Phe Phe Ser Asn Val Thr Trp
    50                  55                  60


Phe His Ala Ile His Val Ser Gly Thr Asn Val Ile Lys Arg Phe Asp
65                  70                  75                  80


Asn Pro Val Leu Pro Phe Asn Asp Gly Val Tyr Phe Ala Ser Thr Glu
                85                  90                  95


Lys Ser Asn Ile Ile Arg Gly Trp Ile Phe Gly Thr Thr Leu Asp Ser
            100                 105                 110


Lys Thr Gln Ser Leu Leu Ile Val Asn Asn Ala Thr Asn Val Val Ile
        115                 120                 125


Lys Val Cys Glu Phe Gln Phe Cys Asn Asp Pro Phe Leu Gly Val Tyr
    130                 135                 140


Tyr His Lys Asn Asn Lys Ser Trp Met Glu Ser Glu Phe Arg Val Tyr
145                 150                 155                 160


Ser Ser Ala Asn Asn Cys Thr Phe Glu Tyr Val Ser Gln Pro Phe Leu
                165                 170                 175


Met Asp Leu Glu Gly Lys Gln Gly Asn Phe Lys Asn Leu Arg Glu Phe

            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Asn Ser Ser Ser Gly Trp Thr Ala Gly Ala Ala
                245                 250                 255

Ala Tyr Tyr Val Gly Tyr Leu Gln Pro Arg Thr Phe Leu Leu Lys Tyr
            260                 265                 270

Asn Glu Asn Gly Thr Ile Thr Asp Ala Val Asp Cys Ala Leu Asp Pro
        275                 280                 285

Leu Ser Glu Thr Lys Cys Thr Leu Lys Ser Phe Thr Val Glu Lys Gly
    290                 295                 300

Ile Tyr Gln Thr Ser Asn Phe Arg Val Gln Pro Thr Glu Ser Ile Val
305                 310                 315                 320

Arg Phe Pro Asn Ile Thr Asn Leu Cys Pro Phe Gly Glu Val Phe Asn
                325                 330                 335

Ala Thr Arg Phe Ala Ser Val Tyr Ala Trp Asn Arg Lys Arg Ile Ser
            340                 345                 350

Asn Cys Val Ala Asp Tyr Ser Val Leu Tyr Asn Ser Ala Ser Phe Ser
        355                 360                 365

Thr Phe Lys Cys Tyr Gly Val Ser Pro Thr Lys Leu Asn Asp Leu Cys
    370                 375                 380

Phe Thr Asn Val Tyr Ala Asp Ser Phe Val Ile Arg Gly Asp Glu Val
385                 390                 395                 400

Arg Gln Ile Ala Pro Gly Gln Thr Gly Lys Ile Ala Asp Tyr Asn Tyr
                405                 410                 415

Lys Leu Pro Asp Asp Phe Thr Gly Cys Val Ile Ala Trp Asn Ser Asn
            420                 425                 430

Asn Leu Asp Ser Lys Val Gly Gly Asn Tyr Asn Tyr Gln Tyr Arg Leu
        435                 440                 445

Phe Arg Lys Ser Asn Leu Lys Pro Phe Glu Arg Asp Ile Ser Thr Glu
    450                 455                 460

Ile Tyr Gln Ala Gly Ser Thr Pro Cys Asn Gly Val Glu Gly Phe Asn
465                 470                 475                 480

Cys Tyr Ser Pro Leu Gln Ser Tyr Gly Phe Gln Pro Thr Asn Gly Val
                485                 490                 495

Gly Tyr Gln Pro Tyr Arg Val Val Val Leu Ser Phe Glu Leu Leu His
            500                 505                 510

Ala Pro Ala Thr Val Cys Gly Pro Lys Lys Ser Thr Asn Leu Val Lys
        515                 520                 525

Asn Lys Cys Val Asn Phe Asn Phe Asn Gly Leu Thr Gly Thr Gly Val
    530                 535                 540

Leu Thr Glu Ser Asn Lys Lys Phe Leu Pro Phe Gln Gln Phe Gly Arg
545                 550                 555                 560

Asp Ile Ala Asp Thr Thr Asp Ala Val Arg Asp Pro Gln Thr Leu Glu
                565                 570                 575

Ile Leu Asp Ile Thr Pro Cys Ser Phe Gly Gly Val Ser Val Ile Thr
            580                 585                 590

Pro Gly Thr Asn Thr Ser Asn Gln Val Ala Val Leu Tyr Gln Gly Val
        595                 600                 605

Asn Cys Thr Glu Val Pro Val Ala Ile His Ala Asp Gln Leu Thr Pro
    610                 615                 620

Thr Trp Arg Val Tyr Ser Thr Gly Ser Asn Val Phe Gln Thr Arg Ala
625                 630                 635                 640

Gly Cys Leu Ile Gly Ala Glu His Val Asn Asn Ser Tyr Glu Cys Asp
                645                 650                 655

Ile Pro Ile Gly Ala Gly Ile Cys Ala Ser Tyr Gln Thr Gln Thr Asn
            660                 665                 670

Ser Pro Arg Arg Ala Arg Ser Val Ala Ser Gln Ser Ile Ile Ala Tyr
        675                 680                 685

Thr Met Ser Leu Gly Ala Glu Asn Ser Val Ala Tyr Ser Asn Asn Ser
    690                 695                 700

Ile Ala Ile Pro Thr Asn Phe Thr Ile Ser Val Thr Thr Glu Ile Leu
705                 710                 715                 720

Pro Val Ser Met Thr Lys Thr Ser Val Asp Cys Thr Met Tyr Ile Cys
                725                 730                 735

Gly Asp Ser Thr Glu Cys Ser Asn Leu Leu Leu Gln Tyr Gly Ser Phe
            740                 745                 750

Cys Thr Gln Leu Asn Arg Ala Leu Thr Gly Ile Ala Val Glu Gln Asp
        755                 760                 765

Lys Asn Thr Gln Glu Val Phe Ala Gln Val Lys Gln Ile Tyr Lys Thr
    770                 775                 780

Pro Pro Ile Lys Asp Phe Gly Gly Phe Asn Phe Ser Gln Ile Leu Pro
785                 790                 795                 800

Asp Pro Ser Lys Pro Ser Lys Arg Ser Phe Ile Glu Asp Leu Leu Phe
                805                 810                 815

Asn Lys Val Thr Leu Ala Asp Ala Gly Phe Ile Lys Gln Tyr Gly Asp
            820                 825                 830

Cys Leu Gly Asp Ile Ala Ala Arg Asp Leu Ile Cys Ala Gln Lys Phe
        835                 840                 845

Asn Gly Leu Asn Val Leu Pro Pro Leu Leu Thr Asp Glu Met Ile Ala
    850                 855                 860

Gln Tyr Thr Ser Ala Leu Leu Ala Gly Thr Ile Thr Ser Gly Trp Thr
865                 870                 875                 880

Phe Gly Ala Gly Ala Ala Leu Gln Ile Pro Phe Ala Met Gln Met Ala
                885                 890                 895

Tyr Arg Phe Asn Gly Ile Gly Val Thr Gln Asn Val Leu Tyr Glu Asn
            900                 905                 910

Gln Lys Leu Ile Ala Asn Gln Phe Asn Ser Ala Ile Gly Lys Ile Gln
        915                 920                 925

Asp Ser Leu Ser Ser Thr Ala Ser Ala Leu Gly Lys Leu Gln Asp Val
    930                 935                 940

Val Asn Gln Asn Ala Gln Ala Leu Asn Thr Leu Val Lys Gln Leu Ser
945                 950                 955                 960

Ser Asn Phe Gly Ala Ile Ser Ser Val Leu Asn Asp Ile Leu Ser Arg
                965                 970                 975

Leu Asp Lys Val Glu Ala Glu Val Gln Ile Asp Arg Leu Ile Thr Gly
            980                 985                 990

Arg Leu Gln Ser Leu Gln Thr Tyr Val Thr Gln Gln Leu Ile Arg Ala
        995                 1000                1005

Ala Glu Ile Arg Ala Ser Ala Asn Leu Ala Ala Thr Lys Met Ser
    1010                1015                1020

Glu Cys Val Leu Gly Gln Ser Lys Arg Val Asp Phe Cys Gly Lys
    1025                1030                1035

Gly Tyr His Leu Met Ser Phe Pro Gln Ser Ala Pro His Gly Val
    1040                1045                1050

Val Phe Leu His Val Thr Tyr Val Pro Ala Gln Glu Lys Asn Phe
    1055                1060                1065

Thr Thr Ala Pro Ala Ile Cys His Asp Gly Lys Ala His Phe Pro
    1070                1075                1080

Arg Glu Gly Val Phe Val Ser Asn Gly Thr His Trp Phe Val Thr
    1085                1090                1095

Gln Arg Asn Phe Tyr Glu Pro Gln Ile Ile Thr Thr Asp Asn Thr
    1100                1105                1110

Phe Val Ser Gly Asn Cys Asp Val Val Ile Gly Ile Val Asn Asn
    1115                1120                1125

Thr Val Tyr Asp Pro Leu Gln Pro Glu Leu Asp Ser Phe Lys Glu
    1130                1135                1140

Glu Leu Asp Lys Tyr Phe Lys Asn His Thr Ser Pro Asp Val Asp
    1145                1150                1155

Leu Gly Asp Ile Ser Gly Ile Asn Ala Ser Val Val Asn Ile Gln
    1160                1165                1170

Lys Glu Ile Asp Arg Leu Asn Glu Val Ala Lys Asn Leu Asn Glu

1175                    1180                    1185

Ser Leu  Ile Asp Leu Gln Glu  Leu Gly Lys Tyr Glu  Gln Tyr Ile
    1190                    1195                    1200

Lys Trp  Pro Trp Tyr Ile Trp  Leu Gly Phe Ile Ala  Gly Leu Ile
    1205                    1210                    1215

Ala Ile  Val Met Val Thr Ile  Met Leu Cys Cys Met  Thr Ser Cys
    1220                    1225                    1230

Cys Ser  Cys Leu Lys Gly Cys  Cys Ser Cys Gly Ser  Cys Cys Lys
    1235                    1240                    1245

Phe Asp  Glu Asp Asp Ser Glu  Pro Val Leu Lys Gly  Val Lys Leu
    1250                    1255                    1260

His Tyr  Thr
    1265

<210>  7
<211>  1270
<212>  PRT
<213>  Artificial Sequence

<220>
<223>  SARS-CoV-2 spike like protein

<400>  7

Met Phe Val Phe Leu Val Leu Leu Pro Leu Val Ser Ser Gln Cys Val
1               5                   10                  15

Asn Phe Thr Asn Arg Thr Gln Leu Pro Ser Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile Ser Gly Thr Asn Gly Thr Lys Arg Phe Asp Asn Pro
65                  70                  75                  80

Val Leu Pro Phe Asn Asp Gly Val Tyr Phe Ala Ser Thr Glu Lys Ser
                85                  90                  95

Asn Ile Ile Arg Gly Trp Ile Phe Gly Thr Thr Leu Asp Ser Lys Thr
            100                 105                 110

Gln Ser Leu Leu Ile Val Asn Asn Ala Thr Asn Val Val Ile Lys Val
        115                 120                 125

Cys Glu Phe Gln Phe Cys Asn Tyr Pro Phe Leu Gly Val Tyr His Lys
    130                 135                 140

Asn Asn Lys Ser Trp Met Glu Ser Glu Phe Arg Val Tyr Ser Ser Ala
145                 150                 155                 160

Asn Asn Cys Thr Phe Glu Tyr Val Ser Gln Pro Phe Leu Met Asp Leu
                165                 170                 175

Glu Gly Lys Gln Gly Asn Phe Lys Asn Leu Ser Glu Phe Val Phe Lys
            180                 185                 190

Asn Ile Asp Gly Tyr Phe Lys Ile Tyr Ser Lys His Thr Pro Ile Asn
        195                 200                 205

Leu Val Arg Asp Leu Pro Gln Gly Phe Ser Ala Leu Glu Pro Leu Val
    210                 215                 220

Asp Leu Pro Ile Gly Ile Asn Ile Thr Arg Phe Gln Thr Leu Leu Ala
225                 230                 235                 240

Leu His Arg Ser Tyr Leu Thr Pro Gly Asp Ser Ser Ser Gly Trp Thr
                245                 250                 255

Ala Gly Ala Ala Ala Tyr Tyr Val Gly Tyr Leu Gln Pro Arg Thr Phe
            260                 265                 270

Leu Leu Lys Tyr Asn Glu Asn Gly Thr Ile Thr Asp Ala Val Asp Cys
        275                 280                 285

Ala Leu Asp Pro Leu Ser Glu Thr Lys Cys Thr Leu Lys Ser Phe Thr
    290                 295                 300

Val Glu Lys Gly Ile Tyr Gln Thr Ser Asn Phe Arg Val Gln Pro Thr
305                 310                 315                 320

Glu Ser Ile Val Arg Phe Pro Asn Ile Thr Asn Leu Cys Pro Phe Gly
                325                 330                 335

Glu Val Phe Asn Ala Thr Arg Phe Ala Ser Val Tyr Ala Trp Asn Arg
            340                 345                 350

Lys Arg Ile Ser Asn Cys Val Ala Asp Tyr Ser Val Leu Tyr Asn Ser

        355                 360                 365

Ala Ser Phe Ser Thr Phe Lys Cys Tyr Gly Val Ser Pro Thr Lys Leu
    370                 375                 380

Asn Asp Leu Cys Phe Thr Asn Val Tyr Ala Asp Ser Phe Val Ile Arg
385                 390                 395                 400

Gly Asp Glu Val Arg Gln Ile Ala Pro Gly Gln Thr Gly Thr Ile Ala
                405                 410                 415

Asp Tyr Asn Tyr Lys Leu Pro Asp Asp Phe Thr Gly Cys Val Ile Ala
            420                 425                 430

Trp Asn Ser Asn Asn Leu Asp Ser Lys Val Gly Gly Asn Tyr Asn Tyr
        435                 440                 445

Leu Tyr Arg Leu Phe Arg Lys Ser Asn Leu Lys Pro Phe Glu Arg Asp
    450                 455                 460

Ile Ser Thr Glu Ile Tyr Gln Ala Gly Ser Thr Pro Cys Asn Gly Val
465                 470                 475                 480

Lys Gly Phe Asn Cys Tyr Phe Pro Leu Gln Ser Tyr Gly Phe Gln Pro
                485                 490                 495

Thr Tyr Gly Val Gly Tyr Gln Pro Tyr Arg Val Val Val Leu Ser Phe
            500                 505                 510

Glu Leu Leu His Ala Pro Ala Thr Val Cys Gly Pro Lys Lys Ser Thr
        515                 520                 525

Asn Leu Val Lys Asn Lys Cys Val Asn Phe Asn Phe Asn Gly Leu Thr
    530                 535                 540

Gly Thr Gly Val Leu Thr Glu Ser Asn Lys Lys Phe Leu Pro Phe Gln
545                 550                 555                 560

Gln Phe Gly Arg Asp Ile Asp Asp Thr Thr Asp Ala Val Arg Asp Pro
                565                 570                 575

Gln Thr Leu Glu Ile Leu Asp Ile Thr Pro Cys Ser Phe Gly Gly Val
            580                 585                 590

Ser Val Ile Thr Pro Gly Thr Asn Thr Ser Asn Gln Val Ala Val Leu
        595                 600                 605

Tyr Gln Gly Val Asn Cys Thr Glu Val Pro Val Ala Ile His Ala Asp
    610                 615                 620

Gln Leu Thr Pro Thr Trp Arg Val Tyr Ser Thr Gly Ser Asn Val Phe
625                 630                 635                 640

Gln Thr Arg Ala Gly Cys Leu Ile Gly Ala Glu Tyr Val Asn Asn Ser
                645                 650                 655

Tyr Glu Cys Asp Ile Pro Ile Gly Ala Gly Ile Cys Ala Ser Tyr Gln
            660                 665                 670

Thr Gln Thr Asn Ser His Arg Arg Ala Arg Ser Val Ala Ser Gln Ser
        675                 680                 685

Ile Ile Ala Tyr Thr Met Ser Leu Gly Ala Glu Asn Ser Val Ala Tyr
    690                 695                 700

Ser Asn Asn Ser Ile Ala Ile Pro Ile Asn Phe Thr Ile Ser Val Thr
705                 710                 715                 720

Thr Glu Ile Leu Pro Val Ser Met Thr Lys Thr Ser Val Asp Cys Thr
                725                 730                 735

Met Tyr Ile Cys Gly Asp Ser Thr Glu Cys Ser Asn Leu Leu Leu Gln
            740                 745                 750

Tyr Gly Ser Phe Cys Thr Gln Leu Asn Arg Ala Leu Thr Gly Ile Ala
        755                 760                 765

Val Glu Gln Asp Lys Asn Thr Gln Glu Val Phe Ala Gln Val Lys Gln
    770                 775                 780

Ile Tyr Lys Thr Pro Pro Ile Lys Asp Phe Gly Gly Phe Asn Phe Ser
785                 790                 795                 800

Gln Ile Leu Pro Asp Pro Ser Lys Pro Ser Lys Arg Ser Pro Ile Glu
                805                 810                 815

Asp Leu Leu Phe Asn Lys Val Thr Leu Ala Asp Ala Gly Phe Ile Lys
            820                 825                 830

Gln Tyr Gly Asp Cys Leu Gly Asp Ile Ala Ala Arg Asp Leu Ile Cys
        835                 840                 845

Ala Gln Lys Phe Asn Gly Leu Thr Val Leu Pro Pro Leu Leu Thr Asp
    850                 855                 860

Glu Met Ile Ala Gln Tyr Thr Ser Ala Leu Leu Ala Gly Thr Ile Thr
865                 870                 875                 880

Ser Gly Trp Thr Phe Gly Ala Gly Pro Ala Leu Gln Ile Pro Phe Pro
                885                 890                 895

Met Gln Met Ala Tyr Arg Phe Asn Gly Ile Gly Val Thr Gln Asn Val
            900                 905                 910

Leu Tyr Glu Asn Gln Lys Leu Ile Ala Asn Gln Phe Asn Ser Ala Ile
        915                 920                 925

Gly Lys Ile Gln Asp Ser Leu Ser Ser Thr Pro Ser Ala Leu Gly Lys
    930                 935                 940

Leu Gln Asp Val Val Asn Gln Asn Ala Gln Ala Leu Asn Thr Leu Val
945                 950                 955                 960

Lys Gln Leu Ser Ser Asn Phe Gly Ala Ile Ser Ser Val Leu Asn Asp
                965                 970                 975

Ile Leu Ala Arg Leu Asp Pro Pro Glu Ala Glu Val Gln Ile Asp Arg
            980                 985                 990

Leu Ile Thr Gly Arg Leu Gln Ser  Leu Gln Thr Tyr Val  Thr Gln Gln
        995                 1000                1005

Leu Ile  Arg Ala Ala Glu Ile  Arg Ala Ser Ala Asn  Leu Ala Ala
    1010                1015                1020

Ile Lys  Met Ser Glu Cys Val  Leu Gly Gln Ser Lys  Arg Val Asp
    1025                1030                1035

Phe Cys  Gly Lys Gly Tyr His  Leu Met Ser Phe Pro  Gln Ser Ala
    1040                1045                1050

Pro His  Gly Val Val Phe Leu  His Val Thr Tyr Val  Pro Ala Gln
    1055                1060                1065

Glu Lys  Asn Phe Thr Thr Ala  Pro Ala Ile Cys His  Asp Gly Lys
    1070                1075                1080

Ala His  Phe Pro Arg Glu Gly  Val Phe Val Ser Asn  Gly Thr His
    1085                1090                1095

Trp Phe  Val Thr Gln Arg Asn  Phe Tyr Glu Pro Gln  Ile Ile Thr
    1100                1105                1110

Thr His Asn Thr Phe Val Ser Gly Asn Cys Asp Val Val Ile Gly
    1115                1120                1125

Ile Val Asn Asn Thr Val Tyr Asp Pro Leu Gln Pro Glu Leu Asp
    1130                1135                1140

Ser Phe Lys Glu Glu Leu Asp Lys Tyr Phe Lys Asn His Thr Ser
    1145                1150                1155

Pro Asp Val Asp Leu Gly Asp Ile Ser Gly Ile Asn Ala Ser Val
    1160                1165                1170

Val Asn Ile Gln Lys Glu Ile Asp Arg Leu Asn Glu Val Ala Lys
    1175                1180                1185

Asn Leu Asn Glu Ser Leu Ile Asp Leu Gln Glu Leu Gly Lys Tyr
    1190                1195                1200

Glu Gln Tyr Ile Lys Trp Pro Trp Tyr Ile Trp Leu Gly Phe Ile
    1205                1210                1215

Ala Gly Leu Ile Ala Ile Val Met Val Thr Ile Met Leu Cys Cys
    1220                1225                1230

Met Thr Ser Cys Cys Ser Cys Leu Lys Gly Cys Cys Ser Cys Gly
    1235                1240                1245

Ser Cys Cys Lys Phe Asp Glu Asp Asp Ser Glu Pro Val Leu Lys
    1250                1255                1260

Gly Val Lys Leu His Tyr Thr
    1265                1270

<210>  8
<211>  1270
<212>  PRT
<213>  Artificial Sequence

<220>
<223>  SARS-CoV-2 spike like protein

<400>  8

Met Phe Val Phe Leu Val Leu Leu Pro Leu Val Ser Ser Gln Cys Val
1               5                   10                  15

Asn Phe Thr Asn Arg Thr Gln Leu Pro Ser Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile Ser Gly Thr Asn Gly Thr Lys Arg Phe Asp Asn Pro
65                  70                  75                  80

Val Leu Pro Phe Asn Asp Gly Val Tyr Phe Ala Ser Thr Glu Lys Ser
                85                  90                  95

Asn Ile Ile Arg Gly Trp Ile Phe Gly Thr Thr Leu Asp Ser Lys Thr
            100                 105                 110

Gln Ser Leu Leu Ile Val Asn Asn Ala Thr Asn Val Val Ile Lys Val
        115                 120                 125

Cys Glu Phe Gln Phe Cys Asn Tyr Pro Phe Leu Gly Val Tyr His Lys
    130                 135                 140

Asn Asn Lys Ser Trp Met Glu Ser Glu Phe Arg Val Tyr Ser Ser Ala
145                 150                 155                 160

Asn Asn Cys Thr Phe Glu Tyr Val Ser Gln Pro Phe Leu Met Asp Leu
                165                 170                 175

Glu Gly Lys Gln Gly Asn Phe Lys Asn Leu Ser Glu Phe Val Phe Lys
            180                 185                 190

Asn Ile Asp Gly Tyr Phe Lys Ile Tyr Ser Lys His Thr Pro Ile Asn
        195                 200                 205

Leu Val Arg Asp Leu Pro Gln Gly Phe Ser Ala Leu Glu Pro Leu Val
    210                 215                 220

Asp Leu Pro Ile Gly Ile Asn Ile Thr Arg Phe Gln Thr Leu Leu Ala
225                 230                 235                 240

Leu His Arg Ser Tyr Leu Thr Pro Gly Asp Ser Ser Ser Gly Trp Thr
                245                 250                 255

Ala Gly Ala Ala Ala Tyr Tyr Val Gly Tyr Leu Gln Pro Arg Thr Phe
            260                 265                 270

Leu Leu Lys Tyr Asn Glu Asn Gly Thr Ile Thr Asp Ala Val Asp Cys
        275                 280                 285

Ala Leu Asp Pro Leu Ser Glu Thr Lys Cys Thr Leu Lys Ser Phe Thr
    290                 295                 300

Val Glu Lys Gly Ile Tyr Gln Thr Ser Asn Phe Arg Val Gln Pro Thr
305                 310                 315                 320

Glu Ser Ile Val Arg Phe Pro Asn Ile Thr Asn Leu Cys Pro Phe Gly
                325                 330                 335

Glu Val Phe Asn Ala Thr Arg Phe Ala Ser Val Tyr Ala Trp Asn Arg
            340                 345                 350

Lys Arg Ile Ser Asn Cys Val Ala Asp Tyr Ser Val Leu Tyr Asn Ser
        355                 360                 365

Ala Ser Phe Ser Thr Phe Lys Cys Tyr Gly Val Ser Pro Thr Lys Leu
    370                 375                 380

Asn Asp Leu Cys Phe Thr Asn Val Tyr Ala Asp Ser Phe Val Ile Arg
385                 390                 395                 400

Gly Asp Glu Val Arg Gln Ile Ala Pro Gly Gln Thr Gly Thr Ile Ala
                405                 410                 415

Asp Tyr Asn Tyr Lys Leu Pro Asp Asp Phe Thr Gly Cys Val Ile Ala
            420                 425                 430

Trp Asn Ser Asn Asn Leu Asp Ser Lys Val Gly Gly Asn Tyr Asn Tyr
        435                 440                 445

Leu Tyr Arg Leu Phe Arg Lys Ser Asn Leu Lys Pro Phe Glu Arg Asp
    450                 455                 460

Ile Ser Thr Glu Ile Tyr Gln Ala Gly Ser Thr Pro Cys Asn Gly Val
465                 470                 475                 480

Lys Gly Phe Asn Cys Tyr Phe Pro Leu Gln Ser Tyr Gly Phe Gln Pro
                485                 490                 495

Thr Tyr Gly Val Gly Tyr Gln Pro Tyr Arg Val Val Val Leu Ser Phe
            500                 505                 510

Glu Leu Leu His Ala Pro Ala Thr Val Cys Gly Pro Lys Lys Ser Thr
        515                 520                 525

Asn Leu Val Lys Asn Lys Cys Val Asn Phe Asn Phe Asn Gly Leu Thr

530                 535                 540

Gly Thr Gly Val Leu Thr Glu Ser Asn Lys Lys Phe Leu Pro Phe Gln
545                 550                 555                 560

Gln Phe Gly Arg Asp Ile Asp Asp Thr Thr Asp Ala Val Arg Asp Pro
                565                 570                 575

Gln Thr Leu Glu Ile Leu Asp Ile Thr Pro Cys Ser Phe Gly Gly Val
            580                 585                 590

Ser Val Ile Thr Pro Gly Thr Asn Thr Ser Asn Gln Val Ala Val Leu
        595                 600                 605

Tyr Gln Gly Val Asn Cys Thr Glu Val Pro Val Ala Ile His Ala Asp
    610                 615                 620

Gln Leu Thr Pro Thr Trp Arg Val Tyr Ser Thr Gly Ser Asn Val Phe
625                 630                 635                 640

Gln Thr Arg Ala Gly Cys Leu Ile Gly Ala Glu Tyr Val Asn Asn Ser
                645                 650                 655

Tyr Glu Cys Asp Ile Pro Ile Gly Ala Gly Ile Cys Ala Ser Tyr Gln
            660                 665                 670

Thr Gln Thr Asn Ser His Arg Arg Ala Arg Ser Val Ala Ser Gln Ser
        675                 680                 685

Ile Ile Ala Tyr Thr Met Ser Leu Gly Ala Glu Asn Ser Val Ala Tyr
    690                 695                 700

Ser Asn Asn Ser Ile Ala Ile Pro Ile Asn Phe Thr Ile Ser Val Thr
705                 710                 715                 720

Thr Glu Ile Leu Pro Val Ser Met Thr Lys Thr Ser Val Asp Cys Thr
                725                 730                 735

Met Tyr Ile Cys Gly Asp Ser Thr Glu Cys Ser Asn Leu Leu Leu Gln
            740                 745                 750

Tyr Gly Ser Phe Cys Thr Gln Leu Asn Arg Ala Leu Thr Gly Ile Ala
        755                 760                 765

Val Glu Gln Asp Lys Asn Thr Gln Glu Val Phe Ala Gln Val Lys Gln
    770                 775                 780

Ile Tyr Lys Thr Pro Pro Ile Lys Asp Phe Gly Gly Phe Asn Phe Ser
785                 790                 795                 800

Gln Ile Leu Pro Asp Pro Ser Lys Pro Ser Lys Arg Ser Pro Ile Glu
                805                 810                 815

Asp Leu Leu Phe Asn Lys Val Thr Leu Ala Asp Ala Gly Phe Ile Lys
            820                 825                 830

Gln Tyr Gly Asp Cys Leu Gly Asp Ile Ala Ala Arg Asp Leu Ile Cys
        835                 840                 845

Ala Gln Lys Phe Asn Gly Leu Thr Val Leu Pro Pro Leu Leu Thr Asp
    850                 855                 860

Glu Met Ile Ala Gln Tyr Thr Ser Ala Leu Leu Ala Gly Thr Ile Thr
865                 870                 875                 880

Ser Gly Trp Thr Phe Gly Ala Gly Pro Ala Leu Gln Ile Pro Phe Pro
                885                 890                 895

Met Gln Met Ala Tyr Arg Phe Asn Gly Ile Gly Val Thr Gln Asn Val
            900                 905                 910

Leu Tyr Glu Asn Gln Lys Leu Ile Ala Asn Gln Phe Asn Ser Ala Ile
        915                 920                 925

Gly Lys Ile Gln Asp Ser Leu Ser Ser Thr Pro Ser Ala Leu Gly Lys
    930                 935                 940

Leu Gln Asp Val Val Asn Gln Asn Ala Gln Ala Leu Asn Thr Leu Val
945                 950                 955                 960

Lys Gln Leu Ser Ser Asn Phe Gly Ala Ile Ser Ser Val Leu Asn Asp
                965                 970                 975

Ile Leu Ala Arg Leu Asp Pro Pro Glu Ala Glu Val Gln Ile Asp Arg
            980                 985                 990

Leu Ile Thr Gly Arg Leu Gln Ser  Leu Gln Thr Tyr Val  Thr Gln Gln
        995                 1000                1005

Leu Ile  Arg Ala Ala Glu Ile  Arg Ala Ser Ala Asn  Leu Ala Ala
    1010                1015                1020

Ile Lys  Met Ser Glu Cys Val  Leu Gly Gln Ser Lys  Arg Val Asp
    1025                1030                1035

Phe Cys Gly Lys Gly Tyr His Leu Met Ser Phe Pro Gln Ser Ala
    1040                1045                1050

Pro His Gly Val Val Phe Leu His Val Thr Tyr Val Pro Ala Gln
    1055                1060                1065

Glu Lys Asn Phe Thr Thr Ala Pro Ala Ile Cys His Asp Gly Lys
    1070                1075                1080

Ala His Phe Pro Arg Glu Gly Val Phe Val Ser Asn Gly Thr His
    1085                1090                1095

Trp Phe Val Thr Gln Arg Asn Phe Tyr Glu Pro Gln Ile Ile Thr
    1100                1105                1110

Thr His Asn Thr Phe Val Ser Gly Asn Cys Asp Val Val Ile Gly
    1115                1120                1125

Ile Val Asn Asn Thr Val Tyr Asp Pro Leu Gln Pro Glu Leu Asp
    1130                1135                1140

Ser Phe Lys Glu Glu Leu Asp Lys Tyr Phe Lys Asn His Thr Ser
    1145                1150                1155

Pro Asp Val Asp Leu Gly Asp Ile Ser Gly Ile Asn Ala Ser Val
    1160                1165                1170

Val Asn Ile Gln Lys Glu Ile Asp Arg Leu Asn Glu Val Ala Lys
    1175                1180                1185

Asn Leu Asn Glu Ser Leu Ile Asp Leu Gln Glu Leu Gly Lys Tyr
    1190                1195                1200

Glu Gln Tyr Ile Lys Trp Pro Trp Tyr Ile Trp Leu Gly Phe Ile
    1205                1210                1215

Ala Gly Leu Ile Ala Ile Val Met Val Thr Ile Met Leu Cys Cys
    1220                1225                1230

Met Thr Ser Cys Cys Ser Cys Leu Lys Gly Cys Cys Ser Cys Gly
    1235                1240                1245

Ser Cys Cys Lys Phe Asp Glu Asp Asp Ser Glu Pro Val Leu Lys
    1250                1255                1260

Gly Val Lys Leu His Tyr Thr
    1265                1270

<210>  9
<211>  1261
<212>  PRT
<213>  Artificial Sequence

<220>
<223>  SARS-CoV-2 spike like protein

<400>  9

Met Phe Val Phe Leu Val Leu Leu Pro Leu Val Ser Ser Gln Cys Val
1               5                   10                  15

Asn Phe Arg Asn Arg Thr Gln Leu Pro Ser Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile Ser Gly Thr Asn Val Ile Lys Arg Phe Asp Asn Pro
65                  70                  75                  80

Val Leu Pro Phe Asn Asp Gly Val Tyr Phe Ala Ser Thr Glu Lys Ser
                85                  90                  95

Asn Ile Ile Arg Gly Trp Ile Phe Gly Thr Thr Leu Asp Ser Lys Thr
            100                 105                 110

Gln Ser Leu Leu Ile Val Asn Asn Ala Thr Asn Val Val Ile Lys Val
        115                 120                 125

Cys Glu Phe Gln Phe Cys Asn Tyr Pro Phe Leu Gly Val Tyr His Lys
    130                 135                 140

Asn Asn Lys Ser Trp Met Glu Ser Gly Val Tyr Ser Ser Ala Asn Asn
145                 150                 155                 160

Cys Thr Phe Glu Tyr Val Ser Gln Pro Phe Leu Met Asp Leu Glu Gly
                165                 170                 175

Lys Gln Gly Asn Phe Lys Asn Leu Ser Glu Phe Val Phe Lys Asn Ile
            180                 185                 190

Asp Gly Tyr Phe Lys Ile Tyr Ser Lys His Thr Pro Ile Asn Leu Val
        195                 200                 205

Arg Gly Leu Pro Gln Gly Phe Ser Ala Leu Glu Pro Leu Val Asp Leu
    210                 215                 220

Pro Ile Gly Ile Asn Ile Thr Arg Phe Gln Thr Leu Leu Ala Leu His
225                 230                 235                 240

Asn Ser Ser Ser Gly Trp Thr Ala Gly Ala Ala Ala Tyr Tyr Val Gly
                245                 250                 255

Tyr Leu Gln Pro Arg Thr Phe Leu Leu Lys Tyr Asn Glu Asn Gly Thr
            260                 265                 270

Ile Thr Asp Ala Val Asp Cys Ala Leu Asp Pro Leu Ser Glu Thr Lys
        275                 280                 285

Cys Thr Leu Lys Ser Phe Thr Val Glu Lys Gly Ile Tyr Gln Thr Ser
    290                 295                 300

Asn Phe Arg Val Gln Pro Thr Glu Ser Ile Val Arg Phe Pro Asn Ile
305                 310                 315                 320

Thr Asn Leu Cys Pro Phe Gly Glu Val Phe Asn Ala Thr Arg Phe Ala
                325                 330                 335

Ser Val Tyr Ala Trp Asn Arg Lys Arg Ile Ser Asn Cys Val Ala Asp
            340                 345                 350

Tyr Ser Val Leu Tyr Asn Ser Ala Ser Phe Ser Thr Phe Lys Cys Tyr
        355                 360                 365

Gly Val Ser Pro Thr Lys Leu Asn Asp Leu Cys Phe Thr Asn Val Tyr
    370                 375                 380

Ala Asp Ser Phe Val Ile Arg Gly Asp Glu Val Arg Gln Ile Ala Pro
385                 390                 395                 400

Gly Gln Thr Gly Thr Ile Ala Asp Tyr Asn Tyr Lys Leu Pro Asp Asp
                405                 410                 415

Phe Thr Gly Cys Val Ile Ala Trp Asn Ser Asn Asn Leu Asp Ser Lys
            420                 425                 430

Val Gly Gly Asn Tyr Asn Tyr Arg Tyr Arg Leu Phe Arg Lys Ser Asn
        435                 440                 445

Leu Lys Pro Phe Glu Arg Asp Ile Ser Thr Glu Ile Tyr Gln Ala Gly
    450                 455                 460

Ser Lys Pro Cys Asn Gly Val Gln Gly Phe Asn Cys Tyr Ser Pro Leu
465                 470                 475                 480

Gln Ser Tyr Gly Phe Gln Pro Thr Tyr Gly Val Gly Tyr Gln Pro Tyr
                485                 490                 495

Arg Val Val Val Leu Ser Phe Glu Leu Leu His Ala Pro Ala Thr Val
            500                 505                 510

Cys Gly Pro Lys Lys Ser Thr Asn Leu Val Lys Asn Lys Cys Val Asn
        515                 520                 525

Phe Asn Phe Asn Gly Leu Thr Gly Thr Gly Val Leu Thr Glu Ser Asn
    530                 535                 540

Lys Lys Phe Leu Pro Phe Gln Gln Phe Gly Arg Asp Ile Ala Asp Thr
545                 550                 555                 560

Thr Asp Ala Val Arg Asp Pro Gln Thr Leu Glu Ile Leu Asp Ile Thr
                565                 570                 575

Pro Cys Ser Phe Gly Gly Val Ser Val Ile Thr Pro Gly Thr Asn Thr
            580                 585                 590

Ser Asn Gln Val Ala Val Leu Tyr Gln Gly Val Asn Cys Thr Glu Val
        595                 600                 605

Pro Val Ala Ile His Ala Asp Gln Leu Thr Pro Thr Trp Arg Val Tyr
    610                 615                 620

Ser Thr Gly Ser Asn Val Phe Gln Thr Arg Ala Gly Cys Leu Ile Gly
625                 630                 635                 640

Ala Glu Tyr Val Asn Asn Ser Tyr Glu Cys Asp Ile Pro Ile Gly Ala
                645                 650                 655

Gly Ile Cys Ala Ser Tyr Gln Thr Gln Thr Asn Ser Arg Arg Arg Ala
            660                 665                 670

Arg Ser Val Ala Ser Gln Ser Ile Ile Ala Tyr Thr Met Ser Leu Gly
        675                 680                 685

Ala Glu Asn Ser Val Ala Tyr Ser Asn Asn Ser Ile Ala Ile Pro Ile
    690                 695                 700

Asn Phe Thr Ile Ser Val Thr Thr Glu Ile Leu Pro Val Ser Met Thr

705                 710                 715                 720

Lys Thr Ser Val Asp Cys Thr Met Tyr Ile Cys Gly Asp Ser Thr Glu
                725                 730                 735

Cys Ser Asn Leu Leu Leu Gln Tyr Gly Ser Phe Cys Thr Gln Leu Asn
            740                 745                 750

Arg Ala Leu Thr Gly Ile Ala Val Glu Gln Asp Lys Asn Thr Gln Glu
        755                 760                 765

Val Phe Ala Gln Val Lys Gln Ile Tyr Lys Thr Pro Pro Ile Lys Asp
    770                 775                 780

Phe Gly Gly Phe Asn Phe Ser Gln Ile Leu Pro Asp Pro Ser Lys Pro
785                 790                 795                 800

Ser Lys Arg Ser Pro Ile Glu Asp Leu Leu Phe Asn Lys Val Thr Leu
                805                 810                 815

Ala Asp Ala Gly Phe Ile Lys Gln Tyr Gly Asp Cys Leu Gly Asp Ile
            820                 825                 830

Ala Ala Arg Asp Leu Ile Cys Ala Gln Lys Phe Asn Gly Leu Asn Val
        835                 840                 845

Leu Pro Pro Leu Leu Thr Asp Glu Met Ile Ala Gln Tyr Thr Ser Ala
    850                 855                 860

Leu Leu Ala Gly Thr Ile Thr Ser Gly Trp Thr Phe Gly Ala Gly Pro
865                 870                 875                 880

Ala Leu Gln Ile Pro Phe Pro Met Gln Met Ala Tyr Arg Phe Asn Gly
                885                 890                 895

Ile Gly Val Thr Gln Asn Val Leu Tyr Glu Asn Gln Lys Leu Ile Ala
            900                 905                 910

Asn Gln Phe Asn Ser Ala Ile Gly Lys Ile Gln Asp Ser Leu Ser Ser
        915                 920                 925

Thr Pro Ser Ala Leu Gly Lys Leu Gln Asn Val Val Asn Gln Asn Ala
    930                 935                 940

Gln Ala Leu Asn Thr Leu Val Lys Gln Leu Ser Ser Asn Phe Gly Ala
945                 950                 955                 960

Ile Ser Ser Val Leu Asn Asp Ile Leu Ala Arg Leu Asp Pro Pro Glu
                965                 970                 975

Ala Glu Val Gln Ile Asp Arg Leu Ile Thr Gly Arg Leu Gln Ser Leu
            980                 985                 990

Gln Thr Tyr Val Thr Gln Gln Leu  Ile Arg Ala Ala Glu  Ile Arg Ala
        995                 1000                 1005

Ser Ala  Asn Leu Ala Ala Ile  Lys Met Ser Glu Cys  Val Leu Gly
    1010                 1015                 1020

Gln Ser  Lys Arg Val Asp Phe  Cys Gly Lys Gly Tyr  His Leu Met
    1025                 1030                 1035

Ser Phe  Pro Gln Ser Ala Pro  His Gly Val Val Phe  Leu His Val
    1040                 1045                 1050

Thr Tyr  Val Pro Ala Gln Glu  Lys Asn Phe Thr Thr  Ala Pro Ala
    1055                 1060                 1065

Ile Cys  His Asp Gly Lys Ala  His Phe Pro Arg Glu  Gly Val Phe
    1070                 1075                 1080

Val Ser  Asn Gly Thr His Trp  Phe Val Thr Gln Arg  Asn Phe Tyr
    1085                 1090                 1095

Glu Pro  Gln Ile Ile Thr Thr  His Asn Thr Phe Val  Ser Gly Asn
    1100                 1105                 1110

Cys Asp  Val Val Ile Gly Ile  Val Asn Asn Thr Val  Tyr Asp Pro
    1115                 1120                 1125

Leu Gln  Pro Glu Leu Asp Ser  Phe Lys Glu Glu Leu  Asp Lys Tyr
    1130                 1135                 1140

Phe Lys  Asn His Thr Ser Pro  Asp Val Asp Leu Gly  Asp Ile Ser
    1145                 1150                 1155

Gly Ile  Asn Ala Ser Phe Val  Asn Ile Gln Lys Glu  Ile Asp Arg
    1160                 1165                 1170

Leu Asn  Glu Val Ala Lys Asn  Leu Asn Glu Ser Leu  Ile Asp Leu
    1175                 1180                 1185

Gln Glu  Leu Gly Lys Tyr Glu  Gln Tyr Ile Lys Trp  Pro Trp Tyr
    1190                 1195                 1200

Ile Trp Leu Gly Phe Ile Ala Gly Leu Ile Ala Ile Val Met Val
    1205                1210                1215

Thr Ile Met Leu Cys Cys Met Thr Ser Cys Cys Ser Cys Leu Lys
    1220                1225                1230

Gly Cys Cys Ser Cys Gly Ser Cys Cys Lys Phe Asp Glu Asp Asp
    1235                1240                1245

Ser Glu Pro Val Leu Lys Gly Val Lys Leu His Tyr Thr
    1250                1255                1260

<210> 10
<211> 1268
<212> PRT
<213> Artificial Sequence

<220>
<223> SARS-CoV-2 spike like protein

<400> 10

Met Phe Val Phe Leu Val Leu Leu Pro Leu Val Ser Ser Gln Cys Val
1               5                   10                  15

Asn Phe Arg Asn Arg Thr Gln Leu Pro Ser Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile Ser Gly Thr Asn Gly Thr Lys Arg Phe Asp Asn Pro
65                  70                  75                  80

Val Leu Pro Phe Asn Asp Gly Val Tyr Phe Ala Ser Thr Glu Lys Ser
                85                  90                  95

Asn Ile Ile Arg Gly Trp Ile Phe Gly Thr Thr Leu Asp Ser Lys Thr
            100                 105                 110

Gln Ser Leu Leu Ile Val Asn Asn Ala Thr Asn Val Val Ile Lys Val
        115                 120                 125

Cys Glu Phe Gln Phe Cys Asn Tyr Pro Phe Leu Gly Val Tyr His Lys
    130                 135                 140

Asn Asn Lys Ser Trp Met Glu Ser Glu Val Tyr Ser Ser Ala Asn Asn
145                 150                 155                 160

Cys Thr Phe Glu Tyr Val Ser Gln Pro Phe Leu Met Asp Leu Glu Gly
                165                 170                 175

Lys Gln Gly Asn Phe Lys Asn Leu Ser Glu Phe Val Phe Lys Asn Ile
            180                 185                 190

Asp Gly Tyr Phe Lys Ile Tyr Ser Lys His Thr Pro Ile Asn Leu Val
        195                 200                 205

Arg Asp Leu Pro Gln Gly Phe Ser Ala Leu Glu Pro Leu Val Asp Leu
    210                 215                 220

Pro Ile Gly Ile Asn Ile Thr Arg Phe Gln Thr Leu Leu Ala Leu His
225                 230                 235                 240

Arg Ser Tyr Leu Thr Pro Gly Asp Ser Ser Ser Gly Trp Thr Ala Gly
                245                 250                 255

Ala Ala Ala Tyr Tyr Val Gly Tyr Leu Gln Pro Arg Thr Phe Leu Leu
            260                 265                 270

Lys Tyr Asn Glu Asn Gly Thr Ile Thr Asp Ala Val Asp Cys Ala Leu
        275                 280                 285

Asp Pro Leu Ser Glu Thr Lys Cys Thr Leu Lys Ser Phe Thr Val Glu
    290                 295                 300

Lys Gly Ile Tyr Gln Thr Ser Asn Phe Arg Val Gln Pro Thr Glu Ser
305                 310                 315                 320

Ile Val Arg Phe Pro Asn Ile Thr Asn Leu Cys Pro Phe Gly Glu Val
                325                 330                 335

Phe Asn Ala Thr Arg Phe Ala Ser Val Tyr Ala Trp Asn Arg Lys Arg
            340                 345                 350

Ile Ser Asn Cys Val Ala Asp Tyr Ser Val Leu Tyr Asn Ser Ala Ser
        355                 360                 365

Phe Ser Thr Phe Lys Cys Tyr Gly Val Ser Pro Thr Lys Leu Asn Asp
    370                 375                 380

Leu Cys Phe Thr Asn Val Tyr Ala Asp Ser Phe Val Ile Arg Gly Asp
385                 390                 395                 400

Glu Val Arg Gln Ile Ala Pro Gly Gln Thr Gly Thr Ile Ala Asp Tyr
                405                 410                 415

Asn Tyr Lys Leu Pro Asp Asp Phe Thr Gly Cys Val Ile Ala Trp Asn
            420                 425                 430

Ser Asn Asn Leu Asp Ser Lys Val Gly Gly Asn Tyr Asn Tyr Arg Tyr
        435                 440                 445

Arg Leu Phe Arg Lys Ser Asn Leu Lys Pro Phe Glu Arg Asp Ile Ser
    450                 455                 460

Thr Glu Ile Tyr Gln Ala Gly Ser Lys Pro Cys Asn Gly Val Lys Gly
465                 470                 475                 480

Phe Asn Cys Tyr Phe Pro Leu Gln Ser Tyr Gly Phe Gln Pro Thr Tyr
                485                 490                 495

Gly Val Gly Tyr Gln Pro Tyr Arg Val Val Val Leu Ser Phe Glu Leu
            500                 505                 510

Leu His Ala Pro Ala Thr Val Cys Gly Pro Lys Lys Ser Thr Asn Leu
        515                 520                 525

Val Lys Asn Lys Cys Val Asn Phe Asn Phe Asn Gly Leu Thr Gly Thr
    530                 535                 540

Gly Val Leu Thr Glu Ser Asn Lys Lys Phe Leu Pro Phe Gln Gln Phe
545                 550                 555                 560

Gly Arg Asp Ile Asp Asp Thr Thr Asp Ala Val Arg Asp Pro Gln Thr
                565                 570                 575

Leu Glu Ile Leu Asp Ile Thr Pro Cys Ser Phe Gly Gly Val Ser Val
            580                 585                 590

Ile Thr Pro Gly Thr Asn Thr Ser Asn Gln Val Ala Val Leu Tyr Gln
        595                 600                 605

Gly Val Asn Cys Thr Glu Val Pro Val Ala Ile His Ala Asp Gln Leu
    610                 615                 620

Thr Pro Thr Trp Arg Val Tyr Ser Thr Gly Ser Asn Val Phe Gln Thr
625                 630                 635                 640

Arg Ala Gly Cys Leu Ile Gly Ala Glu Tyr Val Asn Asn Ser Tyr Glu
                645                 650                 655

Cys Asp Ile Pro Ile Gly Ala Gly Ile Cys Ala Ser Tyr Gln Thr Gln
            660                 665                 670

Thr Asn Ser His Arg Arg Ala Arg Ser Val Ala Ser Gln Ser Ile Ile
        675                 680                 685

Ala Tyr Thr Met Ser Leu Gly Ala Glu Asn Ser Val Ala Tyr Ser Asn
    690                 695                 700

Asn Ser Ile Ala Ile Pro Ile Asn Phe Thr Ile Ser Val Thr Thr Glu
705                 710                 715                 720

Ile Leu Pro Val Ser Met Thr Lys Thr Ser Val Asp Cys Thr Met Tyr
                725                 730                 735

Ile Cys Gly Asp Ser Thr Glu Cys Ser Asn Leu Leu Leu Gln Tyr Gly
            740                 745                 750

Ser Phe Cys Thr Gln Leu Asn Arg Ala Leu Thr Gly Ile Ala Val Glu
        755                 760                 765

Gln Asp Lys Asn Thr Gln Glu Val Phe Ala Gln Val Lys Gln Ile Tyr
    770                 775                 780

Lys Thr Pro Pro Ile Lys Asp Phe Gly Gly Phe Asn Phe Ser Gln Ile
785                 790                 795                 800

Leu Pro Asp Pro Ser Lys Pro Ser Lys Arg Ser Pro Ile Glu Asp Leu
                805                 810                 815

Leu Phe Asn Lys Val Thr Leu Ala Asp Ala Gly Phe Ile Lys Gln Tyr
            820                 825                 830

Gly Asp Cys Leu Gly Asp Ile Ala Ala Arg Asp Leu Ile Cys Ala Gln
        835                 840                 845

Lys Phe Asn Gly Leu Thr Val Leu Pro Pro Leu Leu Thr Asp Glu Met
    850                 855                 860

Ile Ala Gln Tyr Thr Ser Ala Leu Leu Ala Gly Thr Ile Thr Ser Gly
865                 870                 875                 880

Trp Thr Phe Gly Ala Gly Pro Ala Leu Gln Ile Pro Phe Pro Met Gln
                885                 890                 895

Met Ala Tyr Arg Phe Asn Gly Ile Gly Val Thr Gln Asn Val Leu Tyr

            900                 905                 910

Glu Asn Gln Lys Leu Ile Ala Asn Gln Phe Asn Ser Ala Ile Gly Lys
        915                 920                 925

Ile Gln Asp Ser Leu Ser Ser Thr Pro Ser Ala Leu Gly Lys Leu Gln
    930                 935                 940

Asn Val Val Asn Gln Asn Ala Gln Ala Leu Asn Thr Leu Val Lys Gln
945                 950                 955                 960

Leu Ser Ser Asn Phe Gly Ala Ile Ser Ser Val Leu Asn Asp Ile Leu
                965                 970                 975

Ala Arg Leu Asp Pro Pro Glu Ala Glu Val Gln Ile Asp Arg Leu Ile
            980                 985                 990

Thr Gly Arg Leu Gln Ser Leu Gln Thr Tyr Val Thr Gln Gln Leu Ile
        995                 1000                1005

Arg Ala Ala Glu Ile Arg Ala Ser Ala Asn Leu Ala Ala Ile Lys
    1010                1015                1020

Met Ser Glu Cys Val Leu Gly Gln Ser Lys Arg Val Asp Phe Cys
    1025                1030                1035

Gly Lys Gly Tyr His Leu Met Ser Phe Pro Gln Ser Ala Pro His
    1040                1045                1050

Gly Val Val Phe Leu His Val Thr Tyr Val Pro Ala Gln Glu Lys
    1055                1060                1065

Asn Phe Thr Thr Ala Pro Ala Ile Cys His Asp Gly Lys Ala His
    1070                1075                1080

Phe Pro Arg Glu Gly Val Phe Val Ser Asn Gly Thr His Trp Phe
    1085                1090                1095

Val Thr Gln Arg Asn Phe Tyr Glu Pro Gln Ile Ile Thr Thr His
    1100                1105                1110

Asn Thr Phe Val Ser Gly Asn Cys Asp Val Val Ile Gly Ile Val
    1115                1120                1125

Asn Asn Thr Val Tyr Asp Pro Leu Gln Pro Glu Leu Asp Ser Phe
    1130                1135                1140

Lys Glu Glu Leu Asp Lys Tyr Phe Lys Asn His Thr Ser Pro Asp
    1145                1150                1155

Val Asp Leu Gly Asp Ile Ser Gly Ile Asn Ala Ser Val Val Asn
    1160                1165                1170

Ile Gln Lys Glu Ile Asp Arg Leu Asn Glu Val Ala Lys Asn Leu
    1175                1180                1185

Asn Glu Ser Leu Ile Asp Leu Gln Glu Leu Gly Lys Tyr Glu Gln
    1190                1195                1200

Tyr Ile Lys Trp Pro Trp Tyr Ile Trp Leu Gly Phe Ile Ala Gly
    1205                1210                1215

Leu Ile Ala Ile Val Met Val Thr Ile Met Leu Cys Cys Met Thr
    1220                1225                1230

Ser Cys Cys Ser Cys Leu Lys Gly Cys Cys Ser Cys Gly Ser Cys
    1235                1240                1245

Cys Lys Phe Asp Glu Asp Asp Ser Glu Pro Val Leu Lys Gly Val
    1250                1255                1260

Lys Leu His Tyr Thr
    1265

<210>  11
<211>  1271
<212>  PRT
<213>  Severe acute respiratory syndrome-related coronavirus

<400>  11

Met Phe Val Phe Leu Val Leu Leu Pro Leu Val Ser Ser Gln Cys Val
1               5                   10                  15

Asn Leu Arg Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Val Tyr Ser Ser
145                 150                 155                 160

Ala Asn Asn Cys Thr Phe Glu Tyr Val Ser Gln Pro Phe Leu Met Asp
                165                 170                 175

Leu Glu Gly Lys Gln Gly Asn Phe Lys Asn Leu Arg Glu Phe Val Phe
            180                 185                 190

Lys Asn Ile Asp Gly Tyr Phe Lys Ile Tyr Ser Lys His Thr Pro Ile
        195                 200                 205

Asn Leu Val Arg Asp Leu Pro Gln Gly Phe Ser Ala Leu Glu Pro Leu
    210                 215                 220

Val Asp Leu Pro Ile Gly Ile Asn Ile Thr Arg Phe Gln Thr Leu Leu
225                 230                 235                 240

Ala Leu His Arg Ser Tyr Leu Thr Pro Gly Asp Ser Ser Ser Gly Trp
                245                 250                 255

Thr Ala Gly Ala Ala Ala Tyr Tyr Val Gly Tyr Leu Gln Pro Arg Thr
            260                 265                 270

Phe Leu Leu Lys Tyr Asn Glu Asn Gly Thr Ile Thr Asp Ala Val Asp
        275                 280                 285

Cys Ala Leu Asp Pro Leu Ser Glu Thr Lys Cys Thr Leu Lys Ser Phe
    290                 295                 300

Thr Val Glu Lys Gly Ile Tyr Gln Thr Ser Asn Phe Arg Val Gln Pro
305                 310                 315                 320

Thr Glu Ser Ile Val Arg Phe Pro Asn Ile Thr Asn Leu Cys Pro Phe
                325                 330                 335

Gly Glu Val Phe Asn Ala Thr Arg Phe Ala Ser Val Tyr Ala Trp Asn
        340                 345                 350

Arg Lys Arg Ile Ser Asn Cys Val Ala Asp Tyr Ser Val Leu Tyr Asn
    355                 360                 365

Ser Ala Ser Phe Ser Thr Phe Lys Cys Tyr Gly Val Ser Pro Thr Lys
370                 375                 380

Leu Asn Asp Leu Cys Phe Thr Asn Val Tyr Ala Asp Ser Phe Val Ile
385                 390                 395                 400

Arg Gly Asp Glu Val Arg Gln Ile Ala Pro Gly Gln Thr Gly Asn Ile
                405                 410                 415

Ala Asp Tyr Asn Tyr Lys Leu Pro Asp Asp Phe Thr Gly Cys Val Ile
            420                 425                 430

Ala Trp Asn Ser Asn Asn Leu Asp Ser Lys Val Gly Gly Asn Tyr Asn
        435                 440                 445

Tyr Arg Tyr Arg Leu Phe Arg Lys Ser Asn Leu Lys Pro Phe Glu Arg
    450                 455                 460

Asp Ile Ser Thr Glu Ile Tyr Gln Ala Gly Ser Lys Pro Cys Asn Gly
465                 470                 475                 480

Val Glu Gly Phe Asn Cys Tyr Phe Pro Leu Gln Ser Tyr Gly Phe Gln
                485                 490                 495

Pro Thr Asn Gly Val Gly Tyr Gln Pro Tyr Arg Val Val Val Leu Ser
            500                 505                 510

Phe Glu Leu Leu His Ala Pro Ala Thr Val Cys Gly Pro Lys Lys Ser
        515                 520                 525

Thr Asn Leu Val Lys Asn Lys Cys Val Asn Phe Asn Phe Asn Gly Leu
    530                 535                 540

Thr Gly Thr Gly Val Leu Thr Glu Ser Asn Lys Lys Phe Leu Pro Phe
545                 550                 555                 560

Gln Gln Phe Gly Arg Asp Ile Ala Asp Thr Thr Asp Ala Val Arg Asp
                565                 570                 575

Pro Gln Thr Leu Glu Ile Leu Asp Ile Thr Pro Cys Ser Phe Gly Gly
            580                 585                 590

Val Ser Val Ile Thr Pro Gly Thr Asn Thr Ser Asn Gln Val Ala Val
        595                 600                 605

Leu Tyr Gln Gly Val Asn Cys Thr Glu Val Pro Val Ala Ile His Ala
    610                 615                 620

Asp Gln Leu Thr Pro Thr Trp Arg Val Tyr Ser Thr Gly Ser Asn Val
625                 630                 635                 640

Phe Gln Thr Arg Ala Gly Cys Leu Ile Gly Ala Glu His Val Asn Asn
                645                 650                 655

Ser Tyr Glu Cys Asp Ile Pro Ile Gly Ala Gly Ile Cys Ala Ser Tyr
            660                 665                 670

Gln Thr Gln Thr Asn Ser Arg Arg Arg Ala Arg Ser Val Ala Ser Gln
        675                 680                 685

Ser Ile Ile Ala Tyr Thr Met Ser Leu Gly Ala Glu Asn Ser Val Ala
    690                 695                 700

Tyr Ser Asn Asn Ser Ile Ala Ile Pro Thr Asn Phe Thr Ile Ser Val
705                 710                 715                 720

Thr Thr Glu Ile Leu Pro Val Ser Met Thr Lys Thr Ser Val Asp Cys
                725                 730                 735

Thr Met Tyr Ile Cys Gly Asp Ser Thr Glu Cys Ser Asn Leu Leu Leu
            740                 745                 750

Gln Tyr Gly Ser Phe Cys Thr Gln Leu Asn Arg Ala Leu Thr Gly Ile
        755                 760                 765

Ala Val Glu Gln Asp Lys Asn Thr Gln Glu Val Phe Ala Gln Val Lys
    770                 775                 780

Gln Ile Tyr Lys Thr Pro Pro Ile Lys Asp Phe Gly Gly Phe Asn Phe
785                 790                 795                 800

Ser Gln Ile Leu Pro Asp Pro Ser Lys Pro Ser Lys Arg Ser Phe Ile
                805                 810                 815

Glu Asp Leu Leu Phe Asn Lys Val Thr Leu Ala Asp Ala Gly Phe Ile
            820                 825                 830

Lys Gln Tyr Gly Asp Cys Leu Gly Asp Ile Ala Ala Arg Asp Leu Ile

        835                 840                 845

Cys Ala Gln Lys Phe Asn Gly Leu Thr Val Leu Pro Pro Leu Leu Thr
    850                 855                 860

Asp Glu Met Ile Ala Gln Tyr Thr Ser Ala Leu Leu Ala Gly Thr Ile
865                 870                 875                 880

Thr Ser Gly Trp Thr Phe Gly Ala Gly Ala Ala Leu Gln Ile Pro Phe
                885                 890                 895

Ala Met Gln Met Ala Tyr Arg Phe Asn Gly Ile Gly Val Thr Gln Asn
            900                 905                 910

Val Leu Tyr Glu Asn Gln Lys Leu Ile Ala Asn Gln Phe Asn Ser Ala
        915                 920                 925

Ile Gly Lys Ile Gln Asp Ser Leu Ser Ser Thr Ala Ser Ala Leu Gly
    930                 935                 940

Lys Leu Gln Asn Val Val Asn Gln Asn Ala Gln Ala Leu Asn Thr Leu
945                 950                 955                 960

Val Lys Gln Leu Ser Ser Asn Phe Gly Ala Ile Ser Ser Val Leu Asn
                965                 970                 975

Asp Ile Leu Ser Arg Leu Asp Lys Val Glu Ala Glu Val Gln Ile Asp
            980                 985                 990

Arg Leu Ile Thr Gly Arg Leu Gln Ser Leu Gln Thr Tyr Val Thr Gln
        995                 1000                1005

Gln Leu Ile Arg Ala Ala Glu Ile Arg Ala Ser Ala Asn Leu Ala
    1010                1015                1020

Ala Thr Lys Met Ser Glu Cys Val Leu Gly Gln Ser Lys Arg Val
    1025                1030                1035

Asp Phe Cys Gly Lys Gly Tyr His Leu Met Ser Phe Pro Gln Ser
    1040                1045                1050

Ala Pro His Gly Val Val Phe Leu His Val Thr Tyr Val Pro Ala
    1055                1060                1065

Gln Glu Lys Asn Phe Thr Thr Ala Pro Ala Ile Cys His Asp Gly
    1070                1075                1080

Lys Ala His Phe Pro Arg Glu Gly Val Phe Val Ser Asn Gly Thr
    1085                1090                1095

His Trp Phe Val Thr Gln Arg Asn Phe Tyr Glu Pro Gln Ile Ile
    1100                1105                1110

Thr Thr Asp Asn Thr Phe Val Ser Gly Asn Cys Asp Val Val Ile
    1115                1120                1125

Gly Ile Val Asn Asn Thr Val Tyr Asp Pro Leu Gln Pro Glu Leu
    1130                1135                1140

Asp Ser Phe Lys Glu Glu Leu Asp Lys Tyr Phe Lys Asn His Thr
    1145                1150                1155

Ser Pro Asp Val Asp Leu Gly Asp Ile Ser Gly Ile Asn Ala Ser
    1160                1165                1170

Val Val Asn Ile Gln Lys Glu Ile Asp Arg Leu Asn Glu Val Ala
    1175                1180                1185

Lys Asn Leu Asn Glu Ser Leu Ile Asp Leu Gln Glu Leu Gly Lys
    1190                1195                1200

Tyr Glu Gln Tyr Ile Lys Trp Pro Trp Tyr Ile Trp Leu Gly Phe
    1205                1210                1215

Ile Ala Gly Leu Ile Ala Ile Val Met Val Thr Ile Met Leu Cys
    1220                1225                1230

Cys Met Thr Ser Cys Cys Ser Cys Leu Lys Gly Cys Cys Ser Cys
    1235                1240                1245

Gly Ser Cys Cys Lys Phe Asp Glu Asp Asp Ser Glu Pro Val Leu
    1250                1255                1260

Lys Gly Val Lys Leu His Tyr Thr
    1265                1270

<210> 12
<211> 3822
<212> DNA
<213> Severe acute respiratory syndrome-related coronavirus

<400> 12
atgtttgttt ttcttgtttt attgccacta gtctctagtc agtgtgttaa tcttacaacc 60

agaactcaat taccccctgc atacactaat tctttcacac gtggtgttta ttaccctgac 120

aaagttttca gatcctcagt tttacattca actcaggact tgttcttacc tttcttttcc 180

aatgttactt ggttccatgc tatacatgtc tctgggacca atggtactaa gaggtttgat 240

aaccctgtcc taccatttaa tgatggtgtt tattttgctt ccactgagaa gtctaacata 300

ataagaggct ggatttttgg tactacttta gattcgaaga cccagtccct acttattgtt 360

aataacgcta ctaatgttgt tattaaagtc tgtgaatttc aattttgtaa tgatccattt 420

ttgggtgttt attaccacaa aaacaacaaa agttggatgg aaagtgagtt cagagtttat 480

tctagtgcga ataattgcac ttttgaatat gtctctcagc cttttcttat ggaccttgaa 540

ggaaaacagg gtaatttcaa aaatcttagg gaatttgtgt ttaagaatat tgatggttat 600

tttaaaatat attctaagca cacgcctatt aatttagtgc gtgatctccc tcagggtttt 660

tcggctttag aaccattggt agatttgcca ataggtatta acatcactag gtttcaaact 720

ttacttgctt tacatagaag ttatttgact cctggtgatt cttcttcagg ttggacagct 780

ggtgctgcag cttattatgt gggttatctt caacctagga cttttctatt aaaatataat 840

gaaaatggaa ccattacaga tgctgtagac tgtgcacttg accctctctc agaaacaaag 900

tgtacgttga aatccttcac tgtagaaaaa ggaatctatc aaacttctaa ctttagagtc 960

caaccaacag aatctattgt tagatttcct aatattacaa acttgtgccc ttttggtgaa 1020

gtttttaacg ccaccagatt tgcatctgtt tatgcttgga acaggaagag aatcagcaac 1080

tgtgttgctg attattctgt cctatataat tccgcatcat tttccacttt taagtgttat 1140

ggagtgtctc ctactaaatt aaatgatctc tgctttacta atgtctatgc agattcattt 1200

gtaattagag gtgatgaagt cagacaaatc gctccagggc aaactggaaa gattgctgat 1260

tataattata aattaccaga tgattttaca ggctgcgtta tagcttggaa ttctaacaat 1320

cttgattcta aggttggtgg taattataat tacctgtata gattgtttag gaagtctaat 1380

ctcaaacctt ttgagagaga tatttcaact gaaatctatc aggccggtag cacaccttgt 1440

aatggtgttg aaggttttaa ttgttacttt cctttacaat catatggttt ccaacccact 1500

aatggtgttg gttaccaacc atacagagta gtagtacttt cttttgaact tctacatgca 1560

ccagcaactg tttgtggacc taaaaagtct actaatttgg ttaaaaacaa atgtgtcaat 1620

ttcaacttca atggtttaac aggcacaggt gttcttactg agtctaacaa aaagtttctg 1680

cctttccaac aatttggcag agacattgct gacactactg atgctgtccg tgatccacag 1740

acacttgaga ttcttgacat tacaccatgt tcttttggtg gtgtcagtgt tataacacca 1800

ggaacaaata cttctaacca ggttgctgtt ctttatcagg atgttaactg cacagaagtc 1860

cctgttgcta ttcatgcaga tcaacttact cctacttggc gtgtttattc tacaggttct 1920

aatgtttttc aaacacgtgc aggctgttta ataggggctg aacatgtcaa caactcatat 1980

gagtgtgaca tacccattgg tgcaggtata tgcgctagtt atcagactca gactaattct 2040

cctcggcggg cacgtagtgt agctagtcaa tccatcattg cctacactat gtcacttggt 2100

gcagaaaatt cagttgctta ctctaataac tctattgcca tacccacaaa ttttactatt 2160

agtgttacca cagaaattct accagtgtct atgaccaaga catcagtaga ttgtacaatg 2220

tacatttgtg gtgattcaac tgaatgcagc aatcttttgt tgcaatatgg cagtttttgt 2280

acacaattaa accgtgcttt aactggaata gctgttgaac aagacaaaaa cacccaagaa 2340

gtttttgcac aagtcaaaca aatttacaaa acaccaccaa ttaaagattt tggtggtttt 2400

aatttttcac aaatattacc agatccatca aaaccaagca agaggtcatt tattgaagat 2460

ctacttttca acaaagtgac acttgcagat gctggcttca tcaaacaata tggtgattgc 2520

cttggtgata ttgctgctag agacctcatt tgtgcacaaa agtttaacgg ccttactgtt 2580

ttgccacctt tgctcacaga tgaaatgatt gctcaataca cttctgcact gttagcgggt 2640

acaatcactt ctggttggac ctttggtgca ggtgctgcat tacaaatacc atttgctatg 2700

caaatggctt ataggtttaa tggtattgga gttacacaga atgttctcta tgagaaccaa 2760

aaattgattg ccaaccaatt taatagtgct attggcaaaa ttcaagactc actttcttcc 2820

acagcaagtg cacttggaaa acttcaagat gtggtcaacc aaaatgcaca agctttaaac 2880

acgcttgtta aacaacttag ctccaatttt ggtgcaattt caagtgtttt aaatgatatc 2940

ctttcacgtc ttgacaaagt tgaggctgaa gtgcaaattg ataggttgat cacaggcaga 3000

cttcaaagtt tgcagacata tgtgactcaa caattaatta gagctgcaga aatcagagct 3060

tctgctaatc ttgctgctac taaaatgtca gagtgtgtac ttggacaatc aaaaagagtt 3120

gattttgtgg gaaagggcta tcatcttatg tccttccctc agtcagcacc tcatggtgta 3180

gtcttcttgc atgtgactta tgtccctgca caagaaaaga acttcacaac tgctcctgcc 3240

atttgtcatg atggaaaagc acactttcct cgtgaaggtg tctttgtttc aaatggcaca 3300

cactggtttg taacacaaag gaatttttat gaaccacaaa tcattactac agacaacaca 3360

tttgtgtctg gtaactgtga tgttgtaata ggaattgtca acaacacagt ttatgatcct 3420

ttgcaacctg aattagactc attcaaggag gagttagata aatattttaa gaatcataca 3480

tcaccagatg ttgatttagg tgacatctct ggcattaatg cttcagttgt aaacattcaa 3540

aaagaaattg accgcctcaa tgaggttgcc aagaatttaa atgaatctct catcgatctc 3600

caagaacttg gaaagtatga gcagtatata aaatggccat ggtacatttg gctaggtttt 3660

atagctggct tgattgccat agtaatggtg acaattatgc tttgctgtat gaccagttgc 3720

tgtagttgtc tcaagggctg ttgttcttgt ggatcctgct gcaaatttga tgaagacgac 3780

tctgagccag tgctcaaagg agtcaaatta cattacacat aa 3822

<210> 13
<211> 3813

<212>  DNA
<213>  Severe acute respiratory syndrome-related coronavirus

<400>  13
atgtttgttt ttcttgtttt attgccacta gtctctagtc agtgtgttaa tcttacaacc      60

agaactcaat taccccctgc atacactaat tctttcacac gtggtgttta ttaccctgac     120

aaagttttca gatcctcagt tttacattca actcaggact tgttcttacc tttcttttcc     180

aatgttactt ggttccatgc tatctctggg accaatggta ctaagaggtt tgataaccct     240

gtcctaccat ttaatgatgg tgtttatttt gcttccactg agaagtctaa cataataaga     300

ggctggattt ttggtactac tttagattcg aagacccagt ccctacttat tgttaataac     360

gctactaatg ttgttattaa agtctgtgaa tttcaatttt gtaatgatcc atttttgggt     420

gtttaccaca aaaacaacaa aagttggatg gaaagtgagt tcagagttta ttctagtgcg     480

aataattgca cttttgaata tgtctctcag ccttttctta tggaccttga aggaaaacag     540

ggtaatttca aaaatcttag ggaatttgtg tttaagaata ttgatggtta ttttaaaata     600

tattctaagc acacgcctat taatttagtg cgtgatctcc ctcagggttt ttcggcttta     660

gaaccattgg tagatttgcc aataggtatt aacatcacta ggtttcaaac tttacttgct     720

ttacatagaa gttatttgac tcctggtgat tcttcttcag gttggacagc tggtgctgca     780

gcttattatg tgggttatct tcaacctagg acttttctat taaaatataa tgaaaatgga     840

accattacag atgctgtaga ctgtgcactt gaccctctct cagaaacaaa gtgtacgttg     900

aaatccttca ctgtagaaaa aggaatctat caaacttcta actttagagt ccaaccaaca     960

gaatctattg ttagatttcc taatattaca aacttgtgcc cttttggtga agtttttaac    1020

gccaccagat ttgcatctgt ttatgcttgg aacaggaaga gaatcagcaa ctgtgttgct    1080

gattattctg tcctatataa ttccgcatca ttttccactt ttaagtgtta tggagtgtct    1140

cctactaaat taaatgatct ctgctttact aatgtctatg cagattcatt tgtaattaga    1200

ggtgatgaag tcagacaaat cgctccaggg caaactggaa agattgctga ttataattat    1260

aaattaccag atgattttac aggctgcgtt atagcttgga attctaacaa tcttgattct    1320

aaggttggtg gtaattataa ttacctgtat agattgttta ggaagtctaa tctcaaacct    1380

tttgagagag atatttcaac tgaaatctat caggccggta gcacaccttg taatggtgtt    1440

gaaggtttta attgttactt tcctttacaa tcatatggtt tccaacccac ttatggtgtt    1500

ggttaccaac catacagagt agtagtactt tcttttgaac ttctacatgc accagcaact    1560

gtttgtggac ctaaaaagtc tactaatttg gttaaaaaca aatgtgtcaa tttcaacttc    1620

aatggtttaa caggcacagg tgttcttact gagtctaaca aaaagtttct gcctttccaa    1680

caatttggca gagacattga tgacactact gatgctgtcc gtgatccaca gacacttgag    1740

attcttgaca ttacaccatg ttcttttggt ggtgtcagtg ttataacacc aggaacaaat    1800

acttctaacc aggttgctgt tctttatcag ggtgttaact gcacagaagt ccctgttgct 1860

attcatgcag atcaacttac tcctacttgg cgtgtttatt ctacaggttc taatgttttt 1920

caaacacgtg caggctgttt aataggggct gaacatgtca acaactcata tgagtgtgac 1980

atacccattg gtgcaggtat atgcgctagt tatcagactc agactaattc tcatcggcgg 2040

gcacgtagtg tagctagtca atccatcatt gcctacacta tgtcacttgg tgcagaaaat 2100

tcagttgctt actctaataa ctctattgcc atacccataa attttactat tagtgttacc 2160

acagaaattc taccagtgtc tatgaccaag acatcagtag attgtacaat gtacatttgt 2220

ggtgattcaa ctgaatgcag caatcttttg ttgcaatatg gcagtttttg tacacaatta 2280

aaccgtgctt taactggaat agctgttgaa caagacaaaa acacccaaga agtttttgca 2340

caagtcaaac aaatttacaa aacaccacca attaaagatt ttggtggttt taatttttca 2400

caaatattac cagatccatc aaaaccaagc aagaggtcat ttattgaaga tctacttttc 2460

aacaaagtga cacttgcaga tgctggcttc atcaaacaat atggtgattg ccttggtgat 2520

attgctgcta gagacctcat ttgtgcacaa aagtttaacg gccttactgt tttgccacct 2580

ttgctcacag atgaaatgat tgctcaatac acttctgcac tgttagcggg tacaatcact 2640

tctggttgga cctttggtgc aggtgctgca ttacaaatac catttgctat gcaaatggct 2700

tataggttta atggtattgg agttacacag aatgttctct atgagaacca aaaattgatt 2760

gccaaccaat ttaatagtgc tattggcaaa attcaagact cactttcttc cacagcaagt 2820

gcacttggaa aacttcaaga tgtggtcaac caaaatgcac aagctttaaa cacgcttgtt 2880

aaacaactta gctccaattt tggtgcaatt tcaagtgttt taaatgatat ccttgcacgt 2940

cttgacaaag ttgaggctga agtgcaaatt gataggttga tcacaggcag acttcaaagt 3000

ttgcagacat atgtgactca acaattaatt agagctgcag aaatcagagc ttctgctaat 3060

cttgctgcta ctaaaatgtc agagtgtgta cttggacaat caaaaagagt tgatttttgt 3120

ggaaagggct atcatcttat gtccttccct cagtcagcac ctcatggtgt agtcttcttg 3180

catgtgactt atgtccctgc acaagaaaag aacttcacaa ctgctcctgc catttgtcat 3240

gatggaaaag cacactttcc tcgtgaaggt gtctttgttt caaatggcac acactggttt 3300

gtaacacaaa ggaattttta tgaaccacaa atcattacta cacacaacac atttgtgtct 3360

ggtaactgtg atgttgtaat aggaattgtc aacaacacag tttatgatcc tttgcaacct 3420

gaattagact cattcaagga ggagttagat aaatatttta agaatcatac atcaccagat 3480

gttgatttag gtgacatctc tggcattaat gcttcagttg taaacattca aaaagaaatt 3540

gaccgcctca atgaggttgc caagaattta aatgaatctc tcatcgatct ccaagaactt 3600

ggaaagtatg agcagtatat aaaatggcca tggtacattt ggctaggttt tatagctggc 3660

ttgattgcca tagtaatggt gacaattatg ctttgctgta tgaccagttg ctgtagttgt 3720

ctcaagggct gttgttcttg tggatcctgc tgcaaatttg atgaagacga ctctgagcca 3780

gtgctcaaag gagtcaaatt acattacaca taa 3813

<210> 14
<211> 3813
<212> DNA
<213> Severe acute respiratory syndrome-related coronavirus

<400> 14
atgtttgttt ttcttgtttt attgccacta gtctctagtc agtgtgttaa ttttacaacc 60

agaactcaat taccccctgc atacactaat tctttcacac gtggtgttta ttaccctgac 120

aaagttttca gatcctcagt tttacattca actcaggact tgttcttacc tttcttttcc 180

aatgttactt ggttccatgc tatacatgtc tctgggacca atggtactaa gaggtttgct 240

aaccctgtcc taccatttaa tgatggtgtt tattttgctt ccactgagaa gtctaacata 300

ataagaggct ggatttttgg tactacttta gattcgaaga cccagtccct acttattgtt 360

aataacgcta ctaatgttgt tattaaagtc tgtgaatttc aattttgtaa tgatccattt 420

ttgggtgttt attaccacaa aaacaacaaa agttggatgg aaagtgagtt cagagtttat 480

tctagtgcga ataattgcac ttttgaatat gtctctcagc cttttcttat ggaccttgaa 540

ggaaaacagg gtaatttcaa aaatcttagg gaatttgtgt ttaagaatat tgatggttat 600

tttaaaatat attctaagca cacgcctatt aatttagtgc gtggtctccc tcagggtttt 660

tcggctttag aaccattggt agatttgcca ataggtatta acatcactag gtttcaaact 720

ttacatagaa gttatttgac tcctggtgat tcttcttcag gttggacagc tggtgctgca 780

gcttattatg tgggttatct tcaacctagg acttttctat taaaatataa tgaaaatgga 840

accattacag atgctgtaga ctgtgcactt gaccctctct cagaaacaaa gtgtacgttg 900

aaatccttca ctgtagaaaa aggaatctat caaacttcta actttagagt ccaaccaaca 960

gaatctattg ttagatttcc taatattaca aacttgtgcc cttttggtga agtttttaac 1020

gccaccagat ttgcatctgt ttatgcttgg aacaggaaga gaatcagcaa ctgtgttgct 1080

gattattctg tcctatataa ttccgcatca ttttccactt ttaagtgtta tggagtgtct 1140

cctactaaat taaatgatct ctgctttact aatgtctatg cagattcatt tgtaattaga 1200

ggtgatgaag tcagacaaat cgctccaggg caaactggaa atattgctga ttataattat 1260

aaattaccag atgattttac aggctgcgtt atagcttgga attctaacaa tcttgattct 1320

aaggttggtg gtaattataa ttacctgtat agattgttta ggaagtctaa tctcaaacct 1380

tttgagagag atatttcaac tgaaatctat caggccggta gcacaccttg taatggtgtt 1440

aaaggtttta attgttactt tcctttacaa tcatatggtt tccaacccac ttatggtgtt 1500

ggttaccaac catacagagt agtagtactt tcttttgaac ttctacatgc accagcaact 1560

gtttgtggac ctaaaaagtc tactaatttg gttaaaaaca aatgtgtcaa tttcaacttc 1620

aatggtttaa caggcacagg tgttcttact gagtctaaca aaaagtttct gcctttccaa 1680

caatttggca gagacattgc tgacactact gatgctgtcc gtgatccaca gacacttgag 1740

attcttgaca ttacaccatg ttcttttggt ggtgtcagtg ttataacacc aggaacaaat 1800

acttctaacc aggttgctgt tctttatcag ggtgttaact gcacagaagt ccctgttgct 1860

attcatgcag atcaacttac tcctacttgg cgtgtttatt ctacaggttc taatgttttt 1920

caaacacgtg caggctgttt aataggggct gaacatgtca acaactcata tgagtgtgac 1980

atacccattg gtgcaggtat atgcgctagt tatcagactc agactaattc tcctcggcgg 2040

gcacgtagtg tagctagtca atccatcatt gcctacacta tgtcacttgg tgtagaaaat 2100

tcagttgctt actctaataa ctctattgcc atacccacaa attttactat tagtgttacc 2160

acagaaattc taccagtgtc tatgaccaag acatcagtag attgtacaat gtacatttgt 2220

ggtgattcaa ctgaatgcag caatcttttg ttgcaatatg gcagtttttg tacacaatta 2280

aaccgtgctt taactggaat agctgttgaa caagacaaaa acacccaaga agtttttgca 2340

caagtcaaac aaatttacaa aacaccacca attaaagatt ttggtggttt taatttttca 2400

caaatattac cagatccatc aaaaccaagc aagaggtcat ttattgaaga tctacttttc 2460

aacaaagtga cacttgcaga tgctggcttc atcaaacaat atggtgattg ccttggtgat 2520

attgctgcta gagacctcat ttgtgcacaa aagtttaacg gccttactgt tttgccacct 2580

ttgctcacag atgaaatgat tgctcaatac acttctgcac tgttagcggg tacaatcact 2640

tctggttgga cctttggtgc aggtgctgca ttacaaatac catttgctat gcaaatggct 2700

tataggttta atggtattgg agttacacag aatgttctct atgagaacca aaaattgatt 2760

gccaaccaat ttaatagtgc tattggcaaa attcaagact cactttcttc cacagcaagt 2820

gcacttggaa aacttcaaga tgtggtcaac caaaatgcac aagctttaaa cacgcttgtt 2880

aaacaactta gctccaattt tggtgcaatt tcaagtgttt taaatgatat cctttcacgt 2940

cttgacaaag ttgaggctga agtgcaaatt gataggttga tcacaggcag acttcaaagt 3000

ttgcagacat atgtgactca acaattaatt agagctgcag aaatcagagc ttctgctaat 3060

cttgctgcta ctaaaatgtc agagtgtgta cttggacaat caaaaagagt tgatttttgt 3120

ggaaagggct atcatcttat gtccttccct cagtcagcac ctcatggtgt agtcttcttg 3180

catgtgactt atgtccctgc acaagaaaag aacttcacaa ctgctcctgc catttgtcat 3240

gatggaaaag cacactttcc tcgtgaaggt gtctttgttt caaatggcac acactggttt 3300

gtaacacaaa ggaattttta tgaaccacaa atcattacta cagacaacac atttgtgtct 3360

ggtaactgtg atgttgtaat aggaattgtc aacaacacag tttatgatcc tttgcaacct 3420

gaattagact cattcaagga ggagttagat aaatatttta agaatcatac atcaccagat 3480

gttgatttag gtgacatctc tggcattaat gcttcagttg taaacattca aaaagaaatt 3540

gaccgcctca atgaggttgc caagaattta aatgaatctc tcatcgatct ccaagaactt 3600

ggaaagtatg agcagtatat aaaatggcca tggtacattt ggctaggttt tatagctggc 3660

ttgattgcca tagtaatggt gacaattatg ctttgctgta tgaccagttg ctgtagttgt 3720

ctcaagggct gttgttcttg tggatcctgc tgcaaatttg atgaagacga ctctgagcca 3780

gtgctcaaag gagtcaaatt acattacaca taa 3813

<210> 15
<211> 3786
<212> DNA
<213> Artificial Sequence

<220>
<223> SARS-CoV-2 spike like protein

<400> 15
atgttcgtgt tcctggtgct gctgcctctg gtgtccagcc agtgcgtgaa cttccggaac 60

agaacccagc tgcctagcgc ctacaccaac agctttacca gaggcgtgta ctaccccgac 120

aaggtgttca gatccagcgt gctgcactct acccaggacc tgttcctgcc tttcttcagc 180

aacgtgacct ggttccacgc catcagcggc accaacgtga tcaagagatt cgacaacccc 240

gtgctgccct tcaacgacgg ggtgtacttt gccagcaccg agaagtccaa catcatcaga 300

ggctggatct tcggcaccac actggacagc aagacccaga gcctgctgat cgtgaacaac 360

gccaccaacg tcgtgatcaa agtgtgcgag ttccagttct gcaactaccc cttcctgggc 420

gtctaccaca agaacaacaa gagctggatg gaaagcggcg tgtacagcag cgccaacaac 480

tgcaccttcg agtacgtgtc ccagcctttc ctgatggacc tggaaggcaa gcagggcaac 540

ttcaagaacc tgtccgagtt cgtgtttaag aacatcgacg gctacttcaa gatctacagc 600

aagcacaccc ctatcaacct cgtgcgggat ctgcctcagg gcttctctgc tctggaaccc 660

ctggtggatc tgcccatcgg catcaacatc acccggtttc agacactgct ggccctgcac 720

aatagcagct ctggatggac tgctggcgcc gctgcctact atgtgggata cctgcagcct 780

cggaccttcc tgctgaagta caacgagaac ggcaccatca ccgacgccgt ggattgtgct 840

ctggaccctc tgagcgagac aaagtgcacc ctgaagtcct tcaccgtgga aaagggcatc 900

taccagacca gcaacttccg ggtgcagccc accgaatcca tcgtgcggtt ccccaatatc 960

accaatctgt gccccttcgg cgaggtgttc aatgccacca gattcgcctc tgtgtacgcc 1020

tggaaccgga agcggatcag caattgcgtg gccgactact ccgtgctgta caactccgcc 1080

agcttcagca ccttcaagtg ctacggcgtg tcccctacca agctgaacga cctgtgcttc 1140

acaaacgtgt acgccgacag cttcgtgatc cggggagatg aagtgcggca gattgcccct 1200

ggacagacag gcacaatcgc cgactacaac tacaagctgc ccgacgactt caccggctgt 1260

gtgattgcct ggaacagcaa caacctggac tccaaagtcg gcggcaacta caattaccgg 1320

tacaggctgt ttcggaagtc caatctgaag cccttcgagc gggacatctc caccgagatc 1380

tatcaggccg gcagcaagcc ttgcaatggc gtgcagggct tcaactgcta cagcccactg 1440

cagagctacg gcttccagcc aacatacggc gtgggctacc agccttacag agtggtggtg 1500

ctgagcttcg agctgctgca tgctcctgcc acagtgtgcg gccctaagaa aagcaccaat 1560

ctcgtgaaga acaaatgcgt caacttcaat ttcaacggcc tgaccggcac cggcgtgctg 1620

acagagagca acaagaagtt cctgccattc cagcagttcg gccgggatat cgccgatacc 1680

acagatgccg tcagagatcc ccagacactg gaaatcctgg acatcacccc ttgcagcttc 1740

ggcggagtgt ctgtgatcac ccctggcacc aacaccagca atcaggtggc agtgctgtac 1800

cagggcgtta actgtaccga agtgcccgtg gccattcacg ccgatcagct gacacctaca 1860

tggcgggtgt actccaccgg ctccaacgtg ttccagacaa gagccggctg tctgatcgga 1920

gccgagtatg tgaacaacag ctacgagtgc gacatcccca tcggcgctgg catctgcgcc 1980

tcttaccaga cacagaccaa cagcaggcgg agagccagat ctgtggccag ccagagcatc 2040

attgcctaca caatgtctct gggcgccgag aacagcgtgg cctactccaa caactctatc 2100

gctatcccca tcaacttcac catcagcgtg accacagaga tcctgcctgt gtccatgacc 2160

aagaccagcg tggactgcac catgtacatc tgcggcgata gcaccgagtg ctccaacctg 2220

ctgctgcagt acggcagctt ctgcacccag ctgaatagag ccctgaccgg aatcgccgtg 2280

gaacaggaca agaacaccca agaggtgttc gcccaagtga agcagatcta caagacccct 2340

cctatcaagg acttcggcgg ctttaacttc agccagattc tgcccgatcc tagcaagccc 2400

agcaagcgga gccctatcga ggacctgctg ttcaacaaag tgacactggc cgacgccggc 2460

ttcatcaagc agtatggcga ttgtctgggc gacattgccg ccagggatct gatttgcgcc 2520

cagaagttca acggactgaa cgtgctgcct cctctgctga ccgatgagat gatcgcccag 2580

tacacatctg ccctgctggc cggaacaatc accagcggct ggacatttgg agctggaccc 2640

gctctgcaga tcccatttcc aatgcagatg gcctaccggt tcaacggcat cggagtgacc 2700

cagaatgtgc tgtacgagaa ccagaagctg atcgccaacc agttcaacag cgccatcggc 2760

aagatccagg acagcctgag cagcacacct agcgctctgg gcaagctgca gaacgtggtc 2820

aaccagaatg cccaggcact gaacaccctg gtcaagcagc tgtccagcaa tttcggcgcc 2880

atctctagcg tgctgaacga tatcctggcc agactggacc cacctgaggc cgaggtgcag 2940

atcgacagac tgatcacagg acggctgcag tccctgcaga cctacgttac acagcagctg 3000

atcagagccg ccgagattag agcctctgcc aatctggccg ccatcaagat gagcgagtgt 3060

gtgctgggcc agagcaagag agtggacttt tgcggcaagg gctaccacct gatgagcttc 3120

cctcagtctg ctcctcacgg cgtggtgttt ctgcacgtga catacgtgcc cgctcaagag 3180

aagaatttca ccaccgctcc agccatctgc cacgacggca aagcccactt tcctagagaa 3240

ggcgtgttcg tgtccaacgg cacccattgg ttcgtgaccc agcggaactt ctacgagccc 3300

cagatcatca ccacacacaa caccttcgtg tccggcaact gtgacgttgt gatcggcatt 3360

gtgaacaata ccgtgtacga ccctctgcag cccgagctgg actccttcaa agaggaactg 3420

gacaagtact ttaagaacca cacaagcccc gacgtggacc tgggcgatat cagcggaatc 3480

aacgcctcct tcgtgaacat ccagaaagag atcgaccggc tgaacgaggt ggccaagaat 3540

ctgaacgaga gcctgatcga cctgcaagaa ctggggaagt acgagcagta catcaagtgg 3600

ccttggtaca tctggctggg ctttatcgcc ggactgattg ccatcgtgat ggtcacaatc 3660

atgctgtgct gcatgaccag ctgctgctct tgcctgaagg gctgctgtag ctgtggctcc 3720

tgctgcaagt tcgacgagga cgattctgag cccgtgctga agggcgtgaa actgcactac 3780

acatga 3786